# EUROPEAN PATENT APPLICATION

(11) **EP 4 063 505 A1**
(43) Date of publication of application: **28.09.2022**
(21) Application number: 21425011.0
(22) Date of filing: 22.03.2021
(51) Int. Cl.: C12N 15/113, A61K 31/7088, A61P 25/16

(54) **FUNCTIONAL NUCLEIC ACID MOLECULES DIRECTED TO TARGETS FOR NERVOUS SYSTEM DISORDERS**

(71) Applicant: Fondazione Istituto Italiano di Tecnologia, 16163 Genova (IT)
(72) Inventor: Bon, Carlotta, Royston, SG8 5BU (GB); Gustincich, Stefano, 16163 Genova (IT); Espinoza, Stefano, 16163 Genova (IT); Pulcrano, Salvatore, 16163 Genova (IT); Grasso, Laura, Royston, SG8 5BU (GB)
(74) Representative: D Young & Co LLP

(57) **Abstract**

The invention relates to therapeutic agents which enhance protein translation of an endogenous mRNA sequence, particularly for use in a method of treating a disease or disorder of the nervous system. In particular, the therapeutic agent is a functional nucleic acid molecule comprising at least one target determinant sequence and at least one regulatory sequence.

SEQUENCE LISTING

<110>  Fondazione Istituto Italiano Di Tecnologia
       Transine Therapeutics Limited

<120>  FUNCTIONAL NUCLEIC ACID MOLECULES DIRECTED TO TARGETS FOR NERVOUS
        SYSTEM DISORDERS

<130>  TSI-C-P3000EPp

<160>  204

<170>  PatentIn version 3.5

<210>  1
<211>  167
<212>  RNA
<213>  Mus musculus

<400>  1
cagugcuaga ggaggucaga agagggcauu ggauccccca gaacuggagu uauacgguaa      60

ccucguggug guugugaacc accaugugga uggauauuga guuccaaaca cugguccugu     120

gcaagagcau ccagugcucu uaagugcuga gccaucucuu uagcucc                   167

<210>  2
<211>  77
<212>  RNA
<213>  Mus musculus

<400>  2
   gaacuggagu uauacgguaa ccucguggug guugugaacc accaugugga uggauauuga      60
   guuccaaaca cuggucc                                                    77

<210>  3
<211>  29
<212>  RNA
<213>  Mus musculus

<400>  3
ccucguggug guugugaacc accaugugg                                        29

<210>  4
<211>  61
<212>  RNA
<213>  Mus musculus

<400>  4
guuauacggu aaccucgugg ugguugugaa ccaccaugug gauggauauu gaguuccaaa      60

c                                                                 61

<210>  5
<211>  98
<212>  RNA
<213>  Mus musculus

<400>  5
aucccccaga acuggaguua uacgguaacc ucgugguggu ugugaaccac cauguggaug      60

gauauugagu uccaaacacu gguccugugc aagagcau                              98

<210>  6
<211>  129
<212>  RNA
<213>  Mus musculus

<400>  6
gaagagggca uuggaucccc cagaacugga guuauacggu aaccucgugg ugguugugaa      60

ccaccaugug gauggauauu gaguuccaaa cacugguccu gugcaagagc auccagugcu     120

cuuaagugc                                                             129

<210>  7
<211>  183
<212>  RNA
<213>  Mus musculus

<400>  7
gggcagugcu agaggagguc agaagagggc auuggauccc ccagaacugg aguuauacgg      60

uaaccucgug gugguuguga accaccaugu ggauggauau ugaguuccaa acacuggucc     120

ugugcaagag cauccagugc ucuuaagugc ugagccaucu cuuuagcucc agucucuuaa     180

gcu                                                                   183

<210>  8
<211>  67
<212>  RNA
<213>  Mus musculus

<400>  8
gaacuggagu uauacgguaa ccucguggug guugugaacc accaugugga uggauauuga      60

guuccaa                                                                67

<210>  9
<211>  107
<212>  RNA
<213>  Mus musculus

<400>  9
ggauccccca gaacuggagu uauacgguaa ccucguggug guugugaacc accaugugga      60

uggauauuga guuccaaaca cugguccugu gcaagagcau ccagugc                  107


<210>  10
<211>  127
<212>  RNA
<213>  Mus musculus

<400>  10
agagggcauu ggauccccca gaacuggagu uauacgguaa ccucguggug guugugaacc      60

accaugugga uggauauuga guuccaaaca cugguccugu gcaagagcau ccagugcucu     120

uaagugc                                                              127


<210>  11
<211>  38
<212>  RNA
<213>  Mus musculus

<400>  11
gguaaccucg uggugguugu gaaccaccau guggaugg                            38


<210>  12
<211>  180
<212>  RNA
<213>  Artificial sequence

<220>
<223>  Artificial sequence

<400>  12
cagugcuaga ggaggucaga agagggcauu ggauccccca gaacuggagu uauacgauaa      60

ccucguggug guugugaacc accaugugga uggauauuga guuccaaaca cugguccugu     120

gcaagagcau ccagugcucu uaagugcuga gccaucucuu uagcuccagu cucuuaagcu     180


<210>  13
<211>  180
<212>  RNA
<213>  Artificial sequence

<220>
<223>  Artificial sequence

<400>  13
cagugcuaga ggaggucaga agagggcauu ggauccccca gaacuggagu uauacgcuaa      60

ccucguggug guugugaacc accaugugga uggauauuga guuccaaaca cugguccugu     120

gcaagagcau ccagugcucu uaagugcuga gccaucucuu uagcuccagu cucuuaagcu     180


<210>  14
<211>  180
<212>  RNA
<213>  Artificial sequence

<220>
<223>  Artificial sequence

<400>  14
cagugcuaga ggaggucaga agagggcauu ggauccccca gaacuggcgu uauacgguaa      60

ccucguggug guugugaacc accaugugga uggauauuga guuccaaaca cugguccugu     120

gcaagagcau ccagugcucu uaagugcuga gccaucucuu uagcuccagu cucuuaagcu     180


<210>  15
<211>  180
<212>  RNA
<213>  Artificial sequence

<220>
<223>  Artificial sequence

<400>  15
cagugcuaga ggaggucaga agagggcauu ggauccccca gaaguggagu uauacgguaa      60

ccucguggug guugugaacc accaugugga uggauauuga guuccaaaca cugcuccugu     120

gcaagagcau ccagugcucu uaagugcuga gccaucucuu uagcuccagu cucuuaagcu     180


<210>  16
<211>  180
<212>  RNA
<213>  Artificial sequence

<220>
<223>  Artificial sequence

<400>  16
cagugcuaga ggaggucaga agagggcauu ggauccccca gauggugagu uauacgguaa      60

ccucguggug guugugaacc accaugugga uggauauuga guuccaaaca cgucaccugu 120

gcaagagcau ccagugcucu uaagugcuga gccaucucuu uagcuccagu cucuuaagcu 180


<210> 17
<211> 180
<212> RNA
<213> Artificial sequence

<220>
<223> Artificial sequence

<400> 17
cagugcuaga ggaggucaga agagggcauu ggauccccca gaacugcacu auacgguaac 60

cucguggugg uugugaacca ccauguggau ggauauugag uuccaaauga gugguccugu 120

gcaagagcau ccagugcucu uaagugcuga gccaucucuu uagcuccagu cucuuaagcu 180


<210> 18
<211> 116
<212> RNA
<213> Artificial sequence

<220>
<223> Artificial sequence

<400> 18
gggcauugga ucccccagaa cuggaguuau acgguaaccu cguggugguu gugaaccacc 60

auguggaugg auauugaguu ccaaacacug guccugugca agagcaucca gugcuc 116


<210> 19
<211> 77
<212> RNA
<213> Artificial sequence

<220>
<223> Artificial sequence

<400> 19
ggacuggagu uauacgguaa ccucguggug guugugaacc accaugugga uggauauuga 60

guuccaaaca cuggucc 77


<210> 20
<211> 77
<212> RNA
<213> Artificial sequence

<220>
<223>  Artificial sequence

<400>  20
gaacuggcgu uauacgguaa ccucguggug guugugaacc accaugugga uggauauuga      60

guuccaaaca cuggucc                                                    77


<210>  21
<211>  107
<212>  RNA
<213>  Mus musculus

<400>  21
gaugccuuag aaguggaguu aagaguugug agcugccguu uuuugguucu gggacucgaa      60

cucguuuccu cugauacuau caaccaccaa gccaucucuu cagcccc                  107


<210>  22
<211>  131
<212>  RNA
<213>  Mus musculus

<400>  22
gccagaagaa guugugggau ucccuggaac uggagcaacc aacaguuugu gugcaccaug      60

uggguaaugg gaaucgaacc uggguccucu auaagacugg ccagugcucu uaacuacuga      120

ggugcauuuc u                                                          131


<210>  23
<211>  187
<212>  RNA
<213>  Mus musculus

<400>  23
uuauuuuaaa uauaugagua uuucaccugc auaggcgcac aguacccaca gagacuagaa      60

gaggguggca gaucuccuga gacuggaguu aaugcuugug agcugccaug uggaugcugg      120

aaaucaaacc cagguccuuu ggaaggcagg caggugcucu uaaucaugga agcaucucuu      180

cagcucc                                                               187


<210>  24
<211>  131
<212>  RNA
<213>  Mus musculus

<400>  24

cagcgacauc agaagaggau auuggauccc auuacagaug guugaaggcc accaugucgu 60

ugcugggaau gaacucaaga ccucuggaag agcagucagu gcucuuaacc ucugagccau 120

cucuccagcc c 131

<210> 25
<211> 114
<212> RNA
<213> Mus musculus

<400> 25
auccccucca aagcucaaga ugguuguaag ccacccugug auugcuggga uuugaacuca 60

agaccuccgg aagagcaauu agugcucuua accgcugagc aaucucucca gccc 114

<210> 26
<211> 357
<212> RNA
<213> Mus musculus

<400> 26
gugcagugcu agaggagguc agaagagggc auuggauccc ccagaacugg aguuauacgg 60

uaaccucgug gugguuguga accaccaugu ggauggauau ugaguuccaa acacuggucc 120

ugugcaagag cauccagugc ucuuaagugc ugagccaucu cuuuagcucc uuauuuuaaa 180

uauaugagua uuucaccugc auaggcgcac aguacccaca gagacuagaa gaggguggca 240

gaucuccuga gacuggaguu aaugcuugug agcugccaug uggaugcugg aaaucaaacc 300

cagguccuuu ggaaggcagg caggugcucu uaaucaugga agcaucucuu cagcucc 357

<210> 27
<211> 532
<212> RNA
<213> Mus musculus

<400> 27
gugcagugcu agaggagguc agaagagggc auuggauccc ccagaacugg aguuauacgg 60

uaaccucgug gugguuguga accaccaugu ggauggauau ugaguuccaa acacuggucc 120

ugugcaagag cauccagugc ucuuaagugc ugagccaucu cuuuagcucc gugcgaauuc 180

ggugcagugc uagaggaggu cagaagaggg cauuggaucc cccagaacug gaguuauacg 240

guaaccucgu ggugguugug aaccaccaug uggauggaua uugaguucca aacacugguc 300

cugugcaaga gcauccagug cucuuaagug cugagccauc ucuuuagcuc cgugcgaauu 360

cggugcagug cuagaggagg ucagaagagg gcauuggauc ccccagaacu ggaguuauac 420

gguaaccucg uggugguugu gaaccaccau guggauggau auugaguucc aaacacuggu 480

ccugugcaag agcauccagu gcucuuaagu gcugagccau cucuuuagcu cc 532

<210> 28
<211> 228
<212> RNA
<213> Mus musculus

<400> 28
uuuuuuuaaa aauuuauuuu uauuuuaugu guaugagugu uuugccugca uguaugucug 60

uguaccacgu gcgugccugg ugcccgcgga ggccagaaga gggcgucgga uccccuggaa 120

cuggaguuac agaugguugu gagccgccau gugggugcug ggaaucgaac ccggguccuc 180

uggaagagca gccagugcuc uuaaccgcug agccaucucu ccagcccc 228

<210> 29
<211> 214
<212> RNA
<213> Mus musculus

<400> 29
uuuuuuuuac uuguauaggu guuuugccug cauguguauc uaucuaugua ccgaauaugu 60

uccugguauc cacagagacc aaaaguggau guuguaucuc cugaaauugg agucauagac 120

aguuaugagc ugccauuuga gugcuuggaa uagaacccag guccucuuaa agagcaucca 180

gugcucuuaa aaacugagac aucucuguag ccuc 214

<210> 30
<211> 200
<212> RNA
<213> Mus musculus

<400> 30
uuuauuuugc uuuauguguc ugaguguuug cuugaaugua ugucugugua ccacgccugu 60

accuugugcc uucagaguug agaggagggc auaggaucuc cuggaacugg aauugcaggu 120

gguugugagc cacccugugg guccugggga ccauacucca gcaagaacau caugugcucu 180

uaauuccuga gucuccaacc 200

<210> 31

<211>  214
<212>  RNA
<213>  Mus musculus

<400>  31
uuuauuuacu uaucuuuaug uguaugagug uguugucaga cuguuauguc ugugugucac      60

augcaugccu gcuguucaug gaguccagaa gagggcaucg gauccccugg aacuggaguu     120

acagaugagu ggccauguga auguuaagaa ccaaaccugg guccucugaa agagcagaca     180

augcucuuaa cuacugagcu gucucuccag cccc                                 214


<210>  32
<211>  205
<212>  RNA
<213>  Mus musculus

<400>  32
uuauuuuauu cguguaagug uuuugccagc aucuaugucu ucgcacuaug ugcaggucug      60

gugccugagg gguccagacg agagcacugg gucuccggga acuggaguua cagaucauug     120

ugagccacca ugugggugca gggaaucgaa ccugggaccu cuggaggagc agccacugcu     180

cuuaaccacu acacuauuuc uccag                                           205


<210>  33
<211>  121
<212>  RNA
<213>  Mus musculus

<400>  33
ucuguggacc acuguguaca gaagccugag aaggcuagca gauccccaga acuggaacug      60

ugagacgcug ugcuauggag gugcuaggaa cugaaaaugg auggguccuc ugcaagagca     120

g                                                                     121


<210>  34
<211>  191
<212>  RNA
<213>  Mus musculus

<400>  34
uuguuuuaau ugaauggcua uaggguguuu cuucuguaug uauaucuaug uuugguaccu      60

acagaggcau cagauccucu ggaacuguag uugcugacag uugugagcug ucauggggau     120

gcuggaauug aaccuggauc cuaugaaaga acagccagug uucuuaaccg cugagcuauc     180

ucuccaggcc c                                                          191

<210>  35
<211>  205
<212>  RNA
<213>  Mus musculus

<400>  35
uuuuuuuuuu aauuuuaaaa aaaaagauuu uauuuauuua uuuuauauau gaugaguaca      60

cugucacucu uuucagacac ccuagaaaag gggggcauca gaucccauua cagaugguug     120

ugagccacau gguugcuggg aauugaccuc aggaccucug aaagagcagu cagugcucuc     180

aaccuuugag ucaucucucc agccc                                           205

<210>  36
<211>  190
<212>  RNA
<213>  Mus musculus

<400>  36
auguauaucu guaaugggac auacucacau acaugggcac gugaguauaa aaggccagaa      60

gagagcacug gacccucugg aguugagauu cuaagcaguu gugaaccauc ugauguaggu     120

gcugggaacu gaacuugggu ccuuugcuag agaaguaugu cucuuaacca cugagccgua     180

ucuccauccc                                                            190

<210>  37
<211>  169
<212>  RNA
<213>  Mus musculus

<400>  37
uaaagauuua uucauuaagu acacuguagc uaucuucaga cgcaucagaa gagggcguca      60

gaucucuuua caggugguug ugagccacca ugugguugcu ggaauuugaa cucaggaccu     120

ucaaaagagc agucaguguu cuuaaccgcu gagccaucuc uccaacccc                 169

<210>  38
<211>  159
<212>  RNA
<213>  Mus musculus

<400>  38
uuauuuauua uaaguacacu guagcugucu ucagacacaa caaaagaggg cgucagaucu      60

cauuacaggu gguugagcca ccaugugguu gcugggauuu gaacucagga ccuucagaac 120

agucagugcu cuuacccacu gagccagcga gccagcccc 159

<210> 39
<211> 77
<212> RNA
<213> Artificial sequence

<220>
<223> Artificial sequence

<400> 39
gaacuggagu uauacgguaa ccucguggug guugggaacc accaugugga uggauauuga 60

guuccaaaca cuggucc 77

<210> 40
<211> 77
<212> RNA
<213> Artificial sequence

<220>
<223> Artificial sequence

<400> 40
gaacuggagu uauacgguaa ccucguggug guucccaacc accaugugga uggauauuga 60

guuccaaaca cuggucc 77

<210> 41
<211> 77
<212> RNA
<213> Artificial sequence

<220>
<223> Artificial sequence

<400> 41
ggaccggagu uauacgguaa ccgcguggug guugugaacc accacgcgga uggauauuga 60

guuccaaaca ccggucc 77

<210> 42
<211> 77
<212> RNA
<213> Artificial sequence

<220>
<223> Artificial sequence

<400>  42
gaacuagagu uauacgguaa ccacauggug guugugaacc accaugugga uggauauuga      60

guuccaaaca cuaguuc                                                     77


<210>  43
<211>  206
<212>  RNA
<213>  Artificial sequence

<220>
<223>  Artificial sequence

<400>  43
uuauuuuaaa uauaugagua uuucaccugc auaggcgcac aguacccaca gagacuagaa      60

gaggguagua gauccccuag aacuggaguu auacgguaac cucgguggug uugugagcua     120

ccauguggau ggauacuggg aaucaaaccc agguccugug gaaggcaggc aggugcucuc     180

aagcacugag ccaucucuuc agcucc                                          206


<210>  44
<211>  206
<212>  RNA
<213>  Artificial sequence

<220>
<223>  Artificial sequence

<400>  44
uuauuuuaaa uauaugagua uuucaccugc auaggcgcac agugcucaag gagaucagaa      60

gagggcauca gaucuccuga gacuggaguu auacgguaac cucgugaugg uugugaacua     120

ccauguggau ggauauugag uuccaaacac agguccugug caagagcagc aggugcucuu     180

aagcacggaa ccaucucuuu agcucc                                          206


<210>  45
<211>  156
<212>  RNA
<213>  Artificial sequence

<220>
<223>  Artificial sequence

<400>  45
gaggcuagaa gaggguauca gauccccuga gacuggaguu auacgguaac cucgguggug      60

uugugagcca ccauguggau ggauacugag aaccaaaccc ugguccugug caagagcauc 120

aggugcucuu aagcacggaa ccaucucuuc agcucc 156

<210> 46
<211> 68
<212> RNA
<213> Artificial sequence

<220>
<223> Artificial sequence

<400> 46
guccugugca agagcaucga acucggugcu cuuaagcaca gaagccacca agccaucucu 60

ucagcccc 68

<210> 47
<211> 110
<212> RNA
<213> Artificial sequence

<220>
<223> Artificial sequence

<400> 47
cagugcuaga ggaggucaga agagggcauc ccccagccuc guggugguug ugaaccacca 60

uguggcugug caagagcaug cucuuaagug cugagccauc ucuuuagcuc 110

<210> 48
<211> 126
<212> RNA
<213> Artificial sequence

<220>
<223> Artificial sequence

<400> 48
gagggcauug gaucccccag aacuggaguu auacgguaac cucguggugg uugugaacca 60

ccauguggau ggauauugag uuccaaacac ugguccugug caagagcauc cagugcucuu 120

aagugc 126

<210> 49
<211> 89
<212> RNA
<213> Artificial sequence

<220>
<223>  Artificial sequence

<400>  49
ggauccccca gaacuggagu uauacgguaa ccucguggug guugugaacc accaugugga      60

uggauauuga guuccaaaca cugguccug                                      89


<210>  50
<211>  72
<212>  RNA
<213>  Artificial sequence

<220>
<223>  Artificial sequence

<400>  50
ugcuagagga ggucagaaga gggcauugga ugcaaaucca gugcucuuaa gugcugagcc      60

aucucuuuag cu                                                        72


<210>  51
<211>  94
<212>  RNA
<213>  Artificial sequence

<220>
<223>  Artificial sequence

<400>  51
gagggcauug gaucccccag aacuggaguu auacgguaac gauggauauu gaguuccaaa      60

cacugguccu gugcaagagc auccagugcu cuua                                94


<210>  52
<211>  383
<212>  RNA
<213>  Hepatitis C virus

<400>  52
gccagccccc ugaugggggc gacacuccac caugaaucac uccccuguga ggaacuacug      60

ucuucacgca gaaagcgucu agccauggcg uuaguaugag ugucgugcag ccuccaggac     120

cccccucccc gggagagcca uaguggucug cggaaccggu gaguacaccg gaauugccag     180

gacgaccggg uccuuucuug gauaaacccg cucaaugccu ggagauuugg gcgugccccc     240

gcaagacugc uagccgagua guguuggguc gcgaaaggcc uugugguacu gccugauagg     300

gugcuugcga gugccccggg aggucucgua gaccgugcac caugagcacg aauccuaaac     360

cucaaagaaa aaccaaacgu aac 383

<210> 53
<211> 383
<212> RNA
<213> Hepatitis C virus

<400> 53
guuacguuug guuuuucuuu gagguuuagg auucgugcuc auggugcacg gucuacgaga 60

ccucccgggg cacucgcaag cacccuauca ggcaguacca caaggccuuu cgcgacccaa 120

cacuacucgg cuagcagucu ugcgggggca cgcccaaauc uccaggcauu gagcggguuu 180

auccaagaaa ggacccgguc guccuggcaa uuccggugua cucaccgguu ccgcagacca 240

cuauggcucu cccgggaggg gggguccugg aggcugcacg acacucauac uaacgccaug 300

gcuagacgcu uucugcguga agacaguagu uccucacagg ggagugauuc augguggagu 360

gucgccccca ucagggggcu ggc 383

<210> 54
<211> 312
<212> RNA
<213> Human poliovirus

<400> 54
augagucugg acaucccuca ccggugacgg ugguccaggc ugcguuggcg gccuaccuau 60

ggcuaacgcc augggacgcu aguugugaac aaggugugaa gagccuauug agcuacauaa 120

gaauccuccg gccccugaau gcggcuaauc ccaaccucgg agcagguggu cacaaaccag 180

ugauuggccu gucguaacgc gcaaguccgu ggcggaaccg acuacuuugg guguccgugu 240

uuccuuuuau uuuauugugg cugcuuaugg ugacaaucac agauuguuau cauaaagcga 300

auuggauugg cc 312

<210> 55
<211> 312
<212> RNA
<213> Human poliovirus

<400> 55
ggccaaucca auucgcuuua ugauaacaau cugugauugu caccauaagc agccacaaua 60

aaauaaaagg aaacacggac acccaaagua gucgguuccg ccacggacuu gcgcguuacg 120

acaggccaau cacugguuug ugaccaccug cuccgagguu gggauuagcc gcauucaggg 180

gccggaggau ucuuauguag cucaauaggc ucuucacacc uuguucacaa cuagcguccc 240

auggcguuag ccauagguag gccgccaacg cagccuggac caccgucacc ggugagggau 300

guccagacuc au 312

<210> 56
<211> 576
<212> RNA
<213> Encephalomyocarditis virus

<400> 56
cccccccucu cccucccccc ccccuaacgu uacuggccga agccgcuugg aauaaggccg 60

gugugcguuu gucuauaugu uauuuuccac cauauugccg ucuuuuggca augugagggc 120

ccggaaaccu ggcccugucu ucuugacgag cauuccuagg ggucuuuccc cucucgccaa 180

aggaaugcaa ggucuguuga augucgugaa ggaagcaguu ccucuggaag cuucuugaag 240

acaaacaacg ucuguagcga cccuuugcag gcagcggaac cccccaccug gcgacaggug 300

ccucugcggc caaaagccac guguauaaga uacaccugca aaggcggcac aaccccagug 360

ccacguugug aguuggauag uuguggaaag agucaaaugg cucuccucaa gcguauucaa 420

caaggggcug aaggaugccc agaagguacc ccauuguaug ggaucugauc uggggccucg 480

gugcacaugc uuuacaugug uuuagucgag guuaaaaaac gucuaggccc cccgaaccac 540

ggggacgugg uuuuccuuug aaaaacacga ugauaa 576

<210> 57
<211> 576
<212> RNA
<213> Encephalomyocarditis virus

<400> 57
uuaucaucgu guuuuucaaa ggaaaaccac guccccgugg uucggggggc cuagacguuu 60

uuuaaccucg acuaaacaca uguaaagcau gugcaccgag gccccagauc agaucccaua 120

caauggggua ccuucugggc auccuucagc cccuuguuga auacgcuuga ggagagccau 180

uugacucuuu ccacaacuau ccaacucaca acguggcacu gggguugugc cgccuuugca 240

gguguaucuu auacacgugg cuuuuggccg cagaggcacc ugucgccagg ugggggguuc 300

cgcugccugc aaagggucgc uacagacguu guuugucuuc aagaagcuuc cagaggaacu 360

gcuuccuuca cgacauucaa cagaccuugc auuccuuugg cgagagggga aagaccccua 420

ggaaugcucg ucaagaagac agggccaggu uuccgggccc ucacauugcc aaaagacggc 480

aauauggugg aaaauaacau auagacaaac gcacaccggc cuuauuccaa gcggcuucgg 540

ccaguaacgu uagggggggg ggagggagag gggggg 576

<210> 58
<211> 192
<212> RNA
<213> Cricket paralysis virus

<400> 58
aaagcaaaaa ugugaucuug cuuguaaaua caauuuugag agguuaauaa auuacaagua 60

gugcuauuuu uguauuuagg uuagcuauuu agcuuuacgu uccaggaugc cuaguggcag 120

ccccacaaua uccaggaagc ccucucugcg guuuuucaga uuagguaguc gaaaaaccua 180

agaaauuuac cu 192

<210> 59
<211> 192
<212> RNA
<213> Cricket paralysis virus

<400> 59
agguaaauuu cuuagguuuu ucgacuaccu aaucugaaaa accgcagaga gggcuuccug 60

gauauugugg ggcugccacu aggcauccug gaacguaaag cuaaauagcu aaccuaaaua 120

caaaaauagc acuacuugua auuuauuaac cucucaaaau uguauuuaca agcaagauca 180

cauuuuugcu uu 192

<210> 60
<211> 231
<212> RNA
<213> Homo sapiens

<400> 60
cagagaucca ggggaggcgc cugugaggcc cggaccugcc ccggggcgaa ggguaugugg 60

cgagacagag cccugcaccc cuaauucccg guggaaaacu ccuguugccg uuucccucca 120

ccggccugga gucucccagu cuugucccgg cagugccgcc cuccccacua agaccuaggc 180

gcaaaggcuu ggcucauggu ugacagcuca gagagagaaa gaucugaggg a 231

<210> 61
<211> 231
<212> RNA
<213> Homo sapiens

<400> 61
ucccucagau cuuucucucu cugagcuguc aaccaugagc caagccuuug cgccuagguc 60

uuagugggga gggcggcacu gccgggacaa gacugggaga cuccaggccg guggagggaa 120

acggcaacag gaguuuucca ccgggaauua ggggugcagg gcucugucuc gccacauacc 180

cuucgccccg gggcaggucc gggccucaca ggcgccuccc cuggaucucu g 231

<210> 62
<211> 460
<212> RNA
<213> Homo sapiens

<400> 62
acuuuuggug ggcauuuaaa aaugugugug uauguguaua uauguaugug uauguaugug 60

uauauaugua uauguaugua uguaucgcgu guaugugugu auguaugcau guguauguau 120

guauaugcau guauguguau guguauauau guaugugugu guauguauau gugugugua u 180

guguaugugu gugugugugu gugugugua u guauguaugu auguauaugu auuauacaca 240

uauacacaua uugguuuuuu uaaucauuug agaguuaguu gaagauaaaa acccaucacc 300

ccuaaaugua uuccaaagaa uaagaacauu guuuuauaca uagcacacuu aacaaaauca 360

agaaauuuaa cauuaauaca guacuguuac cuaauccgua gucgauuuuc aaauuuuguc 420

aguuguucca auaaugucc u uuauauauuc cccgcccagc 460

<210> 63
<211> 460
<212> RNA
<213> Homo sapiens

<400> 63
   gcugggcggg gaauauauaa aggacauuau uggaacaacu gacaaaauuu gaaaaucgac 60
   uacggauuag guaacaguac uguauuaaug uuaaauuucu ugauuuuguu aagugugcua 120
   uguauaaaac aauguucuua uucuuuggaa uacauuuagg ggugaugggu uuuuaucuuc 180
   aacuaacucu caaaugauua aaaaaaccaa uauguguaua uguguauaau acauauacau 240
   acauacauac auacacacac acauacacac acacauacac auacacacac auauacauac 300
   acacacauac auauauacac auacacauac augcauauac auacauacac augcauacau 360
   acacacauac acgcgauaca uacauacaua uacauauaua cacauacaua cacauacaua 420
   uauacacaua cacacacauu uuuaaaugcc caccaaaagu 460

<210> 64
<211> 395
<212> RNA
<213> Homo sapiens

<400> 64
aauuccagcg agaggcagag ggagcgagcg ggcggccggc uaggguggaa gagccgggcg 60

agcagagcug cgcugcgggc guccugggaa gggagauccg gagcgaauag ggggcuucgc 120

cucuggccca gcccucccgc uugauccccc aggccagcgg uccgcaaccc uugccgcauc 180

cacgaaacuu ugcccauagc agcgggcggg cacuuugcac uggaacuuac aacacccgag 240

caaggacgcg acucucccga cgcggggagg cuauucugcc cauuugggga cacuuccccg 300

ccgcugccag gacccgcuuc ucugaaaggc ucuccuugca gcugcuuaga cgcuggauuu 360

uuuucgggua guggaaaacc agcagccucc cgcga 395

<210> 65
<211> 395
<212> RNA
<213> Homo sapiens

<400> 65
ucgcgggagg cugcugguuu uccacuaccc gaaaaaaauc cagcgucuaa gcagcugcaa 60

ggagagccuu ucagagaagc ggguccuggc agcggcgggg aagugucccc aaaugggcag 120

aauagccucc ccgcgucggg agagucgcgu ccuugcucgg guguuguaag uuccagugca 180

aagugcccgc ccgcugcuau gggcaaaguu ucguggaugc ggcaaggguu gcggaccgcu 240

ggccuggggg aucaagcggg agggcugggc cagaggcgaa gcccccuauu cgcuccggau 300

cucccuuccc aggacgcccg cagcgcagcu cugcucgccc ggcucuucca cccuagccgg 360

ccgcccgcuc gcucccucug ccucucgcug gaauu 395

<210> 66
<211> 48
<212> RNA
<213> Homo sapiens

<400>  66
gggcacuuug cacuggaacu uacaacaccc gagcaaggac gcgacucu                    48


<210>  67
<211>  48
<212>  RNA
<213>  Homo sapiens

<400>  67
agagucgcgu ccuugcucgg guguuguaag uuccagugca aagugccc                    48


<210>  68
<211>  71
<212>  RNA
<213>  Homo sapiens

<400>  68
guacugacau cguagaugga aaucauaaac ugacucuugg uuugauuugg aauauaaucc      60

uccacuggca g                                                            71


<210>  69
<211>  71
<212>  RNA
<213>  Homo sapiens

<400>  69
cugccagugg aggauuauau uccaaaucaa accaagaguc aguuuaugau uuccaucuac      60

gaugucagua c                                                            71


<210>  70
<211>  18
<212>  DNA
<213>  Homo sapiens

<400>  70
gcatggcttc agccgagt                                                     18


<210>  71
<211>  22
<212>  DNA
<213>  Homo sapiens

<400>  71
gcatggcttc agccgagtta ca                                                22


<210>  72

<211>  18
<212>  DNA
<213>  Homo sapiens

<400>  72
ggcttcagcc gagttaca                                                    18


<210>  73
<211>  20
<212>  DNA
<213>  Homo sapiens

<400>  73
ggcttcagcc gagttacagg                                                  20


<210>  74
<211>  24
<212>  DNA
<213>  Homo sapiens

<400>  74
gcatggcttc agccgagtta cagg                                             24


<210>  75
<211>  18
<212>  DNA
<213>  Homo sapiens

<400>  75
ccatgctaaa cttgacaa                                                    18


<210>  76
<211>  18
<212>  DNA
<213>  Homo sapiens

<400>  76
gctaaacttg acaaactc                                                    18


<210>  77
<211>  22
<212>  DNA
<213>  Homo sapiens

<400>  77
ccatgctaaa cttgacaaac tc                                               22


<210>  78

<211>  18
<212>  DNA
<213>  Homo sapiens

<400>  78
ccataaaggt actgaagc                                                    18


<210>  79
<211>  18
<212>  DNA
<213>  Homo sapiens

<400>  79
aaaggtactg aagctggg                                                    18


<210>  80
<211>  18
<212>  DNA
<213>  Homo sapiens

<400>  80
cggcggcacc tcggatcg                                                    18


<210>  81
<211>  38
<212>  DNA
<213>  Homo sapiens

<400>  81
taaagtcccg tccggcggcg gcacctcgga tcgggtct                              38


<210>  82
<211>  40
<212>  DNA
<213>  Homo sapiens

<400>  82
cttaaagtcc cgtccggcgg cggcacctcg gatcgggtct                            40


<210>  83
<211>  26
<212>  DNA
<213>  Homo sapiens

<400>  83
cttaaagtcc cgtccggcgg cggcac                                           26


<210>  84

<211>  14
<212>  DNA
<213>  Homo sapiens

<400>  84
cttaaagtcc cgtc                                                          14


<210>  85
<211>  18
<212>  DNA
<213>  Homo sapiens

<400>  85
tcatcttaaa gtcccgtc                                                      18


<210>  86
<211>  19
<212>  DNA
<213>  Homo sapiens

<400>  86
tcatcttaaa gtcccgtcc                                                     19


<210>  87
<211>  20
<212>  DNA
<213>  Homo sapiens

<400>  87
tcatcttaaa gtcccgtccg                                                    20


<210>  88
<211>  31
<212>  DNA
<213>  Homo sapiens

<400>  88
tcatcttaaa gtcccgtccg gcggcggcac c                                       31


<210>  89
<211>  44
<212>  DNA
<213>  Homo sapiens

<400>  89
tcatcttaaa gtcccgtccg gcggcggcac ctcggatcgg gtct                         44


<210>  90

<211>  40
<212>  DNA
<213>  Homo sapiens

<400>  90
aacttcatct taaagtcccg tccggcggcg gcacctcgga                              40


<210>  91
<211>  70
<212>  DNA
<213>  Homo sapiens

<400>  91
tcccataact tcatcttaaa gtcccgtccg gcggcggcac ctcggatcgg gtctccgcag        60

accctaggtt                                                               70


<210>  92
<211>  31
<212>  DNA
<213>  Homo sapiens

<400>  92
catcccataa cttcatctta aagtcccgtc c                                       31


<210>  93
<211>  21
<212>  DNA
<213>  Homo sapiens

<400>  93
tcttaaagtc ccgtccggcg g                                                  21


<210>  94
<211>  18
<212>  DNA
<213>  Homo sapiens

<400>  94
gcatatttga gtcactgc                                                      18


<210>  95
<211>  25
<212>  DNA
<213>  Homo sapiens

<400>  95
atttgagtca ctgctcagcg cgaag                                              25

<210> 96
<211> 46
<212> DNA
<213> Homo sapiens

<400> 96
ccatgacatc atcgaactga tcaggataat cctctggcat atttga 46


<210> 97
<211> 18
<212> DNA
<213> Homo sapiens

<400> 97
ccatgacatc atcgaact 18


<210> 98
<211> 18
<212> DNA
<213> Homo sapiens

<400> 98
acatgcctgc cctacttt 18


<210> 99
<211> 25
<212> DNA
<213> Homo sapiens

<400> 99
tcatcttaaa gtcccgtccg gcggc 25


<210> 100
<211> 21
<212> DNA
<213> Homo sapiens

<400> 100
cttaaagtcc cgtccggcgg c 21


<210> 101
<211> 21
<212> DNA
<213> Homo sapiens

<400> 101
acgacatccc ataacttcat c 21

<210> 102
<211> 18
<212> DNA
<213> Homo sapiens

<400> 102
acatcccata acttcatc 18

<210> 103
<211> 14
<212> DNA
<213> Homo sapiens

<400> 103
atttgagtca ctgc 14

<210> 104
<211> 18
<212> DNA
<213> Homo sapiens

<400> 104
atttgagtca ctgctcag 18

<210> 105
<211> 24
<212> DNA
<213> Homo sapiens

<400> 105
gcatatttga gtcactgctc agcg 24

<210> 106
<211> 40
<212> DNA
<213> Homo sapiens

<400> 106
gggactgcgg ctagggccgg cgggtctgga tggcgggtgc 40

<210> 107
<211> 18
<212> DNA
<213> Homo sapiens

<400> 107
cccgtgcgcg ctggggcc 18

<210>  108
<211>  29
<212>  DNA
<213>  Homo sapiens

<400>  108
ccgtgcgcgc tggggccacg gctggaggg                                          29


<210>  109
<211>  38
<212>  DNA
<213>  Homo sapiens

<400>  109
cccgtgcgcg ctggggccac ggctggaggg actgcggc                                38


<210>  110
<211>  40
<212>  DNA
<213>  Homo sapiens

<400>  110
cgcccgtgcg cgctggggcc acggctggag ggactgcggc                              40


<210>  111
<211>  14
<212>  DNA
<213>  Homo sapiens

<400>  111
cgcccgtgcg cgct                                                          14


<210>  112
<211>  18
<212>  DNA
<213>  Homo sapiens

<400>  112
ccatcgcccg tgcgcgct                                                      18


<210>  113
<211>  19
<212>  DNA
<213>  Homo sapiens

<400>  113
ccatcgcccg tgcgcgctg                                                     19

<210>  114
<211>  20
<212>  DNA
<213>  Homo sapiens

<400>  114
ccatcgcccg tgcgcgctgg                                                    20


<210>  115
<211>  44
<212>  DNA
<213>  Homo sapiens

<400>  115
ccatcgcccg tgcgcgctgg ggccacggct ggagggactg cggc                         44


<210>  116
<211>  40
<212>  DNA
<213>  Homo sapiens

<400>  116
ttcgccatcg cccgtgcgcg ctggggccac ggctggaggg                              40


<210>  117
<211>  70
<212>  DNA
<213>  Homo sapiens

<400>  117
gtcgccttcg ccatcgcccg tgcgcgctgg ggccacggct ggagggactg cggctagggc        60

cggcgggtct                                                               70


<210>  118
<211>  18
<212>  DNA
<213>  Homo sapiens

<400>  118
ccatcaccac ctcctcgc                                                      18


<210>  119
<211>  40
<212>  DNA
<213>  Homo sapiens

<400>  119

caccacctcc tcgcgcgcgc cggcgtgcac ggtgcacacg                                40

<210>  120
<211>  18
<212>  DNA
<213>  Homo sapiens

<400>  120
gcatggtgcg gttctccg                                                        18

<210>  121
<211>  50
<212>  DNA
<213>  Homo sapiens

<400>  121
ggtgcggttc tccgagatgg agaggttccg gttgagaacc agcctatagt                    50

<210>  122
<211>  18
<212>  DNA
<213>  Homo sapiens

<400>  122
ccaatgccac tttgccta                                                        18

<210>  123
<211>  18
<212>  DNA
<213>  Homo sapiens

<400>  123
cggttgcgga cactgagc                                                        18

<210>  124
<211>  18
<212>  DNA
<213>  Homo sapiens

<400>  124
cacggtgtcc tccttccg                                                        18

<210>  125
<211>  44
<212>  DNA
<213>  Homo sapiens

<400>  125

tgtcctcctt ccgcttgaac tccaccacgg cgctggcggt gtcg 44

<210> 126
<211> 18
<212> DNA
<213> Homo sapiens

<400> 126
gccatcgccc gtgcgcgc 18

<210> 127
<211> 24
<212> DNA
<213> Homo sapiens

<400> 127
cctccttccg cttgaactcc acca 24

<210> 128
<211> 17
<212> DNA
<213> Homo sapiens

<400> 128
catcgcccgt gcgcgct 17

<210> 129
<211> 15
<212> DNA
<213> Homo sapiens

<400> 129
ccatcgcccg tgcgc 15

<210> 130
<211> 18
<212> DNA
<213> Homo sapiens

<400> 130
tcgccatcgc ccgtgcgc 18

<210> 131
<211> 20
<212> DNA
<213> Homo sapiens

<400> 131

cttcgccatc gcccgtgcgc                                                    20

<210>  132
<211>  18
<212>  DNA
<213>  Homo sapiens

<400>  132
gacgtcgcct tcgccatc                                                      18

<210>  133
<211>  20
<212>  DNA
<213>  Homo sapiens

<400>  133
cggacgtcgc cttcgccatc                                                    20

<210>  134
<211>  24
<212>  DNA
<213>  Homo sapiens

<400>  134
gcaccggacg tcgccttcgc catc                                               24

<210>  135
<211>  20
<212>  DNA
<213>  Homo sapiens

<400>  135
caccatcacc acctcctcgc                                                    20

<210>  136
<211>  18
<212>  DNA
<213>  Homo sapiens

<400>  136
ggaagggcac catcacca                                                      18

<210>  137
<211>  15
<212>  DNA
<213>  Homo sapiens

<400>  137

aagggcacca tcacc 15

<210> 138
<211> 18
<212> DNA
<213> Homo sapiens

<400> 138
gaggatccac gtcggcga 18

<210> 139
<211> 18
<212> DNA
<213> Homo sapiens

<400> 139
cctcagggtc attagatg 18

<210> 140
<211> 38
<212> DNA
<213> Homo sapiens

<400> 140
cctcagggtc attagatgaa gagaagacca caggggtt 38

<210> 141
<211> 40
<212> DNA
<213> Homo sapiens

<400> 141
cccctcaggg tcattagatg aagagaagac cacaggggtt 40

<210> 142
<211> 44
<212> DNA
<213> Homo sapiens

<400> 142
ccatcccctc agggtcatta gatgaagaga agaccacagg ggtt 44

<210> 143
<211> 14
<212> DNA
<213> Homo sapiens

<400> 143

cccctcaggg tcat 14

<210> 144
<211> 18
<212> DNA
<213> Homo sapiens

<400> 144
ccatcccctc agggtcat 18

<210> 145
<211> 19
<212> DNA
<213> Homo sapiens

<400> 145
ccatcccctc agggtcatt 19

<210> 146
<211> 20
<212> DNA
<213> Homo sapiens

<400> 146
ccatcccctc agggtcatta 20

<210> 147
<211> 40
<212> DNA
<213> Homo sapiens

<400> 147
aactccatcc cctcagggtc attagatgaa gagaagacca 40

<210> 148
<211> 70
<212> DNA
<213> Homo sapiens

<400> 148
cttgaaaact ccatcccctc agggtcatta gatgaagaga agaccacagg ggttccagag 60

tctctgaagg 70

<210> 149
<211> 40
<212> DNA
<213> Homo sapiens

<400>  149
gatgcttacc ctactcaaag gcttgggaca ttcctctctg                              40


<210>  150
<211>  18
<212>  DNA
<213>  Homo sapiens

<400>  150
ccatccgtcg cccactgc                                                      18


<210>  151
<211>  40
<212>  DNA
<213>  Homo sapiens

<400>  151
ccgtcgccca ctgcgtgtac tctcatagcg gctgaaggta                              40


<210>  152
<211>  54
<212>  DNA
<213>  Homo sapiens

<400>  152
atactcagct ccatccgtcg cccactgcgt gtactctcat agcggctgaa ggta              54


<210>  153
<211>  18
<212>  DNA
<213>  Homo sapiens

<400>  153
gcatccctcg atcccagg                                                      18


<210>  154
<211>  18
<212>  DNA
<213>  Homo sapiens

<400>  154
caggggtatc ttgagctt                                                      18


<210>  155
<211>  18
<212>  DNA
<213>  Homo sapiens

<400>  155
gtatcttgag cttggtat                                                    18


<210>  156
<211>  18
<212>  DNA
<213>  Homo sapiens

<400>  156
actcagctcc atccgtcg                                                    18


<210>  157
<211>  30
<212>  DNA
<213>  Homo sapiens

<400>  157
ctggccatgg gtacccggat gatgttatat                                       30


<210>  158
<211>  18
<212>  DNA
<213>  Mus musculus

<400>  158
tgctccacac ggctccct                                                    18


<210>  159
<211>  38
<212>  DNA
<213>  Mus musculus

<400>  159
ctaaagatgt atttttgctc cacacggctc cctaggct                              38


<210>  160
<211>  30
<212>  DNA
<213>  Mus musculus

<400>  160
tatttttgct ccacacggct ccctaggctt                                       30


<210>  161
<211>  18
<212>  DNA
<213>  Mus musculus

<400>  161
ctaaagatgt atttttgc                                                    18


<210>  162
<211>  40
<212>  DNA
<213>  Mus musculus

<400>  162
ggctaaagat gtatttttgc tccacacggc tccctaggct                            40


<210>  163
<211>  27
<212>  DNA
<213>  Mus musculus

<400>  163
ggctaaagat gtatttttgc tccacac                                          27


<210>  164
<211>  14
<212>  DNA
<213>  Mus musculus

<400>  164
ggctaaagat gtat                                                        14


<210>  165
<211>  18
<212>  DNA
<213>  Mus musculus

<400>  165
ccatggctaa agatgtat                                                    18


<210>  166
<211>  19
<212>  DNA
<213>  Mus musculus

<400>  166
ccatggctaa agatgtatt                                                   19


<210>  167
<211>  20
<212>  DNA
<213>  Mus musculus

<400>  167
ccatggctaa agatgtattt                                                    20


<210>  168
<211>  44
<212>  DNA
<213>  Mus musculus

<400>  168
ccatggctaa agatgtattt ttgctccaca cggctcccta ggct                         44


<210>  169
<211>  40
<212>  DNA
<213>  Mus musculus

<400>  169
acatccatgg ctaaagatgt atttttgctc cacacggctc                              40


<210>  170
<211>  70
<212>  DNA
<213>  Mus musculus

<400>  170
tcatgaacac atccatggct aaagatgtat ttttgctcca cacggctccc taggcttctg        60

aagaactccg                                                               70


<210>  171
<211>  42
<212>  DNA
<213>  Mus musculus

<400>  171
tcatgaacac atccatggct aaagatgtat ttttgctcca ca                           42


<210>  172
<211>  21
<212>  DNA
<213>  Mus musculus

<400>  172
tggctaaaga tgtatttttg c                                                  21


<210>  173
<211>  18

<212>  DNA
<213>  Mus musculus

<400>  173
tcatgaacac atccatgg                                                    18


<210>  174
<211>  40
<212>  DNA
<213>  Mus musculus

<400>  174
gaacacatcc atggctaaag atgtattttt gctccacacg                            40


<210>  175
<211>  18
<212>  DNA
<213>  Mus musculus

<400>  175
ctcctcaccc ttgcccat                                                    18


<210>  176
<211>  37
<212>  DNA
<213>  Mus musculus

<400>  176
tacccctcct cacccttgcc catctggtcc ttcttga                              37


<210>  177
<211>  46
<212>  DNA
<213>  Mus musculus

<400>  177
gcatttcata agcctcactg ccaggatcca caggcatgtc ttccag                     46


<210>  178
<211>  18
<212>  DNA
<213>  Mus musculus

<400>  178
ttttggaacc tacataga                                                    18


<210>  179
<211>  44

<212>  DNA
<213>  Mus musculus

<400>  179
cactgttgtc actccatgaa ccactccttc cttagttttg gaac                        44


<210>  180
<211>  18
<212>  DNA
<213>  Homo sapiens

<400>  180
tacaccacac tgtcgtcg                                                     18


<210>  181
<211>  38
<212>  DNA
<213>  Homo sapiens

<400>  181
ctaatgaatt cctttacacc acactgtcgt cgaatggc                               38


<210>  182
<211>  30
<212>  DNA
<213>  Homo sapiens

<400>  182
ttcctttaca ccacactgtc gtcgaatggc                                        30


<210>  183
<211>  18
<212>  DNA
<213>  Homo sapiens

<400>  183
ctaatgaatt cctttaca                                                     18


<210>  184
<211>  40
<212>  DNA
<213>  Homo sapiens

<400>  184
ggctaatgaa ttcctttaca ccacactgtc gtcgaatggc                             40


<210>  185
<211>  26

<212>  DNA
<213>  Homo sapiens

<400>  185
ggctaatgaa ttcctttaca ccacac                                          26


<210>  186
<211>  14
<212>  DNA
<213>  Homo sapiens

<400>  186
ggctaatgaa ttcc                                                       14


<210>  187
<211>  18
<212>  DNA
<213>  Homo sapiens

<400>  187
ccatggctaa tgaattcc                                                   18


<210>  188
<211>  19
<212>  DNA
<213>  Homo sapiens

<400>  188
ccatggctaa tgaattcct                                                  19


<210>  189
<211>  20
<212>  DNA
<213>  Homo sapiens

<400>  189
ccatggctaa tgaattcctt                                                 20


<210>  190
<211>  44
<212>  DNA
<213>  Homo sapiens

<400>  190
ccatggctaa tgaattcctt tacaccacac tgtcgtcgaa tggc                      44


<210>  191
<211>  40

<212>  DNA
<213>  Homo sapiens

<400>  191
acatccatgg ctaatgaatt cctttacacc acactgtcgt                              40


<210>  192
<211>  70
<212>  DNA
<213>  Homo sapiens

<400>  192
tcatgaatac atccatggct aatgaattcc tttacaccac actgtcgtcg aatggccact        60

cccagttctc                                                               70


<210>  193
<211>  41
<212>  DNA
<213>  Homo sapiens

<400>  193
tcatgaatac atccatggct aatgaattcc tttacaccac a                            41


<210>  194
<211>  21
<212>  DNA
<213>  Homo sapiens

<400>  194
tggctaatga attcctttac a                                                  21


<210>  195
<211>  18
<212>  DNA
<213>  Homo sapiens

<400>  195
tcatgaatac atccatgg                                                      18


<210>  196
<211>  40
<212>  DNA
<213>  Homo sapiens

<400>  196
gaatacatcc atggctaatg aattccttta caccacactg                              40

<210>  197
<211>  46
<212>  DNA
<213>  Homo sapiens

<400>  197
gcatttcata agcctcattg tcaggatcca caggcatatc ttccag                    46


<210>  198
<211>  18
<212>  DNA
<213>  Homo sapiens

<400>  198
gcatatcttc cagaattc                                                   18


<210>  199
<211>  18
<212>  DNA
<213>  Homo sapiens

<400>  199
ttttggagcc tacataga                                                   18


<210>  200
<211>  44
<212>  DNA
<213>  Homo sapiens

<400>  200
gagcctacat agagaacacc ctcttttgtc tttcctgctg cttc                      44


<210>  201
<211>  18
<212>  DNA
<213>  Homo sapiens

<400>  201
ttcttcattc ttgcccaa                                                   18


<210>  202
<211>  18
<212>  DNA
<213>  Homo sapiens

<400>  202
gaatacatcc atggctaa                                                   18

<210> 203
<211> 13
<212> RNA
<213> Artificial Sequence

<220>
<223> synthetic

<400> 203
aucugcagaa uuc 13

<210> 204
<211> 6
<212> RNA
<213> Artificial Sequence

<220>
<223> synthetic

<400> 204
gaauuc 6

## Description

### FIELD OF THE INVENTION

The present invention relates to therapeutic agents for use in a method of treating a disease or disorder of the nervous system, wherein the therapeutic agent increases translation of an endogenous mRNA sequence selected from the group consisting of: a Nurr1, GDNF, cRET or GBA mRNA sequence. Also provided are functional nucleic acid molecules comprising: at least one target binding sequence comprising a sequence reverse complementary to a target mRNA sequence selected from the group consisting of: a Nurr1, GDNF, cRET, or GBA mRNA sequence; and at least one regulatory sequence, as well as methods for use of said functional nucleic acid molecule, particularly in the treatment of diseases or disorders of the nervous system. Also provided are functional nucleic acid molecules comprising: at least one target binding sequence comprising a sequence reverse complementary to a SNCA mRNA sequence; and at least one regulatory sequence, as well as methods for use of said functional nucleic acid molecule, particularly in the generation of animal models.

### BACKGROUND OF THE INVENTION

Parkinson's Disease (PD) is one of the most common neurodegenerative disorders, and it is caused by loss of the dopamine (DA) neurons of the substantia nigra pars compacta (SNpc). It manifests its symptomatology mainly as motor-related deficits, such as rigidity, tremor, and bradykinesia (Meissner et al. (2011) Nat. Reviews Drug Discovery 10:377-393). Although familial PD (about 5% of the total cases) has been important to unveil the molecular pathways involved in neurodegeneration, most of the PD patients are sporadic (Obeso et al. (2017) Movement Disorders 32:1264-1310). Currently, no treatments are available that can slow or arrest neurodegeneration. The DA precursor L-DOPA is the gold standard for symptomatic treatments, although over time it may lead to several side effects and resistance (Nagatsua et al. (2009) Parkinsonism Related Disorders 15 (Suppl 1):S3-S8).

Glial cell-derived neurotrophic factor (GDNF) is the most potent neurotrophic factor for DA neurons with great potentiality for clinical application (Kordower et al. (2013) Movement Disorders 28:96-109 and Lin et al. (1993) Science 260:1130-1132). GDNF can protect DA cells and promotes their survival from toxic insults *in vitro* and *in vivo* in mice, rats, and non-human primates (Tomac et al. (1005) Nature 373:33-339, Gash et al. (1996) Nature 380:252-255 and Kirik et al. (2000) Eur. J. of Neuroscience 12:3871-3882). However, clinical trials with recombinant GDNF showed an inconsistency of results, due to the poor spreading of GDNF within the parenchyma and to the unsustainable side effects caused by the high doses of GDNF administered (Nutt et al. (2003) Neurology 60:69-73, Gill et al. (2003) Nat. Med. 9:589-595 and Lang et al. (2006) Ann. Neurol. 59:459-466). Similarly, GDNF expression through viral vectors that showed efficacy in PD animal models may result in uncontrolled, ectopic GDNF overexpression. Therefore, GDNF may be the right candidate for PD therapy, provided that a more specific and physiological expression is achieved to increase its efficacy and safety profile *in vivo.* In this context, a deletion in the 3' UTR of the GDNF gene in a transgenic mouse line led to an increase of 2-fold of endogenous GDNF protein levels (Kumar et al. (2015) PLoS Genet. 11:e1005710). This was sufficient to induce alterations in the DA system and neuroprotection similar to that seen with a large overexpression of GDNF, but without the side effects. These data corroborate the hypothesis that a moderate increase in endogenous GDNF could be beneficial for PD.

cRET (also known as RET) is a tyrosine kinase receptor that is triggered by members of the GDNF family. RET conditional knock-out mice were shown to have late and progressive degeneration of dopaminergic (DA) neurons (Kramer et al. (2007) PLoS Biol. 5:e39), and degeneration of midbrain DA neurons has been indicated to be a key histopathological feature of PD. Furthermore, results from studies involving mutations disrupting the RET51/FKBP52 molecular complex have been suggestive of a possible role for this complex in DA neuron function and PD (Fusco et al. (2010) Hum. Mol. Genet. 19(14):2804-2816).

Orphan nuclear receptor Nurr1 (also known as NR4A2) has also been shown to be involved in the differentiation of midbrain DA neurons. Emerging evidence indicates that impaired Nurr1 function may contribute to the pathogenesis of PD (Decressac et al. (2013) Nat. Rev. Neurol. 9: 629-636).

A new class of long non-coding RNAs (IncRNAs), known as SINEUPs, were previously described to be able to selectively enhance their targets' translation. SINEUP activity relies on the combination of two domains: the overlapping region, or binding domain (BD), that confers specificity, and an embedded inverted SINE B2 element, or effector domain (ED), enhancing target mRNA translation. WO 2012/133947 and WO 2019/150346 disclose functional nucleic acid molecules including SINEUPs. Another class of IncRNAs that use effector domains comprising an internal ribosome entry site (IRES) sequence to provide *trans*acting functional nucleic acid molecules are described in WO 2019/058304.

The aim of the invention is to provide the first gene-specific technology targeting endogenous Nurr1, GDNF, cRET and GBA translation, particularly for use in treating diseases or disorders of the nervous system, such as neurodegenerative disorders.

### SUMMARY OF THE INVENTION

According to a first aspect of the invention, there is provided a therapeutic agent for use in a method of treating a disease or disorder of the nervous system, wherein the therapeutic agent increases translation of an endogenous mRNA sequence selected from the group consisting of: an endogenous Nurr1, human GDNF, cRET or GBA mRNA sequence.

According to a second aspect, there is provided a functional nucleic acid molecule comprising:
at least one target binding sequence comprising a sequence reverse complementary to a target mRNA sequence selected from the group consisting of: a Nurr1, GDNF, cRET or GBA mRNA sequence; and
at least one regulatory sequence comprising a SINE B2 element, a functionally active fragment of a SINE B2 element, an internal ribosome entry site (IRES) sequence or an IRES derived sequence.

According to a further aspect of the invention, there is provided a DNA molecule encoding the functional nucleic acid molecule as defined herein.

According to a yet further aspect, there is provided an expression vector comprising the functional nucleic acid molecule or the DNA molecule as defined herein.

According to a further aspect of the invention, there is provided a composition comprising the functional nucleic acid molecule, the DNA molecule or the expression vector as defined herein.

According to another aspect of the invention, there is provided a method for increasing the protein synthesis efficiency of Nurr1, GDNF, cRET or GBA in a cell comprising administering the functional nucleic acid molecule, the DNA molecule or the expression vector as defined herein, to the cell.

According to a further aspect, there is provided the functional nucleic acid molecule, the DNA molecule or the composition as defined herein, for use as a medicament.

According to a yet further aspect of the invention, there is provided the functional nucleic acid molecule, the DNA molecule or the composition as defined herein, for use in a method of treating a disease or disorder of the nervous system.

According to a further aspect, there is provided method of treating a disease or disorder of the nervous system comprising administering a therapeutic agent that increases translation of an endogenous mRNA sequence selected from the group consisting of: an endogenous Nurr1, human GDNF, cRET or GBA mRNA sequence.

According to a still further aspect, there is provided a method of treating a disease or disorder of the nervous system comprising administering a therapeutically effective amount of the functional nucleic acid molecule, the DNA molecule or the composition as defined herein.

According to a further aspect, there is provided a method of producing a non-human animal model of Parkinson's disease comprising: administering a functional nucleic acid molecule comprising: at least one target binding sequence comprising a sequence reverse complementary to a SNCA mRNA sequence; and at least one regulatory sequence comprising a SINE B2 element, a functionally active fragment of a SINE B2 element, an IRES sequence or an IRES derived sequence, to a non-human animal.

### BRIEF DESCRIPTION OF THE FIGURES

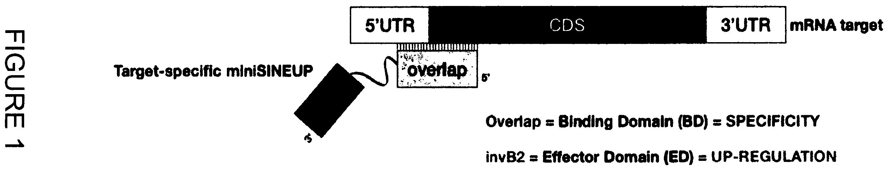

**FIGURE 1****:** Schematic representation of SINEUP functional domains. The overlap is the Binding Domain (BD, grey) that provides SINEUP specificity and is in antisense orientation to the sense protein-coding mRNA (Target mRNA). The inverted SINE B2 (invB2) element from AS Uchl1 is the Effector Domain (ED) and confers enhancement of protein synthesis. 5' to 3' orientation of sense and antisense RNA molecules is indicated. Structural elements of target mRNA are shown: 5' untranslated region (5'UTR, white), coding sequence (CDS, black) and 3' untranslated region (3'UTR, white). Scheme is not drawn in scale.

### DETAILED DESCRIPTION

It is an object of the present invention to provide novel methods of treating a disease or disorder of the nervous system by using a therapeutic agent that increases translation of an endogenous mRNA sequence selected from the group consisting of: an endogenous Nurr1, GDNF, cRET or GBA mRNA sequence. Current therapeutic methods that aim to target such diseases, do so by "replacement" or "supplement" therapy, for example by either delivering the target itself as a protein or by providing the gene (cDNA or mRNA) to encode the therapeutic protein. However, the methods and agents described herein are different because they target the endogenous mRNA sequences encoding therapeutically beneficial proteins already present in the cell (albeit at a reduced level) and modulate the expression of the endogenous protein or isoform thereof in order to ameliorate the disease. In particular, the invention provides functional nucleic acid molecules that increase the level of Nurr1, GDNF, cRET, GBA or SNCA protein expression without overcoming physiological levels. Furthermore, this technology targets endogenous mRNA sequences in a highly gene-specific manner and is only capable of is only driving expression in cells at sites where the endogenous natural product is expressed, thereby limiting side effects. To this end, the inventors have utilised SINEUP technology to develop and target Nurr1, GDNF, cRET, GBA or SNCA mRNA sequences in order to increase the endogenous levels of all Nurr1, GDNF, cRET, GBA or SNCA proteins in human and mouse.

### Definitions

By "functional nucleic acid molecule" there is intended generally that the nucleic acid molecule is capable of enhancing the translation of a target mRNA of interest, in this particular case a Nurr1, GDNF, cRET, GBA or SNCA mRNA. Such molecules may be referred to as "transacting functional nucleic acid molecules" because they promote gene-specific translation up-regulation and act on endogenous mRNAs.

By "endogenous mRNA sequence" there is intended an mRNA sequence of any length (preferably at least 10 nucleotides)that is already present in the target cell and encoded by the listed target gene, particularly selected from: the NR4A2, GDNF, RET, GBA or SNCA gene. Alternative splicing of the endogenous transcripts leads to the generation of various isoforms as described herein.

Alternative splicing of the GDNF mRNA produces 5 protein coding isoforms (UniProt and NCBI database browsers), 3 of which share a at least a portion of the 5' UTR. The isoforms are expressed differentially in different tissues, however all isoforms are expressed in the brain with expression predominantly in the striatum and the highest levels are found in the caudate and lowest in the putamen. The GDNF gene sequence is known in the art, for example see Ensembl ID: ENSG00000168621. The GDNF gene encodes glial cell derived neurotrophic factor (also known as astrocyte-derived trophic factor (ATF)) and is referred to herein as "GDNF protein". The GDNF protein sequence is known in the art, for example see UniProt ID: P39905. GDNF target binding sequences disclosed herein were designed on the isoform NM_000514.4 but it will be readily appreciated that such target binding sequences will bind any isoform or spice variant comprising the sequence to which the target binding sequences bind, such as NM_199231, NM_001190468.1 and NM_001190469.1 which share the 5' UTR with NM_000514.4.

Alternative splicing of the cRET mRNA (also known as RET) produces 3 protein coding isoforms (NCBI database browser) which share a 5' UTR. Of particular interest herein is the long isoform of RET known as RET51. The RET gene sequence is known in the art, for example see Ensembl ID: ENSG00000165731. The RET gene encodes proto-oncogene tyrosine-protein kinase receptor Ret (also known as cadherin family member 12 and proto-oncogene c-Ret) and is referred to herein as "cRET protein". The cRET protein sequence is known in the art, for example see UniProt ID: P07949. RET target binding sequences disclosed herein were designed on the RET51 isoform but it will be readily appreciated that such target binding sequences will bind any isoform or spice variant comprising the sequence to which the target binding sequences bind, such as RET9 (NM_020630.6).

Alternative splicing of the Nurr1 (also known as NR4A2) mRNA produces 8 protein coding isoforms (Ensembl database browser), 4 of which share a portion of the 5' UTR near the AUG start codon. They are expressed in several cell lines of T cell, B cell and fibroblast origin and show strong expression in brain tissue. The Nurr1 protein is encoded by the NR4A2 gene sequence which is known in the art, for example see Ensembl ID: ENSG00000153234. The NR4A2 gene encodes nuclear receptor subfamily 4 group A member 2 (also known as immediate-early response protein NOT, orphan nuclear receptor NURR1 and transcriptionally-inducible nuclear receptor) and is referred to herein as "Nurr1 protein". The Nurr1 protein sequence is known in the art, for example see UniProt ID: P43354. Nurr1 target binding sequences disclosed herein were designed on the isoform NM_006186.4 but it will be readily appreciated that such target binding sequences will bind any isoform or spice variant comprising the sequence to which the target binding sequences bind.

Alternative splicing of the GBA mRNA produces 5 isoforms (UniProt and NCBI database browsers). The GBA gene sequence is known in the art, for example see Ensembl ID: ENSG00000177628. The GBA gene encodes glucosylceramidase beta (also known as β-glucocerebrosidase) and is referred to herein as "GBA protein". The GBA protein sequence is known in the art, for example see UniProt ID: P04062. GBA target binding sequences disclosed herein were designed on the isoform NM_000157.4 but it will be readily appreciated that such target binding sequences will bind any isoform or spice variant comprising the sequence to which the target binding sequences bind, such as NM_001171811.2, NM_001005741.3, NM_001005742.3 and NM_001171812.2 which share a region of the 5' UTR 49bp upstream of M1 compared to NM_000157.4.

Alternative splicing of the SNCA mRNA produces 3 isoforms (UniProt database browser). The SNCA gene sequence is known in the art, for example see Ensembl ID: ENSG00000145335. The SNCA gene encodes α-synuclein (also known as alpha-synuclein or synuclein alpha) and may also be referred to herein as "SNCA protein". The SNCA protein sequence is known in the art, for example see UniProt ID: P37840. SNCA target binding sequences disclosed herein were designed on the isoform NM_000345.4 but it will be readily appreciated that such target binding sequences will bind any isoform or spice variant comprising the sequence to which the target binding sequences bind, such as NM_007308.3 which lacks an alternate in-frame exon compared to NM_000345.4.

The term "SINE" (Short Interspersed Nuclear Element) may be referred to as a non-LTR (long terminal repeat) retrotransposon, and is an interspersed repetitive sequence (a) which encodes a protein having neither reverse-transcription activity nor endonuclease activity or the like and (b) whose complete or incomplete copy sequences exist abundantly in genomes of living organisms.

The term "SINE B2 element" is defined in WO 2012/133947, where specific examples are also provided (see table starting on page 69 of the PCT publication). The term is intended to encompass both SINE B2 elements in direct orientation and in inverted orientation relative to the 5' to 3' orientation of the functional nucleic acid molecule. SINE B2 elements may be identified, for example, using programs like RepeatMask as published (Bedell et al. (2000) Bioinformatics 16(11): 1040-1. MaskerAid: a performance enhancement to RepeatMasker. A sequence may be recognizable as a SINE B2 element by returning a hit in a Repbase database with respect to a consensus sequence of a SINE B2, with a Smith-Waterman (SW) score of over 225, which is the default cutoff in the RepeatMasker program. Generally a SINE B2 element is not less than 20 bp and not more than 400 bp. Preferably, the SINE B2 is derived from tRNA.

By the term "functionally active fragment of a SINE B2 element" there is intended a portion of sequence of a SINE B2 element that retains protein translation enhancing efficiency. This term also includes sequences which are mutated in one or more nucleotides with respect to the wild-type sequences, but retain protein translation enhancing efficiency. The term is intended to encompass both SINE B2 elements in direct orientation and in inverted orientation relative to the 5' to 3' orientation of the functional nucleic acid molecule.

The terms "internal ribosome entry site (IRES) sequence" and "internal ribosome entry site (IRES) derived sequence" are defined in WO 2019/058304. IRES sequences recruit the 40S ribosomal subunit and promote cap-independent translation of a subset of protein coding mRNAs. IRES sequences are generally found in the 5' untranslated region of cellular mRNAs coding for stress-response genes, thus stimulating their translation in *cis.* It will be understood by the term "IRES derived sequence" there is intended a sequence of nucleic acid with a homology to an IRES sequence so as to retain the functional activity thereof, i.e. a translation enhancing activity. In particular, the IRES derived sequence can be obtained from a naturally occurring IRES sequence by genetic engineering or chemical modification, e.g. by isolating a specific sequence of the IRES sequence which remains functional, or mutating/deleting/ introducing one or more nucleotides in the IRES sequence, or replacing one or more nucleotides in the IRES sequence with structurally modified nucleotides or analogs. More in particular, the skilled in the art would know that an IRES derived sequence is a nucleotide sequence capable of promoting translation of a second cistron in a bicistronic construct. Typically, a dual luciferase (Firefly luciferase, Renilla Luciferase) encoding plasmid is used for experimental tests. A major database exists, namely IRESite, for the annotation of nucleotide sequences that have been experimentally validated as IRES, using dual reporter or bicistronic assays (http://iresite.org/IRESite_web.php). Within IRESite, a web-based tool is available to search for sequence-based and structure-based similarities between a query sequence of interest and the entirety of annotated and experimentally validated IRES sequences within the database. The output of the program is a probability score for any nucleotide sequence to be able to act as IRES in a validation experiment with bicistronic constructs. Additional sequence-based and structure-based web-based browsing tools are available to suggest, with a numerical predicting value, the IRES activity potentials of any given nucleotide sequence (http://rna.informatik.uni-freiburg.de/ and http://regrna.mbc.nctu. edu.tw/index1.php).

By the term "miniSINEUP" there is intended a nucleic acid molecule comprising (or consisting of) a binding domain (i.e. a complementary sequence to target mRNA), optionally a spacer sequence, and any SINE or SINE-derived sequence or IRES or IRES-derived sequence as the effector domain (Zucchelli et al. (2015) Front. Cell. Neurosci. 9:174).

By the term "microSINEUP" there is intended a nucleic acid molecule comprising (or consisting of) a binding domain (i.e. a complementary sequence to target mRNA), optionally a spacer sequence, and a functionally active fragment of the SINE or SINE-derived sequence or IRES-derived sequence. For example, the functionally active fragment may be a 77 bp sequence corresponding to nucleotides 44 to 120 of the 167 bp SINE B2 element in AS Uchl1.

Polypeptide or polynucleotide sequences are said to be the same as or "identical" to other polypeptide or polynucleotide sequences, if they share 100% sequence identity over their entire length. Residues in sequences are numbered from left to right, i.e. from N- to C-terminus for polypeptides; from 5' to 3' terminus for polynucleotides.

For the purposes of comparing two closely-related polynucleotide sequences, the "% sequence identity" between a first nucleotide sequence and a second nucleotide sequence may be calculated using NCBI BLAST, using standard settings for nucleotide sequences (BLASTN). For the purposes of comparing two closely-related polypeptide sequences, the "% sequence identity" between a first polypeptide sequence and a second polypeptide sequence may be calculated using NCBI BLAST, using standard settings for polypeptide sequences (BLASTP). A "difference" between sequences refers to an insertion, deletion or substitution of a single nucleotide in a position of the second sequence, compared to the first sequence. Two sequences can contain one, two or more such differences. Insertions, deletions or substitutions in a second sequence which is otherwise identical (100% sequence identity) to a first sequence result in reduced % sequence identity.

References herein to "a disease or disorder of the nervous system" may include diseases of the central nervous system, in particular neurodegenerative disorders. This term may be used generally herein to refer to any disease or disorder that affects the functioning of the nervous system. Nervous system disorders include disorders of the central nervous system (i.e. brain and spinal cord), peripheral nervous system (i.e. all other neural elements, including the peripheral nerves and the autonomic nerves), and mental health and psychiatric disorders. The disorder may be a neurodegenerative disorder (e.g. a disorder associated with the degeneration of neurons). Such disorders include Parkinson's disease, multiple sclerosis, amyotrophic lateral sclerosis (ALS), Huntington's disease, and Alzheimer's disease. The actual symptoms associated with a nervous system disorder disclosed herein are well known and can be determined by a person of ordinary skill in the art by accounting for factors, including, the location, cause and severity of the nervous system disorder. Examples of symptoms can be wide-ranging but may include inflammation, fatigue, dizziness, headache, malaise, elevated fever and high body temperature, weakness and stiffness in muscles and joints, weight changes, digestive or gastrointestinal problems, low or high blood pressure, irritability, anxiety, depression, blurred, impaired or double vision, ataxia, paralysis, impaired muscle coordination, loss of sensation and speech problems. Treatment using the functional nucleic acid molecules described herein may reduce one or more of these symptoms.

### Therapeutic agents and Functional Nucleic Acid Molecules

According to a first aspect of the invention, there is provided a therapeutic agent for use in a method of treating a disease or disorder of the nervous system, wherein the therapeutic agent increases translation of an endogenous mRNA sequence selected from the group consisting of: an endogenous Nurr1, human GDNF, cRET or GBA mRNA sequence.

According to one aspect of the present invention, there is provided a functional nucleic acid molecule comprising:
at least one target binding sequence comprising a sequence reverse complementary to a target mRNA sequence selected from the group consisting of: aNurr1, human GDNF, cRET or GBA mRNA sequence; and
at least one regulatory sequence comprising an RNA comprising a SINE B2 element, a functionally active fragment of a SINE B2 element, an internal ribosome entry site (IRES) sequence or an IRES derived sequence.

As described herein, the listed targets have been indicated to be involved in the pathology of nervous system diseases, such as neurodegenerative diseases. In one embodiment, the target mRNA sequence is selected from the group consisting of: a Nurr1, cRET or GBA mRNA sequence. In one embodiment, the target mRNA sequence is a GBA mRNA sequence. In one embodiment, the target mRNA sequence is a human GDNF mRNA sequence. In one embodiment, the target mRNA sequence is a cRET mRNA sequence. In one embodiment, the target mRNA sequence is a Nurr1 mRNA sequence.

### Regulatory Sequences

The regulatory sequence has protein translation enhancing efficiency. The increase of the protein translation efficiency indicates that the efficiency is increased as compared to a case where the functional nucleic acid molecule according to the present invention is not present in a system. In one embodiment, expression of the protein encoded by the target mRNA is increased by at least 1.5 fold, such as at least 2 fold. In a further embodiment, expression of the protein encoded by the target mRNA is increased between 1.5 to 3 fold, such as between 1.6 and 2.2 fold.

In one embodiment, the regulatory sequence is located 3' of the target binding sequence. The regulatory sequence may be in a direct or inverted orientation relative to the 5' to 3' orientation of the functional nucleic acid molecule. Reference to "direct" refers to the situation in which the regulatory sequence is embedded (inserted) with the same 5' to 3' orientation as the functional nucleic acid molecule. Instead, "inverted" refers to the situation in which the regulatory sequence is 3' to 5' oriented relative to the functional nucleic acid molecule.

Preferably, the at least one regulatory sequence comprises a sequence with at least 90% sequence identity, preferably at least 95% sequence identity, more preferably 100% sequence identity with a sequence selected from the group consisting of SEQ ID NOs: 1-69. In one embodiment, the at least one regulatory sequence consists of a sequence with at least 90% sequence identity, preferably at least 95% sequence identity, more preferably 100% sequence identity with a sequence selected from the group consisting of SEQ ID NOs: 1-69.

In one embodiment, the regulatory sequence comprises a SINE B2 element or a functionally active fragment of a SINE B2 element. The SINE B2 element is preferably in an inverted orientation relative to the 5' to 3' orientation of the functional nucleic acid molecule, i.e. an inverted SINE B2 element. As mentioned in the definitions section, inverted SINE B2 elements are disclosed and exemplified in WO 2012/133947.

In one embodiment, the at least one regulatory sequence comprises a sequence with at least 90% sequence identity, preferably at least 95% sequence identity, more preferably 100% sequence identity with a sequence selected from the group consisting of SEQ ID NOs: 1-51.

SEQ ID NO: 1 (the 167 nucleotide variant of the inverted SINE B2 element in AS Uchl1) and SEQ ID NO: 2 (the 77 nucleotide variant of the inverted SINE B2 element in AS Uchl1 that includes nucleotides 44 to 120) are particularly preferred.

Other inverted SINE B2 elements and functionally active fragments of inverted SINE B2 elements are SEQ ID NO: 3-51. Experimental data showing the protein translation enhancing efficiency of these sequences is not explicitly shown in the present patent application, but is disclosed in a previous patent application in the name of the same applicant. SEQ ID NOs: 3-51 can therefore also be used as regulatory sequences in molecules according to the present invention.

SEQ ID NOs: 3-6, 8-11, 18, 43-51 are functionally active fragments of inverted SINE B2 transposable element derived from AS Uchl1. The use of functional fragments reduces the size of the regulatory sequence which is advantageous if used in an expression vector (e.g. viral vectors which may be size-limited) because this provides more space for the target sequence and/or expression elements.

SEQ ID NO: 7 is a full length 183 nucleotide inverted SINE B2 transposable element derived from AS Uchl1. SEQ ID NOs: 12-17, 19, 20, 39-42 are mutated functionally active fragments of inverted SINE B2 transposable element derived from AS Uchl1.

SEQ ID NOs: 21-25, 28-38 are different SINE B2 transposable elements. SEQ ID NOs: 26 and 27 are sequences in which multiple inverted SINE B2 transposable element have been inserted.

Alternatively, the regulatory sequence comprises an IRES sequence or an IRES derived sequence. Therefore, in one embodiment, the regulatory sequence comprises an IRES sequence or an IRES derived sequence. Said sequence enhances translation of the target mRNA sequence.

Several IRESs having sequences ranging from 48 to 576 nucleotides have been tested with success, e.g. human Hepatitis C Virus (HCV) IRESs (e.g. SEQ ID NOs: 52 and 53), human poliovirus IRESs (e.g. SEQ ID NOs: 54 and 55), human encephalomyocarditis (EMCV) virus (e.g. SEQ ID NOs: 56 and 57), human cricket paralysis (CrPV) virus (e.g. SEQ ID NOs: 58 and 59), human Apaf-1 (e.g. SEQ ID NOs: 60 and 61), human ELG-1 (e.g. SEQ ID NOs: 62 and 63), human c-MYC (e.g. SEQ ID NOs: 64-67), human dystrophin (DMD) (e.g. SEQ ID NOs: 68 and 69).

Such sequences have been disclosed, defined and exemplified in WO 2019/058304. Preferably, such sequences have at least 90% sequence identity, preferably at least 95% sequence identity, more preferably 100% sequence identity to any of SEQ ID NOs: 52-69.

### Target Binding Sequences

In some embodiments, the at least one target binding sequence comprises a sequence reverse complementary to a target mRNA sequence selected from the group consisting of: human Nurr1, human GDNF, human cRET or human GBA mRNA sequence. In other embodiments, the at least one target binding sequence comprises a sequence reverse complementary to a target mRNA sequence selected from the group consisting of: mouse Nurr1, mouse cRET or mouse GBA mRNA sequence. In one embodiment, the at least one target binding sequence does not comprise a sequence reverse complementary to mouse GDNF.

Human GDNF is a highly conserved neurotropic factor which can promote the survival and differentiation of dopaminergic neurons *in vitro* and is able to prevent the axotomy-induced apoptosis of motor neurons. The encoded protein is processed to a mature secreted form that exists as a homodimer which is a ligand for the product of the RET (rearranged during transfection) protooncogene. In addition to the transcript encoding GDNF, two additional alternative transcripts encoding distinct proteins, referred to as astrocyte-derived trophic factors, have been described. GDNF has also been found to regulate kidney development and spermatogenesis, and it promotes hair follicle formation and cutaneous wound healing by targeting resident hair follicle stem cells (BSCs) in the bulge compartment.

Human cRET is encoded by the RET proto-oncogene and is a receptor tyrosine kinase for members of the glial cell line-derived neurotrophic factor (GDNF) family of extracellular signalling molecules. Loss of function mutations have been associated with the development of Hirschsprung's disease, while gain of function mutations are associated with the development of various types of human cancer, including medullary thyroid carcinoma, multiple endocrine neoplasia type 2A and type 2B, pheochromocytoma and parathyroid hyperplasia. The human RET gene is localized to chromosome 10 (10q11.2) and contains 21 exons which are alternatively spliced to produce 3 different isoforms of the protein. RET51, RET43 and RET9 contain 51, 43 and 9 amino acids in their C-terminal tail, respectively. RET51 is one of the most common isoforms produced from the RET gene. Common to each isoform is a domain structure, with each protein divided into three domains: an N-terminal extracellular domain with four cadherin-like repeats and a cysteine-rich region, a hydrophobic transmembrane domain and a cytoplasmic tyrosine kinase domain, which is split by an insertion of 27 amino acids. Within the cytoplasmic tyrosine kinase domain, there are 16 tyrosine residues (Tyr) in RET9 and 18 in RET51. Tyr1090 and Tyr1096 are present only in the RET51 isoform. Activating point mutations in cRET can give rise to the hereditary cancer syndrome known as multiple endocrine neoplasia type 2 (MEN 2) and there is a high degree of correlation between the position of the point mutation and the phenotype of the disease. Chromosomal rearrangements that generate a fusion gene, resulting in the juxtaposition of the C-terminal region of the RET protein with an N-terminal portion of another protein, can also lead to constitutive activation of the RET kinase. These types of rearrangements are primarily associated with papillary thyroid carcinoma (PTC) and non-small cell lung cancer (NSCLC). Several fusion partners have been described, and the most common ones across both cancer types include KIF5B, CCDC6 and NCOA4.

Nuclear receptor related 1 protein (Nurr1) also known as NR4A2 (nuclear receptor subfamily 4, group A, member 2) is a member of the nuclear receptor family of intracellular transcription factors and in humans is encoded by the NR4A2 gene. Nurr1 plays a key role in the maintenance of the dopaminergic system of the brain and mutations in this gene have been associated with disorders related to dopaminergic dysfunction, including Parkinson's disease and schizophrenia. Several transcript variants encoding distinct isoforms have been identified for this gene, although additional alternate splice variants may exist of which the full-length nature has not yet been determined. Nurr1 is believed to be critical to development of the dopamine phenotype in the midbrain, as knockout mice lack expression of this phenotype. This has been further confirmed by studies showing that when forcing Nurr1 expression in naïve precursor cells, there is complete dopamine phenotype gene expression. Nurr1 induces tyrosine hydroxylase (TH) expression, which causes differentiation into dopaminergic neurons, and Nurr1 has been shown to induce differentiation in central nervous system precursor cells *in vitro.* Therefore, Nurr1 modulation has been suggested for the generation of dopaminergic neurons for Parkinson's disease research. Nurr1 may also have a role in inflammation, in particular in inflammatory disorders caused by dopaminergic neuron disease. Inflammation in the central nervous system can result from activated microglia and other pro-inflammatory factors, such as bacterial lipopolysaccharide (LPS). LPS binds to toll-like receptors (TLR), which induces inflammatory gene expression by promoting signal-dependent transcription factors. It has been shown that Nurr1 protects dopaminergic neurons from LPS-induced inflammation, by reducing inflammatory gene expression in microglia and astrocytes. Furthermore, if a short hairpin for Nurr1 is expressed in microglia and astrocytes, these cells produce inflammatory mediators, such as TNFα, NO synthase and IL-1β, demonstrating that reduced Nurr1 promotes inflammation and leads to cell death of dopaminergic neurons. Nurr1 interacts with the transcription factor complex NF-κB-p65 on inflammatory gene promoters, but these interactions are dependent on other factors. Sumoylation of Nurr1 is required and its co-regulating factor, glycogen synthase kinase 3, needs to be phosphorylated for these interactions to occur. Sumolyated Nurr1 recruits CoREST, a complex made of several proteins that assembles chromatin-modifying enzymes, and the resulting Nurr1/CoREST complex inhibits the transcription of inflammatory genes.

GBA, acid beta-glucocerebrosidase, also known as beta-glucosidase, is a lysosomal enzyme that catalyzes the breakdown of the glycolipid glucosylceramide to ceramide and glucose. Alternative names include GBA1, acid beta-glucosidase, beta-glucosidase, beta-GC, glucocerebrosidase or glucosylceramidase. GBA mutations are thought to reduce the enzymatic function of the acid beta-glucocerebrosidase, impairing lysosomal efficiency and the cellular ability to dispose of pathological alpha-synuclein. Heterozygous mutations of the GBA gene are a major genetic risk factor for Parkinson's disease (PD) and dementia with Lewy bodies (DLB). Homozygous mutations in the GBA gene are also linked to Gaucher's disease (GD), a multi-organ disease that can be divided into at least four types, some associated with neuropathology. Enzyme replacement therapy (including Ceredase^{®} or Cerezyme^{®}) is a therapeutic option but does not address the neuropathic forms of the disease (see Deegan et al. Drug Des. Devel. Ther. (2012) 6: 81-106; Avenali et al. Front Aging Neurosci. (2020)12: 97; and Creese et al. Am. J. Med. Genet. B. Neuropsychiatr. Genet. (2018) 177(2): 232-241).

In one embodiment, the target binding sequence comprises a sequence reverse complementary to a portion of the Nurr1, GDNF, cRET or GBA mRNA sequence that is common to all Nurr1, GDNF, cRET or GBA isoforms. By maintaining the reciprocal levels of all Nurr1, GDNF, cRET or GBA isoforms, the functional nucleic acid molecule is able to induce the best molecular pattern of expression to restore physiological homeostasis. This is not possible with a more conventional gene therapy approaches when only one isoform can be ectopically expressed leading to isoform imbalance.

In WO 2012/133947 it was already shown that the target binding sequence needs to have only about 60% similarity with a sequence reverse complementary to the target mRNA. As a matter of fact, the target binding sequence can even display a large number of mismatches and retain activity.

The target binding sequence comprises a sequence which is sufficient in length to bind to the Nurr1, GDNF, cRET or GBA mRNA transcript. Therefore, the target binding sequence may be at least 10 nucleotides long, such as at least 13 nucleotides long, such as least 14 nucleotides long, such as least 15 nucleotides long, such as least 18 nucleotides long. Furthermore, the target binding sequence may be less than 250 nucleotides long, preferably less than 200 nucleotides long, less than 150 nucleotides long, less than 100 nucleotides long, less than 80 nucleotides long, less than 60 nucleotides long, less than 50 nucleotides long or less than 30 nucleotides long. In one embodiment, the target binding sequence is between 4 and 50 nucleotides in length, such as between 13 and 26 nucleotides long.

In one embodiment, the target binding sequence comprises a sequence reverse complementary to a portion of the Nurr1 mRNA sequence that is at least 18 nucleotides long, such as between 18 and 24 nucleotides long. In another embodiment, the target binding sequence comprises a sequence reverse complementary to a portion of the GDNF mRNA sequence that is at least 14 nucleotides long, such as between 14 and 25 nucleotides long. In another embodiment, the target binding sequence comprises a sequence reverse complementary to a portion of the GBA mRNA sequence that is at least 14 nucleotides long, such as between 14 and 44 nucleotides long. In a further embodiment, the target binding sequence comprises a sequence reverse complementary to a portion of the cRET mRNA sequence that is at least 15 nucleotides long, such as between 15 and 24 nucleotides long.

### Nurr1

The target binding sequence may be designed to hybridise with the 5' UTR of the Nurr1 mRNA sequence. In one embodiment, the sequence is reverse complementary to 0 to 20 nucleotides, such as 0 to 18 or 0 to 14 nucleotides of the 5' UTR. Alternatively, or in combination, the target binding sequence may be designed to hybridise to the CDS of the Nurr1 mRNA sequence. In one embodiment, the sequence is reverse complementary to 0 to 10 nucleotides, such as 0 to 4 or 0 nucleotides of the CDS.

The target binding sequence may be designed to hybridise to a region upstream of an AUG site (start codon), such as a start codon within the CDS, of the Nurr1 mRNA sequence. In one embodiment, the sequence is reverse complementary to 0 to 20 nucleotides, such as 0 to 18 or 0 to 14 nucleotides upstream of the AUG site. Alternatively, or in combination, the target binding sequence may be designed to hybridise to the Nurr1 mRNA sequence downstream of said AUG site. In one embodiment, the sequence is reverse complementary to 0 to 10 nucleotides, such as 0 to 4 or 0 nucleotides of the Nurr1 mRNA sequence downstream of said AUG site.

Preferably, the target binding sequence is at least 18 nucleotides long and comprises, from 3' to 5':
1) a sequence reverse complementary to 0 to 20 nucleotides of the 5' UTR and 0 to 4 nucleotides of the CDS of the Nurr1 mRNA sequence; or
2) a sequence reverse complementary to 0 to 18 nucleotides of the region upstream of an AUG site (start codon) of the Nurr1 mRNA and 0 to 4 nucleotides of the CDS of the Nurr1 mRNA sequence downstream of said AUG site.

In case 1) the coding sequence starts on the first AUG site (M1) of the mRNA. In case 2), the preferred AUG site is that corresponding to an internal start codon, such as methionine 44 (M2 at amino acid position M44) or methionine 64 (M3 at amino acid position M64) in exon 3.

In one embodiment, the target binding sequence comprises a binding domain as described in Table 1, i.e. with a sequence reverse complementary to the nucleotides of the region upstream and downstream of the methionine in the Nurr1 mRNA sequence (i.e. start codon) as indicated.

In a particular embodiment, the target binding sequence is 18 nucleotides long and comprises, from 3' to 5' a sequence reverse complementary to 14 nucleotides of the 5' UTR and 4 nucleotides of the CDS of the Nurr1 mRNA sequence. For example, the target binding sequence may comprise a sequence encoded by the DNA sequence of SEQ ID NO: 138 (i.e. -14/+4 of M1).

In one particular embodiment, the target binding sequence is 18 nucleotides long and comprises, from 3' to 5' a sequence reverse complementary to 14 nucleotides of the region upstream of the AUG site (start codon) and 4 nucleotides of the CDS of the Nurr1 mRNA sequence downstream of said AUG site, for example wherein the AUG site is methionine M2 (corresponding to M44 of the Nurr1 mRNA sequence). For example, the target binding sequence may comprise a sequence encoded by the DNA sequence of SEQ ID NO: 143 (i.e. -14/+4 of M2).

In another particular embodiment, the target binding sequence is 18 nucleotides long and comprises, from 3' to 5' a sequence reverse complementary to 18 nucleotides of the region upstream of an AUG site (start codon) and 0 nucleotides of the CDS of the Nurr1 mRNA sequence downstream of said AUG site, for example wherein the AUG site is methionine M2 (corresponding to M44 of the Nurr1 mRNA sequence). For example, the target binding sequence may comprise a sequence encoded by the DNA sequence of SEQ ID NO: 144 (i.e. -18/+0 of M2).

In a further particular embodiment, the target binding sequence is 22 nucleotides long and comprises, from 3' to 5' a sequence reverse complementary to 18 nucleotides of the region upstream of an AUG site (start codon) and 4 nucleotides of the CDS of the Nurr1 mRNA sequence downstream of said AUG site, for example wherein the AUG site is methionine M2 (corresponding to M44 of the Nurr1 mRNA sequence). For example, the target binding sequence may comprise a sequence encoded by the DNA sequence of SEQ ID NO: 145 (i.e. -18/+4 of M2).

In a yet further particular embodiment, the target binding sequence is 18 nucleotides long and comprises, from 3' to 5' a sequence reverse complementary to 14 nucleotides of the region upstream of an AUG site (start codon) and 4 nucleotides of the CDS of the Nurr1 mRNA sequence downstream of said AUG site, for example wherein the AUG site is methionine M3 (corresponding to M64 of the Nurr1 mRNA sequence). For example, the target binding sequence may comprise a sequence encoded by the DNA sequence of SEQ ID NO: 146 (i.e. -14/+4 of M3).

In another particular embodiment, the target binding sequence is 18 nucleotides long and comprises, from 3' to 5' a sequence reverse complementary to 18 nucleotides of the region upstream of an AUG site (start codon) and 0 nucleotides of the CDS of the Nurr1 mRNA sequence downstream of said AUG site, for example wherein the AUG site is methionine M3 (corresponding to M64 of the Nurr1 mRNA sequence). For example, the target binding sequence may comprise a sequence encoded by the DNA sequence of SEQ ID NO: 147 (i.e. -18/+0 of M3).

### GDNF

The target binding sequence may be designed to hybridise with the 5' UTR of the GDNF mRNA sequence. In one embodiment, the sequence is reverse complementary to 0 to 70 nucleotides, such as 0 to 46, 0 to 44, 0 to 40, 0 to 38, 0 to 31, 0 to 26, 0 to 25, 0 to 21, 0 to 20, 0 to 19, 0 to 18, 0 to 16, 0 to 14 or 0 to 1 nucleotides of the 5' UTR. Alternatively, or in combination, the target binding sequence may be designed to hybridise to the CDS of the GDNF mRNA sequence. In one embodiment, the sequence is reverse complementary to 0 to 46 nucleotides, such as 0 to 25, 0 to 24, 0 to 20, 0 to 18, 0 to 17, 0 to 16, 0 to 14, 0 to 8, 0 to 4, 0 to 1, or 0 nucleotides of the CDS.

The target binding sequence may be designed to hybridise to a region upstream of an AUG site (start codon), such as a start codon within the CDS, of the GDNF mRNA sequence. In one embodiment, the sequence is reverse complementary to 0 to 25 nucleotides, such as 0 to 20, 0 to 18 or 0 to 14 nucleotides upstream of the AUG site. Alternatively, or in combination, the target binding sequence may be designed to hybridise to the GDNF mRNA sequence downstream of said AUG site. In one embodiment, the sequence is reverse complementary to 0 to 10 nucleotides, such as 0 to 4 or 0 nucleotides of the GDNF mRNA sequence downstream of said AUG site.

Preferably, the target binding sequence is at least 14 nucleotides long and comprises, from 3' to 5':
1) a sequence reverse complementary to 0 to 56 nucleotides of the 5' UTR and 0 to 20 nucleotides of the CDS of the GDNF mRNA sequence; or
2) a sequence reverse complementary to 0 to 42 nucleotides of the region upstream of an AUG site (start codon) of the GDNF mRNA and 0 to 4 nucleotides of the CDS of the GDNF mRNA sequence downstream of said AUG site.

In case 1) the coding sequence starts on the first AUG site (M1) of the mRNA. In case 2), the preferred AUG site is that corresponding to an internal start codon, such as methionine 53 (M2 at amino acid position M53) in exon 3, methionine 65 (M3 at amino acid position M65) and/or methionine 83 (M4 at amino acid position M83).

In one embodiment, the target binding sequence comprises a binding domain as described in Table 1, i.e. with a sequence reverse complementary to the nucleotides of the region upstream and downstream of the methionine in the GDNF mRNA sequence (i.e. start codon) as indicated.

In a particular embodiment, the target binding sequence is 40 nucleotides long and comprises, from 3' to 5' a sequence reverse complementary to 40 nucleotides of the 5' UTR and 0 nucleotides of the CDS of the GDNF mRNA sequence. For example, the target binding sequence may comprise a sequence encoded by the DNA sequence of SEQ ID NO: 82 (i.e. - 40/+0 of M1).

In a particular embodiment, the target binding sequence is 18 nucleotides long and comprises, from 3' to 5' a sequence reverse complementary to 14 nucleotides of the 5' UTR and 4 nucleotides of the CDS of the GDNF mRNA sequence. For example, the target binding sequence may comprise a sequence encoded by the DNA sequence of SEQ ID NO: 85 (i.e. - 14/+4 of M1).

In another particular embodiment, the target binding sequence is 21 nucleotides long and comprises, from 3' to 5' a sequence reverse complementary to 1 nucleotide of the 5' UTR and 20 nucleotides of the CDS of the GDNF mRNA sequence. For example, the target binding sequence may comprise a sequence encoded by the DNA sequence of SEQ ID NO: 101 (i.e. -1/+20 of M1).

In a further particular embodiment, the target binding sequence is 18 nucleotides long and comprises, from 3' to 5' a sequence reverse complementary to 1 nucleotide of the 5' UTR and 17 nucleotides of the CDS of the GDNF mRNA sequence. For example, the target binding sequence may comprise a sequence encoded by the DNA sequence of SEQ ID NO: 102 (i.e. -1/+17 of M1).

In one particular embodiment, the target binding sequence is 18 nucleotides long and comprises, from 3' to 5' a sequence reverse complementary to 14 nucleotides of the region upstream of an AUG site (start codon) of the GDNF mRNA and 4 nucleotides of the CDS of the GDNF mRNA sequence downstream of said AUG site, for example wherein the AUG site is methionine M2 (corresponding to M53 of the GDNF mRNA sequence). For example, the target binding sequence may comprise a sequence encoded by the DNA sequence of SEQ ID NO: 94 (i.e. -14/+4 of M2).

### cRET

The target binding sequence may be designed to hybridise with the 5' UTR of the cRET (RET51) mRNA sequence. In one embodiment, the sequence is reverse complementary to 0 to 56 nucleotides, such as 0 to 40, 0 to 38, 0 to 32, 0 to 29, 0 to 24, 0 to 18, 0 to 16, 0 to 15, 0 to 14, 0 to 13, 0 to 11, 0 to 5, 0 to 4, or 0 to 1 nucleotides of the 5' UTR. Alternatively, or in combination, the target binding sequence may be designed to hybridise to the CDS of the cRET (RET51) mRNA sequence. In one embodiment, the sequence is reverse complementary to 0 to 50 nucleotides, such as 0 to 40, 0 to 25, 0 to 23, 0 to 19, 0 to 17, 0 to 14, 0 to 13, 0 to 11, 0 to 9, 0 to 8, 0 to 7, 0 to 6, 0 to 5, 0 to 4, 0 to 3, or 0 to 1 nucleotides of the CDS.

The target binding sequence may be designed to hybridise to a region upstream of an AUG site (start codon), such as a start codon within the CDS, of the cRET mRNA sequence. In one embodiment, the sequence is reverse complementary to 0 to 20 nucleotides, such as 0 to 14, 0 to 5 or 0 to 4 nucleotides upstream of the AUG site. Alternatively, or in combination, the target binding sequence may be designed to hybridise to the cRET mRNA sequence downstream of said AUG site. In one embodiment, the sequence is reverse complementary to 0 to 15 nucleotides, such as 0 to 13, 0 to 11, 0 to 6 or 0 to 4 nucleotides of the cRET mRNA sequence downstream of said AUG site.

Preferably, the target binding sequence is at least 14 nucleotides long and comprises, from 3' to 5':
1) a sequence reverse complementary to 0 to 56 nucleotides of the 5' UTR and 0 to 23 nucleotides of the CDS of the cRET mRNA sequence; or
2) a sequence reverse complementary to 0 to 50 nucleotides of the region upstream of an AUG site (start codon) of the cRET mRNA and 0 to 19 nucleotides of the CDS of the cRET mRNA sequence downstream of said AUG site.

In case 1) the coding sequence starts on the first AUG site (M1) of the mRNA. In case 2), the preferred AUG site is that corresponding to an internal start codon, such as methionine 255 (M2 at amino acid position M255) and/or methionine 370 (M3 at amino acid position M370).

In one embodiment, the target binding sequence comprises a binding domain as described in Table 1, i.e. with a sequence reverse complementary to the nucleotides of the region upstream and downstream of the methionine in the cRET mRNA sequence (i.e. start codon) as indicated.

In a particular embodiment, the target binding sequence is 40 nucleotides long and comprises, from 3' to 5' a sequence reverse complementary to 40 nucleotides of the 5' UTR and 0 nucleotides of the CDS of the cRET mRNA sequence. For example, the target binding sequence may comprise a sequence encoded by the DNA sequence of SEQ ID NO: 110 (i.e. -40/+0 of M1).

In a particular embodiment, the target binding sequence is 18 nucleotides long and comprises, from 3' to 5' a sequence reverse complementary to 14 nucleotides of the 5' UTR and 4 nucleotides of the CDS of the cRET mRNA sequence. For example, the target binding sequence may comprise a sequence encoded by the DNA sequence of SEQ ID NO: 112 (i.e. -14/+4 of M1).

In one particular embodiment, the target binding sequence is 15 nucleotides long and comprises, from 3' to 5' a sequence reverse complementary to 11 nucleotides of the 5' UTR and 4 nucleotides of the CDS of the cRET mRNA sequence. For example, the target binding sequence may comprise a sequence encoded by the DNA sequence of SEQ ID NO: 129 (i.e. -11/+4 of M1).

In another particular embodiment, the target binding sequence is 18 nucleotides long and comprises, from 3' to 5' a sequence reverse complementary to 11 nucleotides of the 5' UTR and 7 nucleotides of the CDS of the cRET mRNA sequence. For example, the target binding sequence may comprise a sequence encoded by the DNA sequence of SEQ ID NO: 130 (i.e. -11/+7 of M1).

In one particular embodiment, the target binding sequence is 18 nucleotides long and comprises, from 3' to 5' a sequence reverse complementary to 14 nucleotides of the region upstream of an AUG site (start codon) of the cRET mRNA and 4 nucleotides of the CDS of the cRET mRNA sequence downstream of said AUG site, for example wherein the AUG site is methionine M2 (corresponding to M255 of the cRET mRNA sequence). For example, the target binding sequence may comprise a sequence encoded by the DNA sequence of SEQ ID NO: 118 (i.e. -14/+4 of M2).

In another embodiment, the target binding sequence is 40 nucleotides long and comprises, from 3' to 5' a sequence reverse complementary to 40 nucleotides of the region upstream of an AUG site (start codon) of the cRET mRNA and 0 nucleotides of the CDS of the cRET mRNA sequence downstream of said AUG site, for example wherein the AUG site is methionine M2 (corresponding to M255 of the cRET mRNA sequence). For example, the target binding sequence may comprise a sequence encoded by the DNA sequence of SEQ ID NO: 119 (i.e. -40/+0 of M2).

### GBA

The target binding sequence may be designed to hybridise with the 5'-untranslated region (5' UTR) of the GBA mRNA sequence. In one embodiment, the sequence is reverse complementary to 0 to 56 nucleotides, such as 0 to 40, 0 to 32, 0 to 20, 0 to 14 or 0 to 9 nucleotides of the 5' UTR. Alternatively, or in combination, the target binding sequence may be designed to hybridise to the coding sequence (CDS) of the GBA mRNA sequence. In one embodiment, the sequence is reverse complementary to 0 to 20 nucleotides, such as 0 to 18, 0 to 14, 0 to 12, 0 to 9 or 0 to 4 nucleotides of the CDS.

The target binding sequence may be designed to hybridise to a region upstream of an AUG site (start codon), such as a start codon within the CDS, of the GBA mRNA sequence. In one embodiment, the sequence is reverse complementary to 0 to 20 nucleotides, such as 0 to 14 nucleotides upstream of the AUG site. Alternatively, or in combination, the target binding sequence may be designed to hybridise to the GBA mRNA sequence downstream of said AUG site. In one embodiment, the sequence is reverse complementary to 0 to 10 nucleotides, such as 0 to 4 nucleotides of the GBA mRNA sequence downstream of said AUG site.

Preferably, the target binding sequence is at least 14 nucleotides long and comprises, from 3' to 5':
1) a sequence reverse complementary to 0 to 56 nucleotides of the 5' UTR and 0 to 14 nucleotides of the CDS of the GBA mRNA sequence; or
2) a sequence reverse complementary to 0 to 40 nucleotides of the region upstream of an AUG site (start codon) of the GBA mRNA and 0 to 12 nucleotides of the CDS of the GBA mRNA sequence downstream of said AUG site.

In case 1) the coding sequence starts on the first AUG site (M1) of the mRNA. In case 2), the preferred AUG site is that corresponding to an internal start codon. In the context of referencing a sequence reverse complementary to a region in the 5' UTR and the CDS, this is preferably anchored around the AUG site, i.e. the region in the 5' UTR is directly upstream of the AUG site of the target mRNA. For example, reference to a target binding sequence that is "-40/+4 of M1" refers to a target binding sequence that is reverse complementary to the 40 nucleotides within the 5' UTR upstream of the AUG site (-40) and the 4 nucleotides within the CDS downstream of the AUG site (+4).

In one embodiment, the target binding sequence comprises a binding domain as described in Table 1, i.e. with a sequence reverse complementary to the nucleotides of the region upstream and downstream of the methionine in the GBA mRNA sequence (i.e. start codon) as indicated.

Preferably, the target binding sequence is between 14 and 44 nucleotides long and comprises, from 3' to 5', a sequence reverse complementary to up to 40 nucleotides of the region upstream of a start codon of the GBA mRNA sequence and up to 4 nucleotides of the GBA mRNA sequence downstream of said start codon. The region upstream of the start codon may be within the 5' UTR (if the start codon is the first AUG site, i.e. M1) or within the CDS (if the start codon is an internal start codon, such as M2 at amino acid position M21, M3 at amino acid position M88, M6 at amino acid position M162 or M9 at amino acid position M400).

In a particular embodiment, the target binding sequence is 44 nucleotides long and comprises, from 3' to 5' a sequence reverse complementary to 40 nucleotides of the 5' UTR and 4 nucleotides of the CDS of the GBA mRNA sequence. For example, the target binding sequence may comprise a sequence encoded by the DNA sequence of SEQ ID NO: 152 (i.e. -40/+4 of M1).

In another embodiment, the target binding sequence is 14 nucleotides long and comprises, from 3' to 5' a sequence reverse complementary to 14 nucleotides of the 5' UTR and 0 nucleotides of the CDS of the GBA mRNA sequence. For example, the target binding sequence may comprise a sequence encoded by the DNA sequence of SEQ ID NO: 153 (i.e. -14/+0 of M1).

In another embodiment, the target binding sequence is 40 nucleotides long and comprises, from 3' to 5' a sequence reverse complementary to 40 nucleotides of the region upstream of an AUG site (start codon) of the GBA mRNA and 0 nucleotides of the CDS of the GBA mRNA sequence downstream of said AUG site, wherein the AUG site is methionine M2 (corresponding to M21 of the GBA mRNA sequence). For example, the target binding sequence may comprise a sequence encoded by the DNA sequence of SEQ ID NO: 159 (i.e. -40/+0 of M2).

In one embodiment, the target binding sequence comprises a sequence with at least 75% sequence identity, preferably at least 90% sequence identity, more preferably at least 95% sequence identity, even more preferably 100% sequence identity to any of SEQ ID NOs: 70-157, preferably SEQ ID NOs: 70, 75-79, 82, 85, 94, 101, 102, 110, 112, 118, 119, 129, 130, 142, 143 or 149. In a further embodiment, the target binding sequence consists of a sequence with at least 75% sequence identity, preferably at least 90% sequence identity, more preferably at least 95% sequence identity, even more preferably 100% sequence identity to any of SEQ ID NOs: 70-157, preferably SEQ ID NOs: 70, 75-79, 82, 85, 94, 101, 102, 110, 112, 118, 119, 129, 130, 142, 143 or 149.

More specifically, SEQ ID NOs: 70-79 relate to target binding sequences directed to human Nurr1 isoforms, SEQ ID NOs: 80-105 relate to target binding sequences directed to human GDNF isoforms, SEQ ID NOs: 106-137 relate to target binding sequences directed to human cRET isoforms, SEQ ID NOs: 138-157 relate to target binding sequences directed to human GBA isoforms. However, as will be appreciated from the sequence identity of human GBA, GDNF, cRET and Nurr1 isoforms to those of, for example, mouse and rhesus macaque *(Macaca mulatta),* the target binding sequences presented herein may have cross-reactivity with other species.

For example, human Nurr1 mRNA has 98.70% sequence identity across its entire length with *Macaca mulatta* Nurr1 mRNA and 91.82% sequence identity with mouse Nurr1 mRNA. Thus, in a yet further embodiment, the target binding sequence comprising or consisting of any of SEQ ID NOs: 70-79 binds *Macaca mulatta* Nurr1 and/or mouse Nurr1. In another example, human GDNF mRNA has 95.61% sequence identity across its entire length with *Macaca mulatta* GDNF mRNA. Thus, in another embodiment, the target binding sequence comprising or consisting of any of SEQ ID NOs: 80-105 binds *Macaca mulatta* GDNF. In a further example, human cRET mRNA has 96.41% sequence identity across its entire length with *Macaca mulatta* cRET mRNA and 79.20% sequence identity with mouse cRET mRNA. Thus, in a further embodiment, the target binding sequence comprising or consisting of any of SEQ ID NOs: 106-137 binds *Macaca mulatta* cRET and/or mouse cRET. In a yet further example, human GBA mRNA has 96.73% sequence identity across its entire length with *Macaca mulatta* GBA mRNA and 80.42% sequence identity with mouse GBA mRNA. Thus, in a yet further embodiment, the target binding sequence comprising or consisting of any of SEQ ID NOs: 138-157 binds *Macaca mulatta* GBA and/or mouse GBA.

In one embodiment, the functional nucleic acid molecules provided herein are chemically modified. The term "modification" or "chemical modification" refers to a structural change in, or on, the most common, natural ribonucleotides: adenosine, guanosine, cytidine, or uridine ribonucleotides. Chemical modifications may be changes in or on a nucleobase (i.e. a chemical base modification), or in or on a sugar (i.e. a chemical sugar modification). The chemical modifications may be introduced co-transcriptionally (e.g. by substitution of one or more nucleotides with a modified nucleotide during synthesis), or post-transcriptionally (e.g. by the action of an enzyme).

Chemical modifications are known in the art, for example as described in The RNA Modification Database provided by The RNA Institute (https://mods.rna.albany.edu/mods/). Many modifications occur in nature, such as chemical modifications to natural transfer RNAs (tRNAs), which include, for example: 2'-O-Methyl (such as 2'-O-Methyladenosine, 2'-O-Methylguanosine and 2'-O-Methylpseudouridine), 1-Methyladenosine, 2-Methyladenosine, 1-Methylguanosine, 7-Methylguanosine, 2-Thiocytidine, 5-Methylcytidine, 5-Formylcytidine, Pseudouridine, Dihydrouridine, or the like.

### Structural Features

The functional nucleic acid molecule may comprise more than one regulatory sequence, which can be the same sequence repeated more than once, or a different regulatory sequence (i.e. a different SINE B2 element/functionally active fragment of a SINE B2 element/an IRES sequence/an IRES derived sequence).

The at least one target binding sequence and the at least one regulatory sequence are preferably connected by at least one spacer/linker sequence. SEQ ID NOs: 203 or 204 are non-limiting examples of the spacer/linker sequence that may be used.

The functional nucleic acid molecule of the present invention is preferably a circular molecule. This conformation leads to a much more stable molecule that is degraded with greater difficulty within the cell (exonucleases cannot degrade circular molecules) and therefore remains active for a longer time.

Furthermore, the functional nucleic acid molecule may optionally comprise a non-coding 3' tail sequence, which e.g. includes restriction sites useful for cloning the molecule in appropriate plasmids.

In one embodiment, the functional nucleic acid molecule comprises a 3'-polyadenylation (polyA) tail. A "3'-polyA tail" refers to a long chain of adenine nucleotides added to the 3'-end of the transcription which provides stability to the RNA molecule and can promote translation.

In one embodiment the functional nucleic acid molecule comprises a 5'-cap. A "5'-cap" refers to an altered nucleotide at the 5'-end of the transcript which provides stability to the molecule, particularly from degradation from exonucleases, and can promote translation.

It should be noted that the functional nucleic acid molecules can enhance translation of the target gene of interest with no effects on mRNA quantities of the target gene. Therefore, they can successfully be used as molecular tools to validate gene function in cells as well as to implement the pipelines of recombinant protein production.

### DNA Molecules and Vectors

According to a further aspect of the invention, there is provided a DNA molecule encoding any of the functional nucleic acid molecules disclosed herein. According to a further aspect of the invention, there is provided an expression vector comprising said DNA molecule.

Exemplary expression vectors are known in the art and may include, for example, plasmid vectors, viral vectors (for example adenovirus, adeno-associated virus, retrovirus or lentivirus vectors), phage vectors, cosmid vectors and the like. The choice of expression vector may be dependent upon the type of host cell to be used and the purpose of use. In particular the following plasmids have been used for efficient expression of functional nucleic acid molecules:
Mammalian expression plasmids:

| | |
|---|---|
| Plasmid Name: | pCDNA3.1 (-) |
| Expression: | CMV promoter |
| | BGH poly(A) terminator |
| Plasmid Name: | pDUAL-eGFPA (modified from peGFP-C2) |
| Expression: | H1 promoter |
| | BGH poly(A) terminator |
| Plasmid Name: | pCS2+ link (modified from pCS2+) |
| Expression: | CMV IE94 promoter |
| | SV40 poly(A) signal |

Viral vectors:

| | |
|---|---|
| Vector Name: | pAAV |
| Virus: | Adeno-Associated Virus |
| Expression: | CAG promoter / CMV enhancer |
| | SV40 late poly(A) terminator |
| Vector Name: | rcLV -TetOne-Puro |
| Virus: | Lentivirus (3rd generation) |
| Expression: | LTR-TREt (Tre-Tight) promoter (doxycycline-inducible expression) |
| | BGH poly(A) terminator |
| Vector Name: | pLPCX-link |
| Virus: | Retrovirus (3rd generation) |
| Expression: | CMV |

It should be noted that any promoter may be used in the vector and will work just as well as those mentioned above.

### Compositions

The present invention also relates to compositions comprising the functional nucleic acid molecules or the DNA molecules described herein. The composition may comprise components which enable delivery of said functional nucleic acid molecules by viral vectors (AAV, lentivirus and the like) and non-viral vectors (nanoparticles, lipid particles and the like).

The functional nucleic acid molecule of the invention may also be administered as naked or unpackaged RNA. Alternatively, the functional nucleic acid molecule may be administered as part of a composition, for example compositions comprising a suitable carrier. In certain embodiments, the carrier is selected based upon its ability to facilitate the transfection of a target cell with one or more functional nucleic acid molecules.

According to a further aspect of the invention, there is provided the functional nucleic acid molecule, DNA molecule, expression vector or the composition as defined herein for use as a medicament.

It will be understood that the functional nucleic acid molecules of the invention find use in increasing the level of Nurr1, GDNF, cRET or GBA protein within a cell. Nurr1, GDNF, cRET and GBA have functions in the dopaminergic system of the brain, therefore according to a further aspect of the invention there is provided the functional nucleic acid molecule, the DNA molecule or the composition as defined herein, for use in a method of treating a disease or disorder of the nervous system, such as the central nervous system.

According to one aspect of the invention, there is provided a functional nucleic acid molecule for use in a method of treating a disease or disorder of the nervous system, wherein the functional nucleic acid molecule increases translation of an endogenous mRNA sequence selected from the group consisting of: a Nurr1, GDNF, cRET or GBA mRNA sequence.

The above said functional nucleic acid molecules, DNA molecules and/or compositions are used as medicaments, preferably for treating a neurodegenerative disorder, such as Parkinson's disease.

Therefore, in a further embodiment, the disease or disorder of the nervous system is a neurodegenerative disorder, such as Parkinson's disease. Parkinson's disease is one of the most common neurodegenerative disorders and is caused by the loss of dopamine neurons of the substantia nigra pars compacta (SNpc). The main symptoms are well known and include motor-related defects, such as rigidity and tremor. Although Parkinson's disease may be familial (about 5% of cases), most patients are sporadic.

According to a further aspect of the invention, there is provided the use of the functional nucleic acid molecule (or DNA molecule, expression vector or composition) as defined herein for the manufacture of a medicament for the treatment of a disease or disorder of the nervous system, such as a neurodegenerative disorder, for example Parkinson's disease.

### Therapeutic Uses and Methods

According to a further aspect of the invention, there is provided a method for enhancing protein translation of Nurr1, GDNF, cRET or GBA mRNA in a cell comprising administering the functional nucleic acid molecule, DNA molecule, expression vector or composition as defined herein to the cell. Preferably the cell is a mammalian cell, such as a human, mouse or rhesus monkey cell. Most preferably, the cell is a human cell.

According to a further aspect of the invention, there is provided a method for increasing the protein synthesis efficiency of target protein selected from the group consisting of: a Nurr1, GDNF, cRET or GBA protein, in a cell comprising administering the functional nucleic acid molecule, DNA molecule, expression vector or composition as defined herein, to the cell.

Thus, in some embodiments, the translation of endogenous human Nurr1, GDNF, cRET or GBA mRNA is increased. In other embodiments, the translation of mouse Nurr1, cRET or GBA mRNA is increased.

The methods described herein may comprise transfecting into a cell the functional nucleic acid molecule, DNA molecule or expression vector as defined herein. The functional nucleic acid molecule, DNA molecule or expression vector may be administered to target cells using methods known in the art and include, for example, microinjection, lipofection, electroporation, using calcium phosphate, self-infection by the vector or transduction of a virus. Suitable methods for gene delivery are known in the art, for example those described in Sung & Kim (2019) Biomater. Res. 23: 8.

The functional nucleic acid molecules as described herein (e.g. a SINEUP) might be used to directly compensate a precise deficiency in the expression of the protein it targets. The molecules can be designed to increase a target protein level in a disease where the target protein is mutated and the resulting low level of target protein causes the pathology. Such a deficiency could be the result of a genetic mutation leading to haploinsufficiency. In that model, the SINEUP is directly restoring the target protein level where the level is a cause of the disease.

However, some targets (e.g. Nurr1, GDNF and cRET as described herein) are also important "controllers" of beneficial pathways in the CNS, such as pathways for neuroprotection, neurorestoration, neuroregeneration, or for controlling neuroinflammation. They are neurotrophic factors, growth factors, receptors or transcription factors, that are key in transmission of neuroprotective signals. The functional nucleic acid molecules as described herein which boost these proteins could be therapeutically beneficial even though the lack of expression of the proteins themselves might not be the actual root cause of the disease. Such SINEUPs could be used to boost the expression of factors chosen for their functional role in supporting healthy cell function, cell survival or cell repair mechanism, independently of whether that particular factor is specifically mutated or depleted in the indication.

Such molecules may therefore be referred to as "beneficial" SINEUPs and would have a wider indication than the those previously designed to specifically repair a protein deficiency caused by a mutation. For example, therapies aimed to replenish the level of neurotrophic factors such as GDNF may provide important neuronal support in a variety of neurodegenerative pathologies including Alzheimer's (AD), Parkinson's (PD), Huntington's (HD), amyotrophic lateral sclerosis (ALS) or Rett Syndrome (Allen et al. (2013) Pharmacol. Ther. 138 (2): 155-175).

In one embodiment, the cell is Nurr1, GDNF, cRET or GBA haploinsufficient, i.e. wherein the presence of a variant allele in a heterozygous combination results in the amount of product generated by the single wild-type gene is not sufficient for complete or normal function. Generally, haploinsufficiency is a condition that arises when the normal phenotype requires the protein product of both alleles, and reduction to 50% or less of gene function results in an abnormal phenotype.

Methods of the invention result in increased levels of Nurr1, GDNF, cRET or GBA protein in a cell and therefore find use, for example, in methods of treatment for diseases which are associated with Nurr1, GDNF, cRET or GBA defects (i.e. reduced protein levels and/or loss-of-function mutations of these genes). Methods of the invention find particular use in diseases caused by a quantitative decrease in the predetermined, normal protein level. Methods of the invention can be performed *in vitro, ex vivo* or *in vivo.*

Thus, according to one particular aspect of the invention, there is provided a functional nucleic acid molecule for use in a method of treating a disease or disorder of the nervous system (such as the central nervous system, in particular a neurodegenerative disorder) wherein the functional nucleic acid molecule increases translation of endogenous mRNA sequence selected from the group consisting of: a Nurr1, GDNF, cRET or GBA mRNA sequence.

According to a further aspect of the invention, there is provided a method of treating a disease or disorder of the nervous system comprising administering a therapeutic agent that increases translation of an endogenous mRNA sequence selected from the group consisting of: an endogenous Nurr1, GDNF, cRET or GBA mRNA sequence.

According to a further aspect of the invention, there is provided a method of treating a disease or disorder of the nervous system (such as a neurodegenerative disorder, for example Parkinson's disease), comprising administering a therapeutically effective amount of the functional nucleic acid molecule, DNA molecule, expression vector or composition as defined herein.

Gene therapy in neurological diseases, such as neurodegenerative disorders including Parkinson's disease, is challenging because of the requirement to cross the blood-brain barrier (BBB). The BBB is a highly selective semipermeable barrier which prevents solutes in the circulating blood from non-selectively crossing into the extracellular fluid of the central nervous system. Thus, in one embodiment, the functional nucleic acid molecule, DNA molecule or expression vector as defined herein is comprised in a blood-brain barrier-crossing vector, such as a neurotropic viral vector. In a further embodiment, the composition as defined herein comprises a neurotropic viral vector. In certain embodiments, the neurotropic viral vector is an adeno-associated viral vector, such as AAV9.

In one embodiment the therapeutically effective amount is administered in the nervous system, such as the central nervous system, in particular to the brain.

Homozygous mutations in the GBA gene are also linked to Gaucher's disease (GD), a multi-organ disease that is generally classed as a metabolic disorder, in particular a lysosomal storage disorder. GD can be divided into at least four types, some of which are associated with neuropathology (Gaucher disease type 2, also known as acute neuronopathic Gaucher disease, and Gaucher disease type 3, also known as chronic neuronopathic Gaucher disease). Thus, according to one particular aspect of the invention, there is provided a functional nucleic acid molecule for use in a method of treating Gaucher's disease wherein the functional nucleic acid molecule increases translation of endogenous GBA mRNA sequence.

According to a further aspect of the invention, there is provided a method of treating Gaucher's disease comprising administering a therapeutic agent that increases translation of an endogenous GBA mRNA sequence.

According to a further aspect of the invention, there is provided a method of treating Gaucher's disease, comprising administering a therapeutically effective amount of the functional nucleic acid molecule, DNA molecule, expression vector or composition as defined herein, wherein the target mRNA is a GBA mRNA sequence.

### Model systems

Genetic analysis of familiar Parkinson's Disease (PD) has established that the presence of SNCA gene duplication is sufficient to cause PD in humans. In idiopathic PD, subtle rather than large cellular increased α-synuclein expression is found in compromised neurons. These observations suggest that an increase of two-fold of α-synuclein expression throughout the lifetime of an individual is able to provoke both motor and non-motor symptoms of the disease. Current technologies used to establish non-human animal models of PD take advantage of neurochemical intoxications or of ectopic expression of mutant α-synuclein by viral delivery of its cDNA into the brain. In the latter case, the level of expression is usually much higher and less controlled than the one occurring in patients. Described herein is a model of PD which takes advantage of viral delivery of SINEUP-SNCA by using a long non-coding RNA antisense to the endogenous SNCA transcript (microSINEUP-SNCA). SINEUPs are able to increase translation and therefore protein levels of the target gene in the two-fold range, therefore mimicking the expression dynamics naturally occurring in PD patients. This approach will allow studying the effects of increased α-synuclein expression in neuronal cells, thereby testing the differential contribution of dopaminergic subpopulations on motor and affective (anxiety/depression) symptoms of PD, respectively.

Therefore, according to one aspect of the invention, there is provided a method of producing a non-human animal model of PD, particularly a non-human primate (NHP) model of PD, comprising: administering a functional nucleic acid molecule comprising: at least one target binding sequence comprising a sequence reverse complementary to a SNCA mRNA sequence; and at least one regulatory sequence comprising a SINE B2 element, a functionally active fragment of a SINE B2 element, an IRES sequence or an IRES derived sequence, to a non-human animal. Following administration of the functional nucleic acid molecule, the non-human animal will exhibit symptoms of Parkinson's disease. The functional nucleic acid molecule enhances protein translation of SNCA mRNA thereby reproducing the cellular impact of α-synuclein accumulation which is associated with comorbid anxiety/depression-like behaviour and motor deficits. Symptoms of PD would include, but are not limited to, progressive development of classic PD pathology (i.e. loss of neurons), loss of Dorsal Raphe Nucleus Dopamine (DRNDA) neurons and associated anxiety/depression-like behaviour.

Embodiments described herein in relation to functional nucleic acid molecules, DNA molecules, expression vectors or compositions may be applied to the SNCA mRNA sequence. For example, the functional nucleic acid molecule may be administered in any pharmaceutically acceptable carrier and any route of administration as discussed herein. It will be understood that effective amounts of the functional nucleic acid molecule will vary with the route of administration, and the severity of PD disease state desired.

According to a further aspect of the invention, there is provided a method for enhancing protein translation of SNCA mRNA in a cell comprising administering the functional nucleic acid molecule, DNA molecule, expression vector or composition as defined herein to the cell. Preferably the cell is a non-human, mammalian cell, such as a non-human primate or mouse cell.

According to a further aspect of the invention, there is provided a method for increasing the protein synthesis efficiency of SNCA in a cell comprising administering the functional nucleic acid molecule, DNA molecule, expression vector or composition as defined herein, to the cell.

In some embodiments, the translation of endogenous non-human primate SNCA mRNA is increased. In other embodiments, the translation of mouse SNCA mRNA is increased.

In one embodiment, the target binding sequence comprises a sequence reverse complementary to a portion of the SNCA mRNA sequence that is at least 14 nucleotides long, such as between 14 and 70 nucleotides long.

The target binding sequence may be designed to hybridise with the 5'-untranslated region (5' UTR) of the SNCA mRNA sequence. In one embodiment, the sequence is reverse complementary to 0 to 54 nucleotides, such as 0 to 40, 0 to 37, 0 to 32, 0 to 30, 0 to 26, 0 to 25, 0 to 20, 0 to 18, 0 to 16, 0 to 15, 0 to 14 or 0 to 6 nucleotides of the 5' UTR. Alternatively, or in combination, the target binding sequence may be designed to hybridise to the coding sequence (CDS) of the SNCA mRNA sequence. In one embodiment, the sequence is reverse complementary to 0 to 46 nucleotides, such as 0 to 44, 0 to 40, 0 to 37, 0 to 18, 0 to 16, 0 to 14, 0 to 9, 0 to 8, 0 to 4 or 0 to 1 nucleotides of the CDS.

The target binding sequence may be designed to hybridise to a region upstream of an AUG site (start codon), such as a start codon within the CDS, of the SNCA mRNA sequence. In one embodiment, the sequence is reverse complementary to 0 to 20 nucleotides, such as 0 to 14 nucleotides upstream of the AUG site. Alternatively, or in combination, the target binding sequence may be designed to hybridise to the SNCA mRNA sequence downstream of said AUG site. In one embodiment, the sequence is reverse complementary to 0 to 10 nucleotides, such as 0 to 4 nucleotides of the SNCA mRNA sequence downstream of said AUG site.

Preferably, the target binding sequence is at least 14 nucleotides long and comprises, from 3' to 5':
1) a sequence reverse complementary to 0 to 54 nucleotides of the 5' UTR and 0 to 16 nucleotides of the CDS of the SNCA mRNA sequence; or
2) a sequence reverse complementary to 0 to 40 nucleotides of the region upstream of an AUG site (start codon) of the SNCA mRNA and 0 to 37 nucleotides of the CDS of the SNCA mRNA sequence downstream of said AUG site.

In case 1) the coding sequence starts on the first AUG site (M1) of the mRNA. In case 2), the preferred AUG site is that corresponding to an internal start codon. In the context of referencing a sequence reverse complementary to a region in the 5' UTR and the CDS, this is preferably anchored around the AUG site, i.e. the region in the 5' UTR is directly upstream of the AUG site of the target mRNA.

Preferably, the target binding sequence is 18 nucleotides long and comprises, from 3' to 5', a sequence reverse complementary to 14 nucleotides of the region upstream of a start codon of the SNCA mRNA sequence and 4 nucleotides of the SNCA mRNA sequence downstream of said start codon. The region upstream of the start codon may be within the 5' UTR (if the start codon is the first AUG site, i.e. M1) or within the CDS (if the start codon is an internal start codon, such as M2 at amino acid position M5, M3 at amino acid position M100 and/or M5 at amino acid position M127).

In one embodiment, the target binding sequence comprises a binding domain as described in Table 7, i.e. with a sequence reverse complementary to the nucleotides of the region upstream and downstream of the methionine in the SNCA mRNA sequence (i.e. start codon) as indicated.

In a particular embodiment, the target binding sequence is 40 nucleotides long and comprises, from 3' to 5' a sequence reverse complementary to 40 nucleotides of the 5' UTR and 0 nucleotides of the CDS of the SNCA mRNA sequence. For example, the target binding sequence may comprise a sequence encoded by the DNA sequence of SEQ ID NO: 172 (i.e. -40/+0 of M1).

In a particular embodiment, the target binding sequence is 14 nucleotides long and comprises, from 3' to 5' a sequence reverse complementary to 14 nucleotides of the 5' UTR and 0 nucleotides of the CDS of the SNCA mRNA sequence. For example, the target binding sequence may comprise a sequence encoded by the DNA sequence of SEQ ID NO: 174 (i.e. -14/+0 of M1).

In a particular embodiment, the target binding sequence is 14 nucleotides long and comprises, from 3' to 5' a sequence reverse complementary to 14 nucleotides of the 5' UTR and 4 nucleotides of the CDS of the SNCA mRNA sequence. For example, the target binding sequence may comprise a sequence encoded by the DNA sequence of SEQ ID NO: 175 (i.e. -14/+4 of M1).

In one particular embodiment, the target binding sequence is 18 nucleotides long and comprises, from 3' to 5' a sequence reverse complementary to 14 nucleotides of the region upstream of an AUG site (start codon) of the SNCA mRNA and 4 nucleotides of the CDS of the SNCA mRNA sequence downstream of said AUG site, for example wherein the AUG site is methionine M2 (corresponding to M5 of the SNCA mRNA sequence). For example, the target binding sequence may comprise a sequence encoded by the DNA sequence of SEQ ID NO: 183 (i.e. -14/+4 of M2).

In another embodiment, the target binding sequence is 40 nucleotides long and comprises, from 3' to 5' a sequence reverse complementary to 40 nucleotides of the region upstream of an AUG site (start codon) of the SNCA mRNA and 0 nucleotides of the CDS of the SNCA mRNA sequence downstream of said AUG site, for example wherein the AUG site is methionine M2 (corresponding to M5 of the SNCA mRNA sequence). For example, the target binding sequence may comprise a sequence encoded by the DNA sequence of SEQ ID NO: 184 (i.e. -40/+0 of M2).

In a yet further example, human SNCA mRNA has 92.91% sequence identity across its entire length with *Macaca mulatta* SNCA mRNA and 83.73% sequence identity with mouse SNCA mRNA. Thus, in a yet further embodiment, the target binding sequence comprising or consisting of any of SEQ ID NOs: 168-190 binds *Macaca mulatta* SNCA and/or mouse SNCA.

In one embodiment, the target binding sequence comprises a sequence with at least 75% sequence identity, preferably at least 90% sequence identity, more preferably at least 95% sequence identity, even more preferably 100% sequence identity to any of SEQ ID NOs: 158-202, preferably SEQ ID NOs: 158-172, 174, 175, 177-179, 184, 186, 187, 195, 196, 200 or 201, in particular SEQ ID NOs: 158-172, 174, 175, 177-179. In a further embodiment, the target binding sequence consists of a sequence with at least 75% sequence identity, preferably at least 90% sequence identity, more preferably at least 95% sequence identity, even more preferably 100% sequence identity to any of SEQ ID NOs: 158-202, preferably SEQ ID NOs: 158-172, 174, 175, 177-179, 184, 186, 187, 195, 196, 200 or 201, in particular SEQ ID NOs: 158-172, 174, 175, 177-179.

Animal models generated by methods of the invention may be used to screen potential therapeutic agents in the treatment of Parkinson's disease. The model may be used to determine the efficacy, toxicity, side effects, or mechanism of action, of treatment with such an agent. Once a PD disease state is established in an animal, an active substance or potential therapeutic agent may then be administered to the animal model. The protocol and route of administration will vary according to the mechanism of action and the chemical nature of the active substance and may be determined by those skilled in the art. Animals subjected to the potential therapeutic agent may be assessed by any appropriate method including behavioural, biochemical, and histological assessments, for any possible effects of the potential therapeutic agent and compared to control animals (e.g. PD animals that were not treated).

It will be understood that the embodiments described herein may be applied to all aspects of the invention, i.e. the embodiment described for the functional nucleic acid molecules may equally apply to the claimed methods and so forth.

### CLAUSES

1. A therapeutic agent for use in a method of treating a disease or disorder of the nervous system, wherein the therapeutic agent increases translation of an endogenous mRNA sequence selected from the group consisting of: an endogenous Nurr1, human GDNF, cRET or GBA mRNA sequence.
2. The therapeutic agent for use according to clause 1, wherein the disease or disorder of the nervous system is a disease or disorder of the central nervous system.
3. The therapeutic agent for use according to clause 1 or clause 2, wherein the disease or disorder of the nervous system is a neurodegenerative disease.
4. The therapeutic agent for use according to any one of clauses 1 to 3, wherein the therapeutic agent comprises a functional nucleic acid molecule comprising:
   at least one target binding sequence comprising a sequence reverse complementary to a target mRNA sequence selected from the group consisting of: a Nurr1, GDNF, cRET or GBA mRNA sequence; and
   at least one regulatory sequence comprising a SINE B2 element, a functionally active fragment of a SINE B2 element, an internal ribosome entry site (IRES) sequence or an IRES derived sequence.
5. The therapeutic agent for use according to clause 4, wherein the at least one target binding sequence comprises a sequence reverse complementary to a target mRNA sequence selected from the group consisting of: human Nurr1, human GDNF, human cRET or human GBA mRNA sequence.
6. The therapeutic agent for use according to clause 4, wherein the at least one target binding sequence comprises a sequence reverse complementary to a target mRNA sequence selected from the group consisting of: mouse Nurr1, mouse cRET or mouse GBA mRNA sequence.
7. The therapeutic agent for use according to any one of clauses 4 to 6, wherein the at least one target binding sequence comprises a sequence reverse complementary to a portion of the target mRNA sequence that is common to all isoforms.
8. The therapeutic agent for use according to any one of clauses 4 to 7, wherein the at least one target binding sequence is at least 18 nucleotides long and comprises, from 3' to 5':
   a sequence reverse complementary to 0 to 20 nucleotides of the 5' UTR and 0 to 4 nucleotides of the CDS of the Nurr1 mRNA sequence; or
   a sequence reverse complementary to 0 to 18 nucleotides of the region upstream of an AUG site (start codon) of the Nurr1 mRNA and 0 to 4 nucleotides of the CDS of the Nurr1 mRNA sequence downstream of said AUG site.
9. The therapeutic agent for use according to any one of clauses 4 to 7, wherein the at least one target binding sequence is at least 14 nucleotides long and comprises, from 3' to 5':
   a sequence reverse complementary to 0 to 21 nucleotides of the 5' UTR and 0 to 20 nucleotides of the CDS of the GDNF mRNA sequence; or
   a sequence reverse complementary to 0 to 20 nucleotides of the region upstream of an AUG site (start codon) of the GDNF mRNA and 0 to 4 nucleotides of the CDS of the GDNF mRNA sequence downstream of said AUG site.
10. The therapeutic agent for use according to any one of clauses 4 to 7, wherein the at least one target binding sequence is at least 15 nucleotides long and comprises, from 3' to 5':
   a sequence reverse complementary to 0 to 14 nucleotides of the 5' UTR and 0 to 23 nucleotides of the CDS of the cRET mRNA sequence; or
   a sequence reverse complementary to 0 to 14 nucleotides of the region upstream of an AUG site (start codon) of the cRET mRNA and 0 to 13 nucleotides of the CDS of the cRET mRNA sequence downstream of said AUG site.
11. The therapeutic agent for use according to any one of clauses 4 to 7, wherein the at least one target binding sequence is at least 15 nucleotides long and comprises, from 3' to 5':
   a sequence reverse complementary to 0 to 56 nucleotides of the 5' UTR and 0 to 14 nucleotides of the CDS of the GBA mRNA sequence; or
   a sequence reverse complementary to 0 to 40 nucleotides of the region upstream of an AUG site (start codon) of the GBA mRNA and 0 to 12 nucleotides of the CDS of the GBA mRNA sequence downstream of said AUG site.
12. The therapeutic agent for use according to any one of clauses 4 to 11, wherein the at least one regulatory sequence comprises a sequence with at least 75% sequence identity with a sequence selected from the group consisting of SEQ ID NOs: 1-69.
13. The therapeutic agent for use according to clause 12, wherein the at least one regulatory sequence comprises a sequence with at least 90% sequence identity with a sequence selected from the group consisting of SEQ ID NOs: 1-69.
14. The therapeutic agent for use according to any one of clauses 4 to 13, further comprising at least one linker sequence between the at least one target binding sequence and the at least one regulatory sequence.
15. The therapeutic agent for use according to any one of clauses 1 to 14, wherein the functional nucleic acid molecule is circular.
16. A functional nucleic acid molecule comprising:
   at least one target binding sequence comprising a sequence reverse complementary to a target mRNA sequence selected from the group consisting of: a Nurr1, GDNF, cRET or GBA mRNA sequence; and
   at least one regulatory sequence comprising a SINE B2 element, a functionally active fragment of a SINE B2 element, an IRES sequence or an IRES derived sequence.
17. A DNA molecule encoding the functional nucleic acid molecule according to clause 16.
18. An expression vector comprising the functional nucleic acid molecule according to clause 16 or the DNA molecule according to clause 17.
19. A composition comprising the functional nucleic acid molecule according to clause 16, the DNA molecule according to clause 17 or the expression vector according to clause 18.
20. A method for increasing the protein synthesis efficiency of Nurr1, GDNF, cRET or GBA in a cell comprising administering the functional nucleic acid molecule according clause 16, the DNA molecule according to clause 17 or the expression vector according to clause 18 to the cell.
21. The method according to clause 20, wherein the functional nucleic acid molecule is administered as naked RNA.
22. The method according to clause 20 or clause 21, wherein the cell is Nurr1, GDNF, cRET or GBA haploinsufficient.
23. The functional nucleic acid molecule clause 16, the DNA molecule according to clause 17 or the composition according to clause 19, for use as a medicament.
24. The functional nucleic acid molecule according clause 16, the DNA molecule according to clause 17 or the composition according to clause 19, for use in a method of treating a disease or disorder of the nervous system.
25. The functional nucleic acid molecule for use according to clause 24, wherein the disease or disorder of the nervous system is a disease or disorder of the central nervous system.
26. The functional nucleic acid molecule for use according to clause 24 or clause 25, wherein the disease or disorder of the nervous system is a neurodegenerative disorder.
27. The functional nucleic acid molecule for use according to clause 26, wherein the neurodegenerative disorder is Parkinson's disease.
28. A method of treating a disease or disorder of the nervous system comprising administering a therapeutic agent that increases translation of an endogenous mRNA sequence selected from the group consisting of: an endogenous Nurr1, human GDNF, cRET or GBA mRNA sequence.
29. A method of treating a disease or disorder of the nervous system comprising administering a therapeutically effective amount of the functional nucleic acid molecule according to clause 16, the DNA molecule according to clause 17 or the composition according to clause 19.
30 The method according to clause 28 or clause 29, wherein the disease or disorder of the nervous system is a disease or disorder of the central nervous system.
31. The method according to any one of clauses 28 to 30, wherein the disease or disorder of the nervous system is a neurodegenerative disorder.
32. The method according to clause 31, wherein the neurodegenerative disorder is Parkinson's disease.
33. The method according to any one of clauses 28 to 32, wherein the therapeutically effective amount is administered to the brain.
34. A method of producing a non-human animal model of Parkinson's disease comprising: administering a functional nucleic acid molecule comprising: at least one target binding sequence comprising a sequence reverse complementary to a SNCA mRNA sequence; and at least one regulatory sequence comprising a SINE B2 element, a functionally active fragment of a SINE B2 element, an IRES sequence or an IRES derived sequence, to a non-human animal.
35. The method according to clause 34, wherein the non-human animal is a non-human primate.

The invention will now be illustrated with reference to the following non-limiting examples.

### EXAMPLES

### EXAMPLE 1

Binding domain sequences were designed to target human Nurr1, GDNF, cRET and GBA mRNA (summarised in Table 1) and their off-target binding (i.e. binding to mRNAs other than human Nurr1, GDNF, cRET or GBA mRNA) was analysed *in silico* using the UCSC browser (BLAT tool). An e-value greater than or equal to 2.1 represents low target specificity. The results are presented in Tables 2-5.

**Table 1: Summary of designed binding domains for listed targets**

| **Name** | **Binding domain** | **Methionine in target mRNA** |
|---|---|---|
| NURR1 | | |
| hNURR1 BD1 | -14/+4 | M1 |
| hNURR1 BD2 | -18/+4 | M1 |
| hNURR1 BD3 | -18/+0 | M1 |
| hNURR1 BD4 | -20/+0 | M1 |
| hNURR1 BD5 | -20/+4 | M1 |
| hNURR1 BD6 | -14/+4 | M2 |
| hNURR1 BD7 | -18/+0 | M2 |
| hNURR1 BD8 | -18/+4 | M2 |
| hNURR1 BD9 | -14/+4 | M3 |

| GDNF | | |
|---|---|---|
| hGDNF BD1 | -35/-18 | M1 |
| hGDNF BD2 | -40/-3 | M1 |
| hGDNF BD3 | -40/+0 | M1 |
| hGDNF BD4 | -26/+0 | M1 |
| hGDNF BD5 | -14/+0 | M1 |
| hGDNF BD6 | -14/+4 | M1 |
| hGDNF BD7 | -15/+4 | M1 |
| hGDNF BD8 | -16/+4 | M1 |
| hGDNF BD9 | -27/+4 | M1 |
| hGDNF BD10 | -40/+4 | M1 |
| hGDNF BD11 | -32/+8 | M1 |
| hGDNF BD12 | -56/+14 | M1 |
| hGDNF BD13 | -15/+16 | M1 |
| hGDNF BD14 | -20/+1 | M1 |
| hGDNF BD15 | -14/+4 | M2 |
| hGDNF BD16 | -25/+0 | M2 |
| hGDNF BD17 | -42/+4 | M3 |
| hGDNF BD18 | -14/+4 | M3 |
| hGDNF BD19 | +270/+288 | M4 |
| hGDNF BD20 | -21/+4 | M1 |
| hGDNF BD21 | -21/+0 | M1 |
| hGDNF BD22 | -1/+20 | M1 |
| hGDNF BD23 | -1/+17 | M1 |
| hGDNF BD24 | -14/+0 | M2 |
| hGDNF BD25 | -18/+0 | M2 |
| hGDNF BD26 | -20/+4 | M2 |

| cRET | | |
|---|---|---|
| hRET BD1 | -69/-30 | M1 |
| hRET BD2 | -20/-3 | M1 |
| hRET BD3 | -32/-4 | M1 |
| hRET BD4 | -40/-3 | M1 |
| hRET BD5 | -40/+0 | M1 |
| hRET BD6 | -14/+0 | M1 |
| hRET BD7 | -14/+4 | M1 |
| hRET BD8 | -15/+4 | M1 |
| hRET BD9 | -16/+4 | M1 |
| hRET BD10 | -40/+4 | M1 |
| hRET BD11 | -32/+8 | M1 |
| hRET BD12 | -56/+14 | M1 |
| hRET BD13 | -14/+4 | M2 |
| hRET BD14 | -40/+0 | M2 |
| hRET BD15 | -14/+4 | M3 |
| hRET BD16 | -50/+0 | M3 |
| hRET BD17 | +64/+82 | M1 |
| hRET BD18 | +323/341 | M1 |
| hRET BD19 | +96/+114 | M2 |
| hRET BD20 | +65/+109 | M2 |
| hRET BD21 | -13/+5 | M1 |
| hRET BD22 | +82/+106 | M2 |
| hRET BD23 | -14/+3 | M1 |
| hRET BD24 | -11/+4 | M1 |
| hRET BD25 | -11/+7 | M1 |
| hRET BD26 | -11/+9 | M1 |
| hRET BD27 | -1/+17 | M1 |
| hRET BD28 | -1/+19 | M1 |
| hRET BD29 | -1/+23 | M1 |
| hRET BD30 | -14/+6 | M2 |
| hRET BD31 | -5/+13 | M2 |
| hRET BD32 | -4/+11 | M2 |

| GBA | | |
|---|---|---|
| hGBA BD1 | -77/-59 | M1 |
| hGBA BD2 | -20/+3 | M1 |
| hGBA BD3 | -40/-3 | M1 |
| hGBA BD4 | -40/+0 | M1 |
| hGBA BD5 | -40/+4 | M1 |
| hGBA BD6 | -14/+0 | M1 |
| hGBA BD7 | -14/+4 | M1 |
| hGBA BD8 | -15/+4 | M1 |
| hGBA BD9 | -16/+4 | M1 |
| hGBA BD10 | -32/+8 | M1 |
| hGBA BD11 | -56/+14 | M1 |
| hGBA BD12 | -40/+0 | M2 |
| hGBA BD13 | -14/+4 | M3 |
| hGBA BD14 | -40/+0 | M3 |
| hGBA BD15 | -40/+14 | M3 |
| hGBA BD16 | -14/+4 | M9 |
| hGBA BD17 | +96/+114 | M6 |
| hGBA BD18 | -+91/+109 | M6 |
| hGBA BD19 | -6/+12 | M3 |
| hGBA BD20 | -22/+8 | M6 |

**Table 2: In silico off-target binding analysis of Nurr1 target binding sequences/BDs**

| **Binding Domain** | **Gene** | **Length** | **E-val** | **%ID** | **Match Location** |
|---|---|---|---|---|---|
| hNURR1 BD1 | NR4A2 | 18 | 0.009 | 100.00 | |
| hNURR1 BD2 | NR4A2 | 22 | 1.00E-04 | 100.00 | |
| | NDRG1 | 15 | 1.6 | 100.00 | Intron |
| hNURR1 BD3 | NR4A2 | 18 | 0.009 | 100.00 | |
| | NDRG1 | 15 | 0.54 | 100.00 | Intron |
| hNURR1 BD4 | NR4A2 | 20 | 0.001 | 100.00 | |
| | NDRG1 | 15 | 1.1 | 100.00 | Intron |
| hNURR1 BD5 | NR4A2 | 24 | 8.00E-06 | 100.00 | |
| | NDRG1 | 15 | 1.9 | 100.00 | Intron |
| hNURR1 BD6 | NR4A2 | 18 | 0.009 | 100.00 | |
| | NANOG | 15 | 0.54 | 100.00 | 3'UTR |
| hNURR1 BD7 | NR4A2 | 18 | 0.009 | 100.00 | |
| hNURR1 BD8 | NR4A2 | 22 | 1.00E-04 | 100.00 | |
| | GCN1 | 20 | 0.41 | 95.00 | 3'UTR |
| | NANOG | 15 | 1.6 | 100.00 | 3'UTR |
| hNURR1 BD9 | NR4A2 | 18 | 0.009 | 100.00 | |
| | AC090142.3 | 15 | 0.54 | 100.00 | Processed pseudogene |
| hNURR1 BD10 | NR4A2 | 18 | 0.009 | 100.00 | |
| | DCHS1 | 15 | 0.54 | 100.00 | Exon 14 of 21 |

**Table 3: In silico off-target binding analysis of GDNF target binding sequences/BDs**

| **Binding Domain** | **Gene** | **Length** | **E-val** | **%ID** | **Match Location** |
|---|---|---|---|---|---|
| hGDNF BD1 | GDNF | 18 | 0.009 | 100.00 | |
| hGDNF BD2 | GDNF | 38 | 1.00E-13 | 100.00 | |
| hGDNF BD3 | GDNF | 40 | 8.00E-15 | 100.00 | |
| hGDNF BD4 | GDNF | 26 | 7.00E-07 | 100.00 | |
| hGDNF BD5 | GDNF | 14 | 2.1 | 100.00 | |
| hGDNF BD6 | GDNF | 18 | 0.009 | 100.00 | |
| hGDNF BD7 | GDNF | 19 | 0.003 | 100.00 | |
| hGDNF BD8 | GDNF | 20 | 0.001 | 100.00 | |
| hGDNF BD9 | GDNF | 31 | 1.00E-09 | 100.00 | |
| hGDNF BD10 | GDNF | 44 | 4.00E-17 | 100.00 | |
| hGDNF BD11 | GDNF | 40 | 8.00E-15 | 100.00 | |
| hGDNF BD12 | GDNF | 70 | 3.00E-32 | 100.00 | |
| hGDNF BD13 | GDNF | 31 | 1.00E-09 | 100.00 | |
| hGDNF BD14 | GDNF | 21 | 4.00E-04 | 100.00 | |
| hGDNF BD15 | GDNF | 18 | 0.009 | 100.00 | |
| | ZNF785 | 15 | 0.54 | 100.00 | 3' UTR |
| hGDNF BD16 | GDNF | 25 | 2.00E-06 | 100.00 | |
| hGDNF BD17 | GDNF | 46 | 3.00E-18 | 100.00 | |
| hGDNF BD18 | GDNF | 18 | 0.009 | 100.00 | |
| hGDNF BD19 | GDNF | 18 | 0.009 | 100.00 | |
| | CLVS1 | 15 | 0.54 | 100.00 | Retained intron |
| | LAMTOR3 | 15 | 0.54 | 100.00 | 3' UTR |
| | PRCD | 15 | 0.54 | 100.00 | 3' UTR |
| hGDNF BD20 | GDNF | 25 | 2.00E-06 | 100.00 | |
| hGDNF BD21 | GDNF | 21 | 4.00E-04 | 100.00 | |
| hGDNF BD22 | GDNF | 21 | 4.00E-04 | 100.00 | |
| hGDNF BD23 | GDNF | 18 | 0.009 | 100.00 | |
| hGDNF BD24 | GDNF | 14 | 2.1 | 100.00 | |
| hGDNF BD25 | GDNF | 18 | 0.009 | 100.00 | |
| | RNF207 | 15 | 0.54 | 100.00 | 3' UTR |
| hGDNF BD26 | GDNF | 24 | 8.00E-06 | 100.00 | |
| | RNF207 | 15 | 1.9 | 100.00 | 3' UTR |
| | ZNF785 | 15 | 1.9 | 100.00 | 5' UTR |
| | OR8B8 | 19 | 1.9 | 94.74 | 3' UTR |

**Table 4: In silico off-target binding analysis of cRET target binding sequences/BDs**

| **Binding Domain** | **Gene** | **Length** | **E-val** | **%ID** | **Match Location** |
|---|---|---|---|---|---|
| hRET BD1 | RET | 40 | 8.00E-15 | 100.00 | |
| | CKBP1 | 16 | 1.6 | 100.00 | Processed pseudogene |
| hRET BD2 | RET | 18 | 0.009 | 100.00 | |
| hRET BD3 | RET | 29 | 2.00E-08 | 100.00 | |
| | DNM1P51 | 17 | 0.21 | 100.00 | Processed pseudogene |
| | ABCD1P4 | 16 | 0.82 | 100.00 | Unprocessed pseudogene |
| | ABCD1 P5 | 16 | 0.82 | 100.00 | Unprocessed pseudogene |
| | ABCD1 P2 | 16 | 0.82 | 100.00 | Unprocessed pseudogene |
| | ABCD1 | 16 | 0.82 | 100.00 | Exon10 of 10 |
| hRET BD4 | RET | 38 | 1.00E-13 | 100.00 | |
| | DNM1P51 | 17 | 0.37 | 100.00 | Processed pseudogene |
| | ABCD1P4 | 16 | 1.4 | 100.00 | Unprocessed pseudogene |
| | ABCD1 P5 | 16 | 1.4 | 100.00 | Unprocessed pseudogene |
| | ABCD1 P2 | 16 | 1.4 | 100.00 | Unprocessed pseudogene |
| | ABCD1 | 16 | 1.4 | 100.00 | Exon10 of 10 |
| | KIAA1614 | 16 | 1.4 | 100.00 | 3' UTR |
| hRET BD5 | RET | 40 | 8.00E-15 | 100.00 | |
| | DNM1P51 | 17 | 0.40 | 100.00 | Processed pseudogene |
| | ABCD1 P2 | 16 | 1.6 | 100.00 | Unprocessed pseudogene |
| | ABCD1 P4 | 16 | 1.6 | 100.00 | Unprocessed pseudogene |
| | ABCD1 P5 | 16 | 1.6 | 100.00 | Unprocessed pseudogene |
| | ABCD1 | 16 | 1.6 | 100.00 | Exon10 of 10 |
| | KIAA1614 | 16 | 1.6 | 100.00 | 3' UTR |
| hRET BD6 | NRG2 | 14 | 2.1 | 100.00 | Exon8 of 8 |
| | LRRC75A | 14 | 2.1 | 100.00 | M1 -74/-86 |
| | RET | 14 | 2.1 | 100.00 | |
| hRET BD7 | RET | 18 | 0.009 | 100.00 | |
| hRET BD8 | RET | 19 | 0.003 | 100.00 | |
| | NRG2 | 15 | 0.81 | 100.00 | Exon8 of 8 |
| hRET BD9 | RET | 20 | 0.001 | 100.00 | |
| | NRG2 | 15 | 1.1 | 100.00 | |
| hRET BD10 | RET | 44 | 4.00E-17 | 100.00 | |
| | DNM1P51 | 17 | 0.45 | 100.00 | Processed pseudogene |
| | ABCD1 P5 | 16 | 1.8 | 100.00 | Unprocessed pseudogene |
| | ABCD1 P2 | 16 | 1.8 | 100.00 | Unprocessed pseudogene |
| | ABCD1 | 16 | 1.8 | 100.00 | Exon10 of 10 |
| | KIAA1614 | 16 | 1.8 | 100.00 | 3' UTR |
| hRET BD11 | RET | 40 | 8.00E-15 | 100.00 | |
| | DNM1P51 | 17 | 0.40 | 100.00 | Processed pseudogene |
| | ABCD1P5 | 16 | 1.6 | 100.00 | Unprocessed pseudogene |
| | ABCD1 P2 | 16 | 1.6 | 100.00 | Unprocessed pseudogene |
| | ABCD1 | 16 | 1.6 | 100.00 | Exon10 of 10 |
| hRET BD12 | RET | 70 | 3.00E-32 | 100.00 | |
| | DNM1P51 | 17 | 0.91 | 100.00 | Unprocessed pseudogene |
| hRET BD13 | RET | 18 | 0.009 | 100.00 | |
| | S1PR3 | 16 | 0.14 | 100.00 | 3' UTR |
| | LRCH4 | 16 | 0.14 | 100.00 | Intron |
| | ZDHHC3 | 15 | 0.54 | 100.00 | 3' UTR |
| | ARPIN-AP3S2 | 15 | 0.54 | 100.00 | Exon9 of 10 = M329/M326 |
| | AP3S2 | 15 | 0.54 | 100.00 | Exon5 of 6 - M128/M125 |
| hRET BD14 | RET | 40 | 8.00E-15 | 100.00 | |
| | CCDC102A | 17 | 0.40 | 100.00 | M94 +63/+84 |
| | YY1 | 17 | 0.40 | 100.00 | M83 +73/+95 |
| hRET BD15 | RET | 18 | 0.009 | 100.00 | |
| hRET BD16 | RET | 50 | 1.00E-20 | 100.00 | |
| hRET BD17 | RET | 18 | 0.009 | 100.00 | |
| | SLC30A7 | 15 | 0.54 | 100.00 | 3' UTR |
| hRET BD18 | RET | 18 | 0.009 | 100.00 | |
| hRET BD19 | RET | 18 | 0.009 | 100.00 | |
| | ZCCHC2 | 15 | 0.54 | 100.00 | M1 -304/-286 |
| hRET BD20 | RET | 44 | 4.00E-17 | 100.00 | |
| | SLC6A1 | 20 | 1.8 | 95.00 | Exon4 of 14 - M7 +1/+21 |
| hRET BD21 | RET | 18 | 0.009 | 100.00 | |
| | AC020765.5 | 15 | 0.54 | 100.00 | Unprocessed pseudogene |
| | AC020765.3 | 15 | 0.54 | 100.00 | Unprocessed pseudogene |
| | SULT1A1 | 15 | 0.54 | 100.00 | Intron |
| hRET BD22 | RET | 24 | 8.00E-06 | 100.00 | |
| hRET BD23 | RET | 17 | 0.035 | 100.00 | |
| hRET BD24 | RET | 15 | 0.54 | 100.00 | |
| hRET BD25 | RET | 18 | 0.009 | 100.00 | |
| | AC020765.5 | 15 | 0.54 | 100.00 | Unprocessed pseudogene |
| | SULT1A1 | 15 | 0.54 | 100.00 | Intron |
| hRET BD26 | RET | 20 | 0.001 | 100.00 | |
| | AC020765.5 | 15 | 1.1 | 100.00 | Processed pseudogene |
| | SULT1A1 | 15 | 1.1 | 100.00 | Intron |
| hRET BD27 | RET | 18 | 0.009 | 100.00 | |
| hRET BD28 | RET | 20 | 0.001 | 100.00 | |
| hRET BD29 | RET | 24 | 8.00E-06 | 100.00 | |
| hRET BD30 | RET | 20 | 0.001 | 100.00 | |
| | S1PR3 | 18 | 0.018 | 100.00 | 3'UTR |
| | ZDHHC3 | 17 | 0.070 | 100.00 | 3'UTR |
| | LRCH4 | 16 | 0.27 | 100.00 | Intron |
| | SNX13 | 15 | 1.1 | 100.00 | Intron |
| | ARPIN-AP3S2 | 15 | 1.1 | 100.00 | Exon5 of 6 |
| hRET BD31 | RET | 18 | 0.009 | 100.00 | |
| hRET BD32 | RET | 15 | 0.54 | 100.00 | |

**Table 5: In silico off-target binding analysis of GBA target binding sequences/BDs**

| **Binding Domain** | **Gene** | **Length** | **E-val** | **%ID** | **Match Location** |
|---|---|---|---|---|---|
| hGBA BD1 | GBA | 18 | 0.009 | 100.00 | |
| | BEND4 | 16 | 0.14 | 100.00 | Exon2 of 6 |
| hGBA BD2 | GBA | 18 | 0.009 | 100.00 | |
| hGBA BD3 | GBA | 38 | 1.00E-13 | 100.00 | |
| | GBAP1 | 38 | 2.00E-06 | 92.11 | Unprocessed pseudogene |
| | OR11 J7P | 21 | 1.4 | 95.24 | Unprocessed pseudogene |
| | AC183088.2 | 21 | 1.4 | 95.24 | Unprocessed pseudogene |
| | OR11J2P | 21 | 1.4 | 95.24 | Unprocessed pseudogene |
| hGBA BD4 | GBA | 40 | 8.00E-15 | 100.00 | |
| | GBAP1 | 38 | 2.00E-06 | 92.11 | Unprocessed pseudogene |
| | OR11J7P | 21 | 1.6 | 95.24 | Unprocessed pseudogene |
| | AC183088.2 | 21 | 1.6 | 95.24 | Unprocessed pseudogene |
| | OR11J2P | 21 | 1.6 | 95.24 | Unprocessed pseudogene |
| hGBA BD5 | GBA | 44 | 4.00E-17 | 100.00 | |
| | GBAP1 | 44 | 1.00E-07 | 90.91 | Unprocessed pseudogene |
| | OR11J7P | 21 | 1.8 | 95.24 | Unprocessed pseudogene |
| | AC183088.2 | 21 | 1.8 | 95.24 | Unprocessed pseudogene |
| | OR11J2P | 21 | 1.8 | 95.24 | Unprocessed pseudogene |
| hGBA BD6 | GBA | 14 | 2.1 | 100.00 | |
| hGBA BD7 | GBA | 18 | 0.009 | 100.00 | |
| | NDST1 | 15 | 0.54 | 100.00 | 3' UTR |
| hGBA BD8 | GBA | 19 | 0.003 | 100.00 | |
| | NDST1 | 15 | 0.81 | 100.00 | 3' UTR |
| hGBA BD9 | GBA | 20 | 0.001 | 100.00 | |
| | NDST1 | 15 | 1.1 | 100.00 | 3' UTR |
| hGBA BD10 | GBA | 40 | 8.00E-15 | 100.00 | |
| | GBAP1 | 37 | 7.00E-06 | 91.89 | Unprocessed pseudogene |
| hGBA BD11 | GBA | 70 | 3.00E-32 | 100.00 | |
| | GBAP1 | 70 | 9.00E-23 | 94.29 | |
| hGBA BD12 | GBA | 40 | 8.00E-15 | 100.00 | |
| | GBAP1 | 40 | 5.00E-10 | 95.00 | Unprocessed pseudogene |
| | NPHP1 | 16 | 1.6 | 100.00 | 3' UTR |
| hGBA BD13 | GBA | 18 | 0.009 | 100.00 | |
| hGBA BD14 | GBA | 40 | 8.00E-15 | 100.00 | |
| hGBA BD15 | GBA | 54 | 6.00E-23 | 100.00 | |
| | GBAP1 | 51 | 0.040 | 84.31 | Unprocessed pseudogene |
| hGBA BD16 | GBA | 18 | 0.009 | 100.00 | |
| hGBA BD17 | GBA | 18 | 0.009 | 100.00 | |
| | GBAP1 | 18 | 0.009 | 100.00 | Unprocessed pseudogene |
| | MKI67 | 16 | 0.14 | 100.00 | Exon13 of 15 - M2131 |
| hGBA BD18 | GBA | 18 | 0.009 | 100.00 | |
| | DGKA | 15 | 0.54 | 100.00 | Exon21 of 24 - M665 |
| | SRP9 | 15 | 0.54 | 100.00 | Exon3 of 3 - M52 |
| hGBA BD19 | GBA | 18 | 0.009 | 100.00 | |
| hGBA BD20 | GBA | 30 | 4.00E-09 | 100.00 | |
| | AC217785.3 | 30 | 1.00E-06 | 96.67 | Processed pseudogene |
| | GBAP1 | 30 | 1.00E-06 | 96.67 | Unprocessed pseudogene |

Although a number of putative off-target binding targets have been identified for the disclosed binding domains/target binding sequences disclosed herein, the pairing position is not significant for these, based on the binding position, AUG start codon/translation start site (TSS) and in-frame methionine. Therefore the designed BDs present high specificity since relevant off-targets have not been identified.

### EXAMPLE 2

Binding domain sequences were designed to target mouse and human SNCA mRNA (summarised in Table 7) and the off-target binding (i.e. binding to mRNAs other than human SNCA mRNA) was analysed *in silico* using the UCSC browser (BLAT tool). An e-value greater than or equal to 2.1 represents low target specificity. The results are presented in Table 8.

**Table 7: Summary of designed binding domains for SNCA**

| **Name** | **Binding domain** | **Methionine in target mRNA** |
|---|---|---|
| Mouse SNCA (mSNCA) sequences | | |
| mSNCA BD1 | -35/-18 | M1 |
| mSNCA BD2 | -40/-3 | M1 |
| mSNCA BD3 | -41/-12 | M1 |
| mSNCA BD4 | -20/-3 | M1 |
| mSNCA BD5 | -40/+0 | M1 |
| mSNCA BD6 | -26/+0 | M1 |
| mSNCA BD7 | -14/+0 | M1 |
| mSNCA BD8 | -14/+4 | M1 |
| mSNCA BD9 | -15/+4 | M1 |
| mSNCA BD10 | -16+4 | M1 |
| mSNCA BD11 | -40/+4 | M1 |
| mSNCA BD12 | -32/+8 | M1 |
| mSNCA BD13 | -54/+16 | M1 |
| mSNCA BD14 | -26/+16 | M1 |
| mSNCA BD15 | -20/+1 | M1 |
| mSNCA BD16 | -14/+4 | M2 |
| mSNCA BD17 | -40/+0 | M2 |
| mSNCA BD18 | +0/+18 | M3 |
| mSNCA BD19 | -14/+23 | M3 |
| mSNCA BD20 | -42/+4 | M5 |
| mSNCA BD21 | +100/+118 | M2 |
| mSNCA BD22 | +109/+153 | M2 |

| Human SNCA (hSNCA) sequences | | |
|---|---|---|
| hSNCA BD1 | -37/-17 | M1 |
| hSNCA BD2 | -40/-3 | M1 |
| hSNCA BD3 | -40/-11 | M1 |
| hSNCA BD4 | -20/-3 | M1 |
| hSNCA BD5 | -40/+0 | M1 |
| hSNCA BD6 | -26/+0 | M1 |
| hSNCA BD7 | -14/+0 | M1 |
| hSNCA BD8 | -14/+4 | M1 |
| hSNCA BD9 | -15/+4 | M1 |
| hSNCA BD10 | -16/+4 | M1 |
| hSNCA BD11 | -40/+4 | M1 |
| hSNCA BD12 | -32/+8 | M1 |
| hSNCA BD13 | -54/+16 | M1 |
| hSNCA BD14 | -25/+16 | M1 |
| hSNCA BD15 | -20/+1 | M1 |
| hSNCA BD16 | -14/+4 | M2 |
| hSNCA BD17 | -40/+0 | M2 |
| hSNCA BD18 | -9/+37 | M3 |
| hSNCA BD19 | -14/+4 | M3 |
| hSNCA BD20 | +100/+118 | M2 |
| hSNCA BD21 | +69/+113 | M2 |
| hSNCA BD22 | +285/+303 | M2 |
| hSNCA BD23 | -6/+12 | M1 |

**Table 8: In silico off-target binding analysis of SNCA target binding sequences/BDs**

| **Binding Domain** | **Gene** | **Length** | **E-val** | **%ID** | **Match Location** |
|---|---|---|---|---|---|
| Mouse SNCA (mSNCA) sequences | | | | | |
| mSNCA BD1 | SNCA | 18 | 0.009 | 100.00 | |
| | DNAJC11 | 16 | 0.14 | 100.00 | Retained intron |
| mSNCA BD2 | SNCA | 38 | 8.00E-14 | 100.00 | |
| | DNAJC11 | 16 | 0.96 | 100.00 | Retained intron |
| mSNCA BD3 | SNCA | 30 | 3.00E-09 | 100.00 | |
| | DNAJC11 | 16 | 0.60 | 100.00 | Retained intron |
| mSNCA BD4 | SNCA | 18 | 0.009 | 100.00 | |
| | GRAP2 | 15 | 0.54 | 100.00 | 3' UTR |
| | SGMS2 | 15 | 0.54 | 100.00 | 3' UTR |
| mSNCA BD5 | SNCA | 40 | 6.00E-15 | 100.00 | |
| | DNAJC11 | 16 | 1.1 | 100.00 | Retained intron |
| mSNCA BD6 | SNCA | 27 | 1.00E-07 | 100.00 | |
| mSNCA BD7 | SNCA | 14 | 1.4 | 100.00 | |
| | RD3 | 14 | 1.4 | 100.00 | 3' UTR |
| mSNCA BD8 | SNCA | 18 | 0.009 | 100.00 | |
| | VMN1R168 | 15 | 0.54 | 100.00 | 3' UTR |
| | PPOX | 15 | 0.54 | 100.00 | Exon 4 of 9 |
| mSNCA BD9 | SNCA | 19 | 0.002 | 100.00 | |
| | VMN1R168 | 15 | 0.54 | 100.00 | 3' UTR |
| | PPOX | 15 | 0.54 | 100.00 | Exon 4 of 9 |
| mSNCA BD10 | SNCA | 20 | 8.00E-04 | 100.00 | |
| | VMN1R168 | 15 | 0.72 | 100.00 | 3' UTR |
| | PPOX | 15 | 0.72 | 100.00 | Exon 4 of 9 |
| mSNCA BD11 | SNCA | 44 | 3.00E-17 | 100.00 | |
| | DNAJC11 | 16 | 1.2 | 100.00 | Retained intron |
| mSNCA BD12 | SNCA | 40 | 6.00E-15 | 100.00 | |
| | PPOX | 16 | 1.1 | 100.00 | Exon 4 of 9 |
| mSNCA BD13 | SNCA | 42 | 4.00E-16 | 100.00 | |
| | PPOX | 16 | 1.1 | 100.00 | Exon 4 of 9 |
| mSNCA BD14 | SNCA | 42 | 4.00E-16 | 100.00 | |
| | PPOX | 16 | 1.1 | 100.00 | Exon 4 of 9 |
| mSNCA BD15 | SNCA | 21 | 2.00E-04 | 100.00 | |
| | GRAP2 | 15 | 0.90 | 100.00 | 3' UTR |
| | SGMS2 | 15 | 0.90 | 100.00 | 3' UTR |
| mSNCA BD16 | SNCA | 18 | 0.009 | 100.00 | |
| | VMN2R-PS87 | 15 | 0.54 | 100.00 | Processed transcript |
| | VMN2R77 | 15 | 0.54 | 100.00 | -96/-57 M1 |
| mSNCA BD17 | SNCA | 40 | 6.00E-15 | 100.00 | |
| | PPOX | 16 | 1.1 | 100.00 | Exon 4 of 9 |
| mSNCA BD18 | SNCA | 18 | 0.009 | 100.00 | |
| | MAPK11 | 15 | 0.54 | 100.00 | Intron |
| | ARHGAP4 | 15 | 0.54 | 100.00 | 3' UTR |
| mSNCA BD19 | SNCA | 37 | 3.00E-13 | 100.00 | |
| | LGFBP2 | 16 | 0.92 | 100.00 | Exon 2 or 4 M36 |
| | WFDC8 | 16 | 0.92 | 100.00 | M1 |
| | STAC3 | 16 | 0.92 | 100.00 | Exon 11-12 of 12 |
| mSNCA BD20 | SNCA | 46 | 2.00E-18 | 100.00 | |
| | GM18814 | 17 | 0.34 | 100.00 | Processed pseudogene |
| | ING5 | 17 | 0.34 | 100.00 | Exon 6 of 8 |
| | WDSUB1 | 21 | 0.34 | 95.24 | Intron |
| | IKBKE | 16 | 1.3 | 100.00 | Intron |
| | SLC18A1 | 16 | 1.3 | 100.00 | Exon 17 or 17 |
| mSNCA BD21 | SNCA | 18 | 0.009 | 100.00 | |
| mSNCA BD22 | SNCA | 44 | 3.00E-17 | 100.00 | |
| | ASNS | 16 | 1.2 | 100.00 | Exon 12 of 12 |

| Human SNCA (hSNCA) sequences | | | | | |
|---|---|---|---|---|---|
| hSNCA BD1 | SNCA | 18 | 0.009 | 100.00 | |
| hSNCA BD2 | SNCA | 38 | 1.00E-13 | 100.00 | |
| hSNCA BD3 | SNCA | 30 | 4.00E-09 | 100.00 | |
| hSNCA BD4 | SNCA | 18 | 0.009 | 100.00 | |
| hSNCA BD5 | SNCA | 40 | 8.00E-15 | 100.00 | |
| hSNCA BD6 | SNCA | 26 | 7.00E-07 | 100.00 | |
| hSNCA BD7 | SNCA | 14 | 2.2 | 100.00 | |
| hSNCA BD8 | SNCA | 18 | 0.009 | 100.00 | |
| hSNCA BD9 | SNCA | 20 | 0.001 | 100.00 | |
| hSNCA BD10 | SNCA | 20 | 0.001 | 100.00 | |
| hSNCA BD11 | SNCA | 44 | 4.00E-17 | 100.00 | |
| hSNCA BD12 | SNCA | 40 | 8.00E-15 | 100.00 | |
| hSNCA BD13 | SNCA | 70 | 3.00E-32 | 100.00 | |
| hSNCA BD14 | SNCA | 41 | 2.00E-15 | 100.00 | |
| hSNCA BD15 | SNCA | 21 | 4.00E-04 | 100.00 | |
| hSNCA BD16 | SNCA | 18 | 0.009 | 100.00 | |
| | AC020915.3 | 15 | 0.55 | 100.00 | Processed pseudogene |
| | QSOX2 | 15 | 0.55 | 100.00 | 3' UTR |
| hSNCA BD17 | SNCA | 40 | 8.00E-15 | 100.00 | |
| hSNCA BD18 | SNCA | 46 | 3.00E-18 | 100.00 | |
| | ING4 | 17 | 0.50 | 100.00 | Exon6 of 8 - M189 |
| hSNCA BD19 | SNCA | 18 | 0.009 | 100.00 | |
| | NUP160 | 15 | 0.55 | 100.00 | No matches found |
| hSNCA BD20 | SNCA | 18 | 0.009 | 100.00 | |
| hSNCA BD21 | SNCA | 44 | 4.00E-17 | 100.00 | |
| | NUP85 | 16 | 1.8 | 100.00 | Intron |
| | ECPAS | 16 | 1.8 | 100.00 | No matches found |
| hSNCA BD22 | SNCA | 18 | 0.009 | 100.00 | |
| | ALDH7A1 | 16 | 0.14 | 100.00 | |
| | NHLH2 | 15 | 0.55 | 100.00 | 5' UTR |
| hSNCA BD23 | SNCA | 18 | 0.009 | 100.00 | |

### SEQUENCE LISTING

| SEQ ID NO | Sequence | **Name/Description** |
|---|---|---|
| 1 | CAGUGCUAGAGGAGGUCAGAAGAGGGCAUUGGAUCCCCCAGAACUGGAGUUAUACGGU AACCUCGUGGUGGUUGUGAACCACCAUGUGGAUGGAUAUUGAGUUCCAAACACUGGUC CUGUGCAAGAGCAUCCAGUGCUCUUAAGUGCUGAGCCAUCUCUUUAGCUCC | AS Uchl1 SINEB2 |
| 2 | GAACUGGAGUUAUACGGUAACCUCGUGGUGGUUGUGAACCACCAUGUGGAUGGAUAUU GAGUUCCAAACACUGGUCC | nt 44-120 of inverted SINE B2 transposable element derived from AS Uchl1 |
| 3 | CCUCGUGGUGGUUGUGAACCACCAUGUGG | nt 64-92 of inverted SINE B2 transposable element derived from AS Uchl1 |
| 4 | GUUAUACGGUAACCUCGUGGUGGUUGUGAACCACCAUGUGGAUGGAUAUUGAGUUCCA AAC | nt 52-112 of inverted SINE B2 transposable element derived from AS Uchl1 |
| 5 | AUCCCCCAGAACUGGAGUUAUACGGUAACCUCGUGGUGGUUGUGAACCACCAUGUGGA UGGAUAUUGAGUUCCAAACACUGGUCCUGUGCAAGAGCAU | nt 36-133 of inverted SINE B2 transposable element derived from AS Uchl1 |
| 6 | GAAGAGGGCA UUGGAUCCCC CAGAACUGGA GUUAUACGGU AACCUCGUGG UGGUUGUGAA CCACCAUGUG GAUGGAUAUU GAGUUCCAAA CACUGGUCCU GUGCAAGAGC AUCCAGUGCU CUUAAGUGC | nt 22-150 of inverted SINE B2 transposable element derived from AS Uchl1 |
| 7 | GGGCAGUGCU AGAGGAGGUC AGAAGAGGGC AUUGGAUCCC CCAGAACUGG AGUUAUACGG UAACCUCGUG GUGGUUGUGA ACCACCAUGU GGAUGGAUAU UGAGUUCCAA ACACUGGUCC UGUGCAAGAG CAUCCAGUGC UCUUAAGUGC UGAGCCAUCU CUUUAGCUCC AGUCUCUUAA GCU | nt 1-183 of inverted SINE B2 transposable element derived from AS Uchl1 |
| 8 | GAACUGGAGU UAUACGGUAA CCUCGUGGUG GUUGUGAACC ACCAUGUGGA UGGAUAUUGA GUUCCAA | nt 44-110 of inverted SINE B2 transposable element derived from AS Uchl1 |
| 9 | GGAUCCCCCA GAACUGGAGU UAUACGGUAA CCUCGUGGUG GUUGUGAACC ACCAUGUGGA UGGAUAUUGA GUUCCAAACA CUGGUCCUGU GCAAGAGCAU CCAGUGC | nt 34-140 of inverted SINE B2 transposable element derived from AS Uchl1 |
| 10 | AGAGGGCAUU GGAUCCCCCA GAACUGGAGU UAUACGGUAA CCUCGUGGUG GUUGUGAACC ACCAUGUGGA UGGAUAUUGA GUUCCAAACA CUGGUCCUGU GCAAGAGCAU CCAGUGCUCU UAAGUGC | nt 24-150 of inverted SINE B2 transposable element derived from AS Uchl1 |
| 11 | GGUAACCUCG UGGUGGUUGU GAACCACCAU GUGGAUGG | nt 59-96 of inverted SINE B2 transposable element derived from AS Uchl1 |
| 12 | CAGUGCUAGA GGAGGUCAGA AGAGGGCAUU GGAUCCCCCA GAACUGGAGU UAUACGAUAA CCUCGUGGUG GUUGUGAACC ACCAUGUGGA UGGAUAUUGA GUUCCAAACA CUGGUCCUGU GCAAGAGCAU CCAGUGCUCU UAAGUGCUGA GCCAUCUCUU UAGCUCCAGU CUCUUAAGCU | G60A mutant of inverted SINE B2 transposable element derived from AS Uchl1 |
| 13 | CAGUGCUAGA GGAGGUCAGA AGAGGGCAUU GGAUCCCCCA GAACUGGAGU UAUACGCUAA CCUCGUGGUG GUUGUGAACC ACCAUGUGGA UGGAUAUUGA GUUCCAAACA CUGGUCCUGU GCAAGAGCAU CCAGUGCUCU UAAGUGCUGA GCCAUCUCUU UAGCUCCAGU CUCUUAAGCU | G60C mutant of inverted SINE B2 transposable element derived from AS Uchl1 |
| 14 | CAGUGCUAGA GGAGGUCAGA AGAGGGCAUU GGAUCCCCCA GAACUGGCGU UAUACGGUAA CCUCGUGGUG GUUGUGAACC ACCAUGUGGA UGGAUAUUGA GUUCCAAACA CUGGUCCUGU GCAAGAGCAU CCAGUGCUCU UAAGUGCUGA GCCAUCUCUU UAGCUCCAGU CUCUUAAGCU | A51C mutant of inverted SINE B2 transposable element derived from AS Uchl1 |
| 15 | CAGUGCUAGA GGAGGUCAGA AGAGGGCAUU GGAUCCCCCA GAAGUGGAGU UAUACGGUAA CCUCGUGGUG GUUGUGAACC ACCAUGUGGA UGGAUAUUGA GUUCCAAACA CUGCUCCUGU GCAAGAGCAU CCAGUGCUCU UAAGUGCUGA GCCAUCUCUU UAGCUCCAGU CUCUUAAGCU | C47G and G117C mutant of inverted SINE B2 transposable element derived from AS Uchl1 |
| 16 | CAGUGCUAGA GGAGGUCAGA AGAGGGCAUU GGAUCCCCCA GAUGGUGAGU UAUACGGUAA CCUCGUGGUG GUUGUGAACC ACCAUGUGGA UGGAUAUUGA GUUCCAAACA CGUCACCUGU GCAAGAGCAU CCAGUGCUCU UAAGUGCUGA GCCAUCUCUU UAGCUCCAGU CUCUUAAGCU | 46-49/115-118 stem swap mutant of inverted SINE B2 transposable element derived from AS Uchl1 |
| 17 | CAGUGCUAGA GGAGGUCAGA AGAGGGCAUU GGAUCCCCCA GAACUGCACU AUACGGUAAC CUCGUGGUGG UUGUGAACCA CCAUGUGGAU GGAUAUUGAG UUCCAAAUGA GUGGUCCUGU GCAAGAGCAU CCAGUGCUCU UAAGUGCUGA GCCAUCUCUU UAGCUCCAGU CUCUUAAGCU | 50-53 and 112-114 base swap mutant of inverted SINE B2 transposable element derived from AS Uchl1 |
| 18 | GGGCAUUGGA UCCCCCAGAA CUGGAGUUAU ACGGUAACCU CGUGGUGGUU GUGAACCACC AUGUGGAUGG AUAUUGAGUU CCAAACACUG GUCCUGUGCA AGAGCAUCCA GUGCUC | nt 27-142 of inverted SINE B2 transposable element derived from AS Uchl1 |
| 19 | GGACUGGAGU UAUACGGUAA CCUCGUGGUG GUUGUGAACC ACCAUGUGGA UGGAUAUUGA GUUCCAAACA CUGGUCC | nt 44-120 A45G mutant of inverted SINE B2 transposable element derived from AS Uchl1 |
| 20 | GAACUGGCGU UAUACGGUAA CCUCGUGGUG GUUGUGAACC ACCAUGUGGA UGGAUAUUGA GUUCCAAACA CUGGUCC | nt 44-120 A51C mutant of inverted sine B2 transposable element derived from AS Uchl1 |
| 21 | GAUGCCUUAG AAGUGGAGUU AAGAGUUGUG AGCUGCCGUU UUUUGGUUCU GGGACUCGAA CUCGUUUCCU CUGAUACUAU CAACCACCAA GCCAUCUCUU CAGCCCC | AS Txnip SINEB2 |
| 22 | GCCAGAAGAA GUUGUGGGAU UCCCUGGAAC UGGAGCAACC AACAGUUUGU GUGCACCAUG UGGGUAAUGG GAAUCGAACC UGGGUCCUCU AUAAGACUGG CCAGUGCUCU UAACUACUGA GGUGCAUUUC U | AS Uxt SINEB2 a |
| 23 | UUAUUUUAAA UAUAUGAGUA UUUCACCUGC AUAGGCGCAC AGUACCCACA GAGACUAGAA GAGGGUGGCA GAUCUCCUGA GACUGGAGUU AAUGCUUGUG AGCUGCCAUG UGGAUGCUGG AAAUCAAACC CAGGUCCUUU GGAAGGCAGG CAGGUGCUCU UAAUCAUGGA AGCAUCUCUU CAGCUCC | AS Uxt SINEB2 b |
| 24 | CAGCGACAUC AGAAGAGGAU AUUGGAUCCC AUUACAGAUG GUUGAAGGCC ACCAUGUCGU UGCUGGGAAU GAACUCAAGA CCUCUGGAAG AGCAGUCAGU GCUCUUAACC UCUGAGCCAU CUCUCCAGCC C | AS Gadd45alpha SINEB2 a |
| 25 | AUCCCCUCCA AAGCUCAAGA UGGUUGUAAG CCACCCUGUG AUUGCUGGGA UUUGAACUCA AGACCUCCGG AAGAGCAAUU AGUGCUCUUA ACCGCUGAGC AAUCUCUCCA GCCC | AS Gadd45alpha SINEB2 b |
| 26 | GUGCAGUGCU AGAGGAGGUC AGAAGAGGGC AUUGGAUCCC CCAGAACUGG AGUUAUACGG UAACCUCGUG GUGGUUGUGA ACCACCAUGU GGAUGGAUAU UGAGUUCCAA ACACUGGUCC UGUGCAAGAG CAUCCAGUGC UCUUAAGUGC UGAGCCAUCU CUUUAGCUCC UUAUUUUAAA UAUAUGAGUA UUUCACCUGC AUAGGCGCAC AGUACCCACA GAGACUAGAA GAGGGUGGCA GAUCUCCUGA GACUGGAGUU AAUGCUUGUG AGCUGCCAUG UGGAUGCUGG AAAUCAAACC CAGGUCCUUU GGAAGGCAGG CAGGUGCUCU UAAUCAUGGA AGCAUCUCUU CAGCUCC | AS Uchl1 SINEB2 + AS Uxt SINEB2 b |
| 27 | GUGCAGUGCU AGAGGAGGUC AGAAGAGGGC AUUGGAUCCC CCAGAACUGG AGUUAUACGG UAACCUCGUG GUGGUUGUGA ACCACCAUGU GGAUGGAUAU UGAGUUCCAA ACACUGGUCC UGUGCAAGAG CAUCCAGUGC UCUUAAGUGC UGAGCCAUCU CUUUAGCUCC GUGCGAAUUC GGUGCAGUGC UAGAGGAGGU CAGAAGAGGG CAUUGGAUCC CCCAGAACUG GAGUUAUACG GUAACCUCGU GGUGGUUGUG AACCACCAUG UGGAUGGAUA UUGAGUUCCA AACACUGGUC CUGUGCAAGA GCAUCCAGUG CUCUUAAGUG CUGAGCCAUC UCUUUAGCUC CGUGCGAAUU CGGUGCAGUG CUAGAGGAGG UCAGAAGAGG GCAUUGGAUC CCCCAGAACU GGAGUUAUAC GGUAACCUCG UGGUGGUUGU GAACCACCAU GUGGAUGGAU AUUGAGUUCC AAACACUGGU CCUGUGCAAG AGCAUCCAGU GCUCUUAAGU GCUGAGCCAU CUCUUUAGCU CC | 3x AS Uchl1 SINEB2 |
| 28 | UUUUUUUAAA AAUUUAUUUU UAUUUUAUGU GUAUGAGUGU UUUGCCUGCA UGUAUGUCUG UGUACCACGU GCGUGCCUGG UGCCCGCGGA GGCCAGAAGA GGGCGUCGGA UCCCCUGGAA CUGGAGUUAC AGAUGGUUGU GAGCCGCCAU GUGGGUGCUG GGAAUCGAAC CCGGGUCCUC UGGAAGAGCA GCCAGUGCUC UUAACCGCUG AGCCAUCUCU CCAGCCCC | SINEB2/B3 consensus sequence from RepBase |
| 29 | UUUUUUUUAC UUGUAUAGGU GUUUUGCCUG CAUGUGUAUC UAUCUAUGUA CCGAAUAUGU UCCUGGUAUC CACAGAGACC AAAAGUGGAU GUUGUAUCUC CUGAAAUUGG AGUCAUAGAC AGUUAUGAGC UGCCAUUUGA GUGCUUGGAA UAGAACCCAG GUCCUCUUAA AGAGCAUCCA GUGCUCUUAA AAACUGAGAC AUCUCUGUAG CCUC | AS Nars2 SINEB2/B3 |
| 30 | UUUAUUUUGC UUUAUGUGUC UGAGUGUUUG CUUGAAUGUA UGUCUGUGUA CCACGCCUGU ACCUUGUGCC UUCAGAGUUG AGAGGAGGGC AUAGGAUCUC CUGGAACUGG AAUUGCAGGU GGUUGUGAGC CACCCUGUGG GUCCUGGGGA CCAUACUCCA GCAAGAACAU CAUGUGCUCU UAAUUCCUGA GUCUCCAACC | AS Abhd11 SINEB2/B3 |
| 31 | UUUAUUUACU UAUCUUUAUG UGUAUGAGUG UGUUGUCAGA CUGUUAUGUC UGUGUGUCAC AUGCAUGCCU GCUGUUCAUG GAGUCCAGAA GAGGGCAUCG GAUCCCCUGG AACUGGAGUU ACAGAUGAGU GGCCAUGUGA AUGUUAAGAA CCAAACCUGG GUCCUCUGAA AGAGCAGACA AUGCUCUUAA CUACUGAGCU GUCUCUCCAG CCCC | AS Ebp4.9 SINEB2/B3 |
| 32 | UUAUUUUAUU CGUGUAAGUG UUUUGCCAGC AUCUAUGUCU UCGCACUAUG UGCAGGUCUG GUGCCUGAGG GGUCCAGACG AGAGCACUGG GUCUCCGGGA ACUGGAGUUA CAGAUCAUUG UGAGCCACCA UGUGGGUGCA GGGAAUCGAA CCUGGGACCU CUGGAGGAGC AGCCACUGCU CUUAACCACU ACACUAUUUC UCCAG | AS Wfdc5 SINEB2/B3 |
| 33 | UCUGUGGACC ACUGUGUACA GAAGCCUGAG AAGGCUAGCA GAUCCCCAGA ACUGGAACUG UGAGACGCUG UGCUAUGGAG GUGCUAGGAA CUGAAAAUGG AUGGGUCCUC UGCAAGAGCA G | AS Pgbd1 SINEB2/B3 |
| 34 | UUGUUUUAAU UGAAUGGCUA UAGGGUGUUU CUUCUGUAUG UAUAUCUAUG UUUGGUACCU ACAGAGGCAU CAGAUCCUCU GGAACUGUAG UUGCUGACAG UUGUGAGCUG UCAUGGGGAU GCUGGAAUUG AACCUGGAUC CUAUGAAAGA ACAGCCAGUG UUCUUAACCG CUGAGCUAUC UCUCCAGGCC C | AS Gsk3b SINEB2/B3 |
| 35 | UUUUUUUUUU AAUUUUAAAA AAAAAGAUUU UAUUUAUUUA UUUUAUAUAU GAUGAGUACA CUGUCACUCU UUUCAGACAC CCUAGAAAAG GGGGGCAUCA GAUCCCAUUA CAGAUGGUUG UGAGCCACAU GGUUGCUGGG AAUUGACCUC AGGACCUCUG AAAGAGCAGU CAGUGCUCUC AACCUUUGAG UCAUCUCUCC AGCCC | AS Rhod SINEB2_Mm2 |
| 36 | AUGUAUAUCU GUAAUGGGAC AUACUCACAU ACAUGGGCAC GUGAGUAUAA AAGGCCAGAA GAGAGCACUG GACCCUCUGG AGUUGAGAUU CUAAGCAGUU GUGAACCAUC UGAUGUAGGU GCUGGGAACU GAACUUGGGU CCUUUGCUAG AGAAGUAUGU CUCUUAACCA CUGAGCCGUA UCUCCAUCCC | AS Rhod SINEB2/B3A |
| 37 | UAAAGAUUUA UUCAUUAAGU ACACUGUAGC UAUCUUCAGA CGCAUCAGAA GAGGGCGUCA GAUCUCUUUA CAGGUGGUUG UGAGCCACCA UGUGGUUGCU GGAAUUUGAA CUCAGGACCU UCAAAAGAGC AGUCAGUGUU CUUAACCGCU GAGCCAUCUC UCCAACCCC | AS E4f1 SINEB2_Mm2 |
| 38 | UUAUUUAUUA UAAGUACACU GUAGCUGUCU UCAGACACAA CAAAAGAGGG CGUCAGAUCU CAUUACAGGU GGUUGAGCCA CCAUGUGGUU GCUGGAUUU GAACUCAGGA CCUUCAGAAC AGUCAGUGCU CUUACCCACU GAGCCAGCGA GCCAGCCCC | AS E4f1 SINEB2_Mm1t |
| 39 | GAACUGGAGU UAUACGGUAA CCUCGUGGUG GUUGGGAACC ACCAUGUGGA UGGAUAUUGA GUUCCAAACA CUGGUCC | 44-120 U78G SINEUP |
| 40 | GAACUGGAGU UAUACGGUAA CCUCGUGGUG GUUCCCAACC ACCAUGUGGA UGGAUAUUGA GUUCCAAACA CUGGUCC | 44-120 GUG77-79CCC SINEUP |
| 41 | GGACCGGAGU UAUACGGUAA CCGCGUGGUG GUUGUGAACC ACCACGCGGA UGGAUAUUGA GUUCCAAACA CCGGUCC | 44-120 SINEUP strong |
| 42 | GAACUAGAGU UAUACGGUAA CCACAUGGUG GUUGUGAACC ACCAUGUGGA UGGAUAUUGA GUUCCAAACA CUAGUUC | 44-120 SINEUP weak |
| 43 | UUAUUUUAAA UAUAUGAGUA UUUCACCUGC AUAGGCGCAC AGUACCCACA GAGACUAGAA GAGGGUAGUA GAUCCCCUAG AACUGGAGUU AUACGGUAAC CUCGUGGUGG UUGUGAGCUA CCAUGUGGAU GGAUACUGGG AAUCAAACCC AGGUCCUGUG GAAGGCAGGC AGGUGCUCUC AAGCACUGAG CCAUCUCUUC AGCUCC | SINEUP 071 and miniSINEUP 071 effector domain |
| 44 | UUAUUUUAAA UAUAUGAGUA UUUCACCUGC AUAGGCGCAC AGUGCUCAAG GAGAUCAGAA GAGGGCAUCA GAUCUCCUGA GACUGGAGUU AUACGGUAAC CUCGUGAUGG UUGUGAACUA CCAUGUGGAU GGAUAUUGAG UUCCAAACAC AGGUCCUGUG CAAGAGCAGC AGGUGCUCUU AAGCACGGAA CCAUCUCUUU AGCUCC | SINEUP 072 and miniSINEUP 072 effector domain |
| 45 | GAGGCUAGAA GAGGGUAUCA GAUCCCCUGA GACUGGAGUU AUACGGUAAC CUCGUGGUGG UUGUGAGCCA CCAUGUGGAU GGAUACUGAG AACCAAACCC UGGUCCUGUG CAAGAGCAUC AGGUGCUCUU AAGCACGGAA CCAUCUCUUC AGCUCC | SINEUP 073 and miniSINEUP 073 effector domain |
| 46 | GUCCUGUGCA AGAGCAUCGA ACUCGGUGCU CUUAAGCACA GAAGCCACCA AGCCAUCUCU UCAGCCCC | SINEUP 074 and miniSINEUP 074 effector domain |
| 47 | CAGUGCUAGA GGAGGUCAGA AGAGGGCAUC CCCCAGCCUC GUGGUGGUUG UGAACCACCA UGUGGCUGUG CAAGAGCAUG CUCUUAAGUG CUGAGCCAUC UCUUUAGCUC | SINEUP 075 and miniSINEUP 075 effector domain |
| 48 | GAGGGCAUUG GAUCCCCCAG AACUGGAGUU AUACGGUAAC CUCGUGGUGG UUGUGAACCA CCAUGUGGAU GGAUAUUGAG UUCCAAACAC UGGUCCUGUG CAAGAGCAUC CAGUGCUCUU AAGUGC | 24-150 effector domain |
| 49 | GGAUCCCCCA GAACUGGAGU UAUACGGUAA CCUCGUGGUG GUUGUGAACC ACCAUGUGGA UGGAUAUUGA GUUCCAAACA CUGGUCCUG | C_34-122 effector domain |
| 50 | UGCUAGAGGA GGUCAGAAGA GGGCAUUGGA UGCAAAUCCA GUGCUCUUAA GUGCUGAGCC AUCUCUUUAG CU | TM effector domain |
| 51 | GAGGGCAUUG GAUCCCCCAG AACUGGAGUU AUACGGUAAC GAUGGAUAUU GAGUUCCAAA CACUGGUCCU GUGCAAGAGC AUCCAGUGCU CUUA | MC2 effector domain |
| 52 | GCCAGCCCCC UGAUGGGGGC GACACUCCAC CAUGAAUCAC UCCCCUGUGA GGAACUACUG UCUUCACGCA GAAAGCGUCU AGCCAUGGCG UUAGUAUGAG UGUCGUGCAG CCUCCAGGAC CCCCCCUCCC GGGAGAGCCA UAGUGGUCUG CGGAACCGGU GAGUACACCG GAAUUGCCAG GACGACCGGG UCCUUUCUUG GAUAAACCCG CUCAAUGCCU GGAGAUUUGG GCGUGCCCCC GCAAGACUGC UAGCCGAGUA GUGUUGGGUC GCGAAAGGCC UUGUGGUACU GCCUGAUAGG GUGCUUGCGA GUGCCCCGGG AGGUCUCGUA GACCGUGCAC CAUGAGCACG AAUCCUAAAC CUCAAAGAAA AACCAAACGU AAC | HCV(d) IRES |
| 53 | GUUACGUUUG GUUUUUCUUU GAGGUUUAGG AUUCGUGCUC AUGGUGCACG GUCUACGAGA CCUCCCGGGG CACUCGCAAG CACCCUAUCA GGCAGUACCA CAAGGCCUUU CGCGACCCAA CACUACUCGG CUAGCAGUCU UGCGGGGGCA CGCCCAAAUC UCCAGGCAUU GAGCGGGUUU AUCCAAGAAA GGACCCGGUC GUCCUGGCAA UUCCGGUGUA CUCACCGGUU CCGCAGACCA CUAUGGCUCU CCCGGGAGGG GGGGUCCUGG AGGCUGCACG ACACUCAUAC UAACGCCAUG GCUAGACGCU UUCUGCGUGA AGACAGUAGU UCCUCACAGG GGAGUGAUUC AUGGUGGAGU GUCGCCCCCA UCAGGGGGCU GGC | HCV(i) IRES |
| 54 | AUGAGUCUGG ACAUCCCUCA CCGGUGACGG UGGUCCAGGC UGCGUUGGCG GCCUACCUAU GGCUAACGCC AUGGGACGCU AGUUGUGAAC AAGGUGUGAA GAGCCUAUUG AGCUACAUAA GAAUCCUCCG GCCCCUGAAU GCGGCUAAUC CCAACCUCGG AGCAGGUGGU CACAAACCAG UGAUUGGCCU GUCGUAACGC GCAAGUCCGU GGCGGAACCG ACUACUUUGG GUGUCCGUGU UUCCUUUUAU UUUAUUGUGG CUGCUUAUGG UGACAAUCAC AGAUUGUUAU CAUAAAGCGA AUUGGAUUGG CC | Polio(d) IRES |
| 55 | GGCCAAUCCA AUUCGCUUUA UGAUAACAAU CUGUGAUUGU CACCAUAAGC AGCCACAAUA AAAUAAAAGG AAACACGGAC ACCCAAAGUA GUCGGUUCCG CCACGGACUU GCGCGUUACG ACAGGCCAAU CACUGGUUUG UGACCACCUG CUCCGAGGUU GGGAUUAGCC GCAUUCAGGG GCCGGAGGAU UCUUAUGUAG CUCAAUAGGC UCUUCACACC UUGUUCACAA CUAGCGUCCC AUGGCGUUAG CCAUAGGUAG GCCGCCAACG CAGCCUGGAC CACCGUCACC GGUGAGGGAU GUCCAGACUC AU | Polio(i) IRES |
| 56 | CCCCCCCUCU CCCUCCCCCC CCCCUAACGU UACUGGCCGA AGCCGCUUGG AAUAAGGCCG GUGUGCGUUU GUCUAUAUGU UAUUUUCCAC CAUAUUGCCG UCUUUUGGCA AUGUGAGGGC CCGGAAACCU GGCCCUGUCU UCUUGACGAG CAUUCCUAGG GGUCUUUCCC CUCUCGCCAA AGGAAUGCAA GGUCUGUUGA AUGUCGUGAA GGAAGCAGUU CCUCUGGAAG CUUCUUGAAG ACAAACAACG UCUGUAGCGA CCCUUUGCAG GCAGCGGAAC CCCCCACCUG GCGACAGGUG CCUCUGCGGC CAAAAGCCAC GUGUAUAAGA UACACCUGCA AAGGCGGCAC AACCCCAGUG CCACGUUGUG AGUUGGAUAG UUGUGGAAAG AGUCAAAUGG CUCUCCUCAA GCGUAUUCAA CAAGGGGCUG AAGGAUGCCC AGAAGGUACC CCAUUGUAUG GGAUCUGAUC UGGGGCCUCG GUGCACAUGC UUUACAUGUG UUUAGUCGAG GUUAAAAAAC GUCUAGGCCC CCCGAACCAC GGGGACGUGG UUUUCCUUUG AAAAACACGA UGAUAA | EMCV(d) IRES |
| 57 | UUAUCAUCGU GUUUUUCAAA GGAAAACCAC GUCCCCGUGG UUCGGGGGGC CUAGACGUUU UUUAACCUCG ACUAAACACA UGUAAAGCAU GUGCACCGAG GCCCCAGAUC AGAUCCCAUA CAAUGGGGUA CCUUCUGGGC AUCCUUCAGC CCCUUGUUGA AUACGCUUGA GGAGAGCCAU UUGACUCUUU CCACAACUAU CCAACUCACA ACGUGGCACU GGGGUUGUGC CGCCUUUGCA GGUGUAUCUU AUACACGUGG CUUUUGGCCG CAGAGGCACC UGUCGCCAGG UGGGGGGUUC CGCUGCCUGC AAAGGGUCGC UACAGACGUU GUUUGUCUUC AAGAAGCUUC CAGAGGAACU GCUUCCUUCA CGACAUUCAA CAGACCUUGC AUUCCUUUGG CGAGAGGGGA AAGACCCCUA GGAAUGCUCG UCAAGAAGAC AGGGCCAGGU UUCCGGGCCC UCACAUUGCC AAAAGACGGC AAUAUGGUGG AAAAUAACAU AUAGACAAAC GCACACCGGC CUUAUUCCAA GCGGCUUCGG CCAGUAACGU UAGGGGGGGG GGAGGGAGAG GGGGGG | ECMV(i) IRES |
| 58 | AAAGCAAAAA UGUGAUCUUG CUUGUAAAUA CAAUUUUGAG AGGUUAAUAA AUUACAAGUA GUGCUAUUUU UGUAUUUAGG UUAGCUAUUU AGCUUUACGU UCCAGGAUGC CUAGUGGCAG CCCCACAAUA UCCAGGAAGC CCUCUCUGCG GUUUUUCAGA UUAGGUAGUC GAAAAACCUA AGAAAUUUAC CU | CrPV(d) IRES |
| 59 | AGGUAAAUUU CUUAGGUUUU UCGACUACCU AAUCUGAAAA ACCGCAGAGA GGGCUUCCUG GAUAUUGUGG GGCUGCCACU AGGCAUCCUG GAACGUAAAG CUAAAUAGCU AACCUAAAUA CAAAAAUAGC ACUACUUGUA AUUUAUUAAC CUCUCAAAAU UGUAUUUACA AGCAAGAUCA CAUUUUUGCU UU | CrPV(i) IRES |
| 60 | CAGAGAUCCA GGGGAGGCGC CUGUGAGGCC CGGACCUGCC CCGGGGCGAA GGGUAUGUGG CGAGACAGAG CCCUGCACCC CUAAUUCCCG GUGGAAAACU CCUGUUGCCG UUUCCCUCCA CCGGCCUGGA GUCUCCCAGU CUUGUCCCGG CAGUGCCGCC CUCCCCACUA AGACCUAGGC GCAAAGGCUU GGCUCAUGGU UGACAGCUCA GAGAGAGAAA GAUCUGAGGG A | Apaf-1(d) IRES |
| 61 | UCCCUCAGAU CUUUCUCUCU CUGAGCUGUC AACCAUGAGC CAAGCCUUUG CGCCUAGGUC UUAGUGGGGA GGGCGGCACU GCCGGGACAA GACUGGGAGA CUCCAGGCCG GUGGAGGGAA ACGGCAACAG GAGUUUUCCA CCGGGAAUUA GGGGUGCAGG GCUCUGUCUC GCCACAUACC CUUCGCCCCG GGGCAGGUCC GGGCCUCACA GGCGCCUCCC CUGGAUCUCU G | Apaf-1(i) IRES |
| 62 | ACUUUUGGUG GGCAUUUAAA AAUGUGUGUG UAUGUGUAUA UAUGUAUGUG UAUGUAUGUG UAUAUAUGUA UAUGUAUGUA UGUAUCGCGU GUAUGUGUGU AUGUAUGCAU GUGUAUGUAU GUAUAUGCAU GUAUGUGUAU GUGUAUAUAU GUAUGUGUGU GUAUGUAUAU GUGUGUGUAU GUGUAUGUGU GUGUGUAUGU GUGUGUGUAU GUAUGUAUGU AUGUAUAUGU AUUAUACACA UAUACACAUA UUGGUUUUUU UAAUCAUUUG AGAGUUAGUU GAAGAUAAAA ACCCAUCACC CCUAAAUGUA UUCCAAAGAA UAAGAACAUU GUUUUAUACA UAGCACACUU AACAAAAUCA AGAAAUUUAA CAUUAAUACA GUACUGUUAC CUAAUCCGUA GUCGAUUUUC AAAUUUUGUC AGUUGUUCCA AUAAUGUCCU UUAUAUAUUC CCCGCCCAGC | ELG-1 (d) IRES |
| 63 | GCUGGGCGGG GAAUAUAUAA AGGACAUUAU UGGAACAACU GACAAAAUUU GAAAAUCGAC UACGGAUUAG GUAACAGUAC UGUAUUAAUG UUAAAUUUCU UGAUUUUGUU AAGUGUGCUA UGUAUAAAAC AAUGUUCUUA UUCUUUGGAA UACAUUUAGG GGUGAUGGGU UUUUAUCUUC AACUAACUCU CAAAUGAUUA AAAAAACCAA UAUGUGUAUA UGUGUAUAAU ACAUAUACAU ACAUACAUAC AUACACACAC ACAUACACAC ACACAUACAC AUACACACAC AUAUACAUAC ACACACAUAC AUAUAUACAC AUACACAUAC AUGCAUAUAC AUACAUACAC AUGCAUACAU ACACACAUAC ACGCGAUACA UACAUACAUA UACAUAUAUA CACAUACAUA CACAUACAUA UAUACACAUA CACACACAUU UUUAAAUGCC CACCAAAAGU | ELG-1(i) IRES |
| 64 | AAUUCCAGCG AGAGGCAGAG GGAGCGAGCG GGCGGCCGGC UAGGGUGGAA GAGCCGGGCG AGCAGAGCUG CGCUGCGGGC GUCCUGGGAA GGGAGAUCCG GAGCGAAUAG GGGGCUUCGC CUCUGGCCCA GCCCUCCCGC UUGAUCCCCC AGGCCAGCGG UCCGCAACCC UUGCCGCAUC CACGAAACUU UGCCCAUAGC AGCGGGCGGG CACUUUGCAC UGGAACUUAC AACACCCGAG CAAGGACGCG ACUCUCCCGA CGCGGGGAGG CUAUUCUGCC CAUUUGGGGA CACUUCCCCG CCGCUGCCAG GACCCGCUUC UCUGAAAGGC UCUCCUUGCA GCUGCUUAGA CGCUGGAUUU UUUUCGGGUA GUGGAAAACC AGCAGCCUCC CGCGA | cMYC full length(d) IRES |
| 65 | UCGCGGGAGG CUGCUGGUUU UCCACUACCC GAAAAAAAUC CAGCGUCUAA GCAGCUGCAA GGAGAGCCUU UCAGAGAAGC GGGUCCUGGC AGCGGCGGGG AAGUGUCCCC AAAUGGGCAG AAUAGCCUCC CCGCGUCGGG AGAGUCGCGU CCUUGCUCGG GUGUUGUAAG UUCCAGUGCA AAGUGCCCGC CCGCUGCUAU GGGCAAAGUU UCGUGGAUGC GGCAAGGGUU GCGGACCGCU GGCCUGGGGG AUCAAGCGGG AGGGCUGGGC CAGAGGCGAA GCCCCCUAUU CGCUCCGGAU CUCCCUUCCC AGGACGCCCG CAGCGCAGCU CUGCUCGCCC GGCUCUUCCA CCCUAGCCGG CCGCCCGCUC GCUCCCUCUG CCUCUCGCUG GAAUU | cMYC full length(i) IRES |
| 66 | GGGCACUUUG CACUGGAACU UACAACACCC GAGCAAGGAC GCGACUCU | cMYC short variant(d) IRES |
| 67 | AGAGUCGCGU CCUUGCUCGG GUGUUGUAAG UUCCAGUGCA AAGUGCCC | cMYC short variant(i) IRES |
| 68 | GUACUGACAU CGUAGAUGGA AAUCAUAAAC UGACUCUUGG UUUGAUUUGG AAUAUAAUCC UCCACUGGCA G | DMD(d) IRES |
| 69 | CUGCCAGUGG AGGAUUAUAU UCCAAAUCAA ACCAAGAGUC AGUUUAUGAU UUCCAUCUAC GAUGUCAGUA C | DMD(i) IRES |
| 70 | GCATGGCTTCAGCCGAGT | hNURR1 BD1 |
| 71 | GCATGGCTTCAGCCGAGTTACA | hNURR1 BD2 |
| 72 | GGCTTCAGCCGAGTTACA | hNURR1 BD3 |
| 73 | GGCTTCAGCCGAGTTACAGG | hNURR1 BD4 |
| 74 | GCATGGCTTCAGCCGAGTTACAGG | hNURR1 BD5 |
| 75 | CCATGCTAAACTTGACAA | hNURR1 BD6 |
| 76 | GCTAAACTTGACAAACTC | hNURR1 BD7 |
| 77 | CCATGCTAAACTTGACAAACTC | hNURR1 BD8 |
| 78 | CCATAAAGGTACTGAAGC | hNURR1 BD9 |
| 79 | AAAGGTACTGAAGCTGGG | hNURR1 BD10 |
| 80 | CGGCGGCACCTCGGATCG | hGDNF BD1 |
| 81 | TAAAGTCCCGTCCGGCGGCGGCACCTCGGATCGGGTCT | hGDNF BD2 |
| 82 | CTTAAAGTCCCGTCCGGCGGCGGCACCTCGGATCGGGTCT | hGDNF BD3 |
| 83 | CTTAAAGTCCCGTCCGGCGGCGGCAC | hGDNF BD4 |
| 84 | CTTAAAGTCCCGTC | hGDNF BD5 |
| 85 | TCATCTTAAAGTCCCGTC | hGDNF BD6 |
| 86 | TCATCTTAAAGTCCCGTCC | hGDNF BD7 |
| 87 | TCATCTTAAAGTCCCGTCCG | hGDNF BD8 |
| 88 | TCATCTTAAAGTCCCGTCCGGCGGCGGCACC | hGDNF BD9 |
| 89 | TCATCTTAAAGTCCCGTCCGGCGGCGGCACCTCGGATCGGGTCT | hGDNF BD10 |
| 90 | AACTTCATCTTAAAGTCCCGTCCGGCGGCGGCACCTCGGA | hGDNF BD11 |
| 91 | TCCCATAACTTCATCTTAAAGTCCCGTCCGGCGGCGGCACCTCGGATCGGGTCTCCGCA GACCCTAGGTT | hGDNF BD12 |
| 92 | CATCCCATAACTTCATCTTAAAGTCCCGTCC | hGDNF BD13 |
| 93 | TCTTAAAGTCCCGTCCGGCGG | hGDNF BD14 |
| 94 | GCATATTTGAGTCACTGC | hGDNF BD15 |
| 95 | ATTTGAGTCACTGCTCAGCGCGAAG | hGDNF BD16 |
| 96 | CCATGACATCATCGAACTGATCAGGATAATCCTCTGGCATATTTGA | hGDNF BD17 |
| 97 | CCATGACATCATCGAACT | hGDNF BD18 |
| 98 | ACATGCCTGCCCTACTTT | hGDNF BD19 |
| 99 | TCATCTTAAAGTCCCGTCCGGCGGC | hGDNF BD20 |
| 100 | CTTAAAGTCCCGTCCGGCGGC | hGDNF BD21 |
| 101 | ACGACATCCCATAACTTCATC | hGDNF BD22 |
| 102 | ACATCCCATAACTTCATC | hGDNF BD23 |
| 103 | ATTTGAGTCACTGC | hGDNF BD24 |
| 104 | ATTTGAGTCACTGCTCAG | hGDNF BD25 |
| 105 | GCATATTTGAGTCACTGCTCAGCG | hGDNF BD26 |
| 106 | GGGACTGCGGCTAGGGCCGGCGGGTCTGGATGGCGGGTGC | hRET51 BD1 |
| 107 | CCCGTGCGCGCTGGGGCC | hRET51 BD2 |
| 108 | CCGTGCGCGCTGGGGCCACGGCTGGAGGG | hRET51 BD3 |
| 109 | CCCGTGCGCGCTGGGGCCACGGCTGGAGGGACTGCGGC | hRET51 BD4 |
| 110 | CGCCCGTGCGCGCTGGGGCCACGGCTGGAGGGACTGCGGC | hRET51 BD5 |
| 111 | CGCCCGTGCGCGCT | hRET51 BD6 |
| 112 | CCATCGCCCGTGCGCGCT | hRET51 BD7 |
| 113 | CCATCGCCCGTGCGCGCTG | hRET51 BD8 |
| 114 | CCATCGCCCGTGCGCGCTGG | hRET51 BD9 |
| 115 | CCATCGCCCGTGCGCGCTGGGGCCACGGCTGGAGGGACTGCGGC | hRET51 BD10 |
| 116 | TTCGCCATCGCCCGTGCGCGCTGGGGCCACGGCTGGAGGG | hRET51 BD11 |
| 117 | GTCGCCTTCGCCATCGCCCGTGCGCGCTGGGGCCACGGCTGGAGGGACTGCGGCTAGG GCCGGCGGGTCT | hRET51 BD12 |
| 118 | CCATCACCACCTCCTCGC | hRET51 BD13 |
| 119 | CACCACCTCCTCGCGCGCGCCGGCGTGCACGGTGCACACG | hRET51 BD14 |
| 120 | GCATGGTGCGGTTCTCCG | hRET51 BD15 |
| 121 | GGTGCGGTTCTCCGAGATGGAGAGGTTCCGGTTGAGAACCAGCCTATAGT | hRET51 BD16 |
| 122 | CCAATGCCACTTTGCCTA | hRET51 BD17 |
| 123 | CGGTTGCGGACACTGAGC | hRET51 BD18 |
| 124 | CACGGTGTCCTCCTTCCG | hRET51 BD19 |
| 125 | TGTCCTCCTTCCGCTTGAACTCCACCACGGCGCTGGCGGTGTCG | hRET51 BD20 |
| 126 | GCCATCGCCCGTGCGCGC | hRET51 BD21 |
| 127 | CCTCCTTCCGCTTGAACTCCACCA | hRET51 BD22 |
| 128 | CATCGCCCGTGCGCGCT | hRET51 BD23 |
| 129 | CCATCGCCCGTGCGC | hRET51 BD24 |
| 130 | TCGCCATCGCCCGTGCGC | hRET51 BD25 |
| 131 | CTTCGCCATCGCCCGTGCGC | hRET51 BD26 |
| 132 | GACGTCGCCTTCGCCATC | hRET51 BD27 |
| 133 | CGGACGTCGCCTTCGCCATC | hRET51 BD28 |
| 134 | GCACCGGACGTCGCCTTCGCCATC | hRET51 BD29 |
| 135 | CACCATCACCACCTCCTCGC | hRET51 BD30 |
| 136 | GGAAGGGCACCATCACCA | hRET51 BD31 |
| 137 | AAGGGCACCATCACC | hRET51 BD32 |
| 138 | GAGGATCCACGTCGGCGA | hGBA BD1 |
| 139 | CCTCAGGGTCATTAGATG | hGBA BD2 |
| 140 | CCTCAGGGTCATTAGATGAAGAGAAGACCACAGGGGTT | hGBA BD3 |
| 141 | CCCCTCAGGGTCATTAGATGAAGAGAAGACCACAGGGGTT | hGBA BD4 |
| 142 | CCATCCCCTCAGGGTCATTAGATGAAGAGAAGACCACAGGGGTT | hGBA BD5 |
| 143 | CCCCTCAGGGTCAT | hGBA BD6 |
| 144 | CCATCCCCTCAGGGTCAT | hGBA BD7 |
| 145 | CCATCCCCTCAGGGTCATT | hGBA BD8 |
| 146 | CCATCCCCTCAGGGTCATTA | hGBA BD9 |
| 147 | AACTCCATCCCCTCAGGGTCATTAGATGAAGAGAAGACCA | hGBA BD10 |
| 148 | CTTGAAAACTCCATCCCCTCAGGGTCATTAGATGAAGAGAAGACCACAGGGGTTCCAGAG TCTCTGAAGG | hGBA BD11 |
| 149 | GATGCTTACCCTACTCAAAGGCTTGGGACATTCCTCTCTG | hGBA BD12 |
| 150 | CCATCCGTCGCCCACTGC | hGBA BD13 |
| 151 | CCGTCGCCCACTGCGTGTACTCTCATAGCGGCTGAAGGTA | hGBA BD14 |
| 152 | ATACTCAGCTCCATCCGTCGCCCACTGCGTGTACTCTCATAGCGGCTGAAGGTA | hGBA BD15 |
| 153 | GCATCCCTCGATCCCAGG | hGBA BD16 |
| 154 | CAGGGGTATCTTGAGCTT | hGBA BD17 |
| 155 | GTATCTTGAGCTTGGTAT | hGBA BD18 |
| 156 | ACTCAGCTCCATCCGTCG | hGBA BD19 |
| 157 | CTGGCCATGGGTACCCGGATGATGTTATAT | hGBA BD20 |
| 158 | TGCTCCACACGGCTCCCT | mSNCA BD1 |
| 159 | CTAAAGATGTATTTTTGCTCCACACGGCTCCCTAGGCT | mSNCA BD2 |
| 160 | TATTTTTGCTCCACACGGCTCCCTAGGCTT | mSNCA BD3 |
| 161 | CTAAAGATGTATTTTTGC | mSNCA BD4 |
| 162 | GGCTAAAGATGTATTTTTGCTCCACACGGCTCCCTAGGCT | mSNCA BD5 |
| 163 | GGCTAAAGATGTATTTTTGCTCCACAC | mSNCA BD6 |
| 164 | GGCTAAAGATGTAT | mSNCA BD7 |
| 165 | CCATGGCTAAAGATGTAT | mSNCA BD8 |
| 166 | CCATGGCTAAAGATGTATT | mSNCA BD9 |
| 167 | CCATGGCTAAAGATGTATTT | mSNCA BD10 |
| 168 | CCATGGCTAAAGATGTATTTTTGCTCCACACGGCTCCCTAGGCT | mSNCA BD11 |
| 169 | ACATCCATGGCTAAAGATGTATTTTTGCTCCACACGGCTC | mSNCA BD12 |
| 170 | TCATGAACACATCCATGGCTAAAGATGTATTTTGCTCCACACGGCTCCCTAGGCTTCTGA AGAACTCCG | mSNCA BD13 |
| 171 | TCATGAACACATCCATGGCTAAAGATGTATTTTTGCTCCACA | mSNCA BD14 |
| 172 | TGGCTAAAGATGTATTTTTGC | mSNCA BD15 |
| 173 | TCATGAACACATCCATGG | mSNCA BD16 |
| 174 | GAACACATCCATGGCTAAAGATGTATTTTTGCTCCACACG | mSNCA BD17 |
| 175 | CTCCTCACCCTTGCCCAT | mSNCA BD18 |
| 176 | TACCCCTCCTCACCCTTGCCCATCTGGTCCTTCTTGA | mSNCA BD19 |
| 177 | GCATTTCATAAGCCTCACTGCCAGGATCCACAGGCATGTCTTCCAG | mSNCA BD20 |
| 178 | TTTTGGAACCTACATAGA | mSNCA BD21 |
| 179 | CACTGTTGTCACTCCATGAACCACTCCTTCCTTAGTTTTGGAAC | mSNCA BD22 |
| 180 | TACACCACACTGTCGTCG | hSNCA BD1 |
| 181 | CTAATGAATTCCTTTACACCACACTGTCGTCGAATGGC | hSNCA BD2 |
| 182 | TTCCTTTACACCACACTGTCGTCGAATGGC | hSNCA BD3 |
| 183 | CTAATGAATTCCTTTACA | hSNCA BD4 |
| 184 | GGCTAATGAATTCCTTTACACCACACTGTCGTCGAATGGC | hSNCA BD5 |
| 185 | GGCTAATGAATTCCTTTACACCACAC | hSNCA BD6 |
| 186 | GGCTAATGAATTCC | hSNCA BD7 |
| 187 | CCATGGCTAATGAATTCC | hSNCA BD8 |
| 188 | CCATGGCTAATGAATTCCT | hSNCA BD9 |
| 189 | CCATGGCTAATGAATTCCTT | hSNCA BD10 |
| 190 | CCATGGCTAATGAATTCCTTTACACCACACTGTCGTCGAATGGC | hSNCA BD11 |
| 191 | ACATCCATGGCTAATGAATTCCTTTACACCACACTGTCGT | hSNCA BD12 |
| 192 | TCATGAATACATCCATGGCTAATGAATTCCTTTACACCACACTGTCGTCGAATGGCCACTC CCAGTTCTC | hSNCA BD13 |
| 193 | TCATGAATACATCCATGGCTAATGAATTCCTTTACACCACA | hSNCA BD14 |
| 194 | TGGCTAATGAATTCCTTTACA | hSNCA BD15 |
| 195 | TCATGAATACATCCATGG | hSNCA BD16 |
| 196 | GAATACATCCATGGCTAATGAATTCCTTTACACCACACTG | hSNCA BD17 |
| 197 | GCA TTTCA T AAGCCT C ATTGTCAGGA TCCACAGGCAT A TCTTCCAG | hSNCA BD18 |
| 198 | GCATATCTTCCAGAATTC | hSNCA BD19 |
| 199 | TTTTGGAGCCTACATAGA | hSNCA BD20 |
| 200 | GAGCCTACATAGAGAACACCCTCTTTTGTCTTTCCTGCTGCTTC | hSNCA BD21 |
| 201 | TTCTTCATTCTTGCCCAA | hSNCA BD22 |
| 202 | GAATACATCCATGGCTAA | hSNCA BD23 |
| 203 | AUCUGCAGAAUUC | Linker |
| 204 | GAAUUC | Short Linker |

## Claims

1. A therapeutic agent for use in a method of treating a disease or disorder of the nervous system, wherein the therapeutic agent increases translation of an endogenous mRNA sequence selected from the group consisting of: an endogenous Nurr1, human GDNF, cRET or GBA mRNA sequence.

2. The therapeutic agent for use according to claim 1, wherein the disease or disorder of the nervous system is a disease or disorder of the central nervous system.

3. The therapeutic agent for use according to claim 1 or claim 2, wherein the disease or disorder of the nervous system is a neurodegenerative disease, such as Parkinson's disease.

4. The therapeutic agent for use according to any one of claims 1 to 3, wherein the therapeutic agent comprises a functional nucleic acid molecule comprising:
at least one target binding sequence comprising a sequence reverse complementary to a target mRNA sequence selected from the group consisting of: a Nurr1, GDNF, cRET or GBA mRNA sequence; and
at least one regulatory sequence comprising a SINE B2 element, a functionally active fragment of a SINE B2 element, an internal ribosome entry site (IRES) sequence or an IRES derived sequence.

5. The therapeutic agent for use according claim 4, wherein the at least one target binding sequence is at least 18 nucleotides long and comprises, from 3' to 5':
a sequence reverse complementary to 0 to 20 nucleotides of the 5' UTR and 0 to 4 nucleotides of the CDS of the Nurr1 mRNA sequence; or
a sequence reverse complementary to 0 to 18 nucleotides of the region upstream of an AUG site (start codon) of the Nurr1 mRNA and 0 to 4 nucleotides of the CDS of the Nurr1 mRNA sequence downstream of said AUG site.

6. The therapeutic agent for use according to claim 4, wherein the at least one target binding sequence is at least 14 nucleotides long and comprises, from 3' to 5':
a sequence reverse complementary to 0 to 21 nucleotides of the 5' UTR and 0 to 20 nucleotides of the CDS of the GDNF mRNA sequence; or
a sequence reverse complementary to 0 to 20 nucleotides of the region upstream of an AUG site (start codon) of the GDNF mRNA and 0 to 4 nucleotides of the CDS of the GDNF mRNA sequence downstream of said AUG site.

7. The therapeutic agent for use according to claim 4, wherein the at least one target binding sequence is at least 15 nucleotides long and comprises, from 3' to 5':
a sequence reverse complementary to 0 to 14 nucleotides of the 5' UTR and 0 to 23 nucleotides of the CDS of the cRET mRNA sequence; or
a sequence reverse complementary to 0 to 14 nucleotides of the region upstream of an AUG site (start codon) of the cRET mRNA and 0 to 13 nucleotides of the CDS of the cRET mRNA sequence downstream of said AUG site.

8. The therapeutic agent for use according to claim 4, wherein the at least one target binding sequence is at least 15 nucleotides long and comprises, from 3' to 5':
a sequence reverse complementary to 0 to 56 nucleotides of the 5' UTR and 0 to 14 nucleotides of the CDS of the GBA mRNA sequence; or
a sequence reverse complementary to 0 to 40 nucleotides of the region upstream of an AUG site (start codon) of the GBA mRNA and 0 to 12 nucleotides of the CDS of the GBA mRNA sequence downstream of said AUG site.

9. The therapeutic agent for use according to any one of claims 4 to 8, wherein the at least one regulatory sequence comprises a sequence with at least 75% sequence identity with a sequence selected from the group consisting of SEQ ID NOs: 1-69.

10. A functional nucleic acid molecule comprising:
at least one target binding sequence comprising a sequence reverse complementary to a target mRNA sequence selected from the group consisting of: a Nurr1, GDNF, cRET or GBA mRNA sequence; and
at least one regulatory sequence comprising a SINE B2 element, a functionally active fragment of a SINE B2 element, an IRES sequence or an IRES derived sequence.

11. A DNA molecule encoding the functional nucleic acid molecule according to claim 10.

12. An expression vector comprising the functional nucleic acid molecule according to claim 10 or the DNA molecule according to claim 11.

13. A composition comprising the functional nucleic acid molecule according to claim 10, the DNA molecule according to claim 11 or the expression vector according to claim 12.

14. A method of producing a non-human animal model of Parkinson's disease comprising: administering a functional nucleic acid molecule comprising: at least one target binding sequence comprising a sequence reverse complementary to a SNCA mRNA sequence; and at least one regulatory sequence comprising a SINE B2 element, a functionally active fragment of a SINE B2 element, an IRES sequence or an IRES derived sequence, to a non-human animal.

15. The method according to claim 14, wherein the non-human animal is a non-human primate.
